# EUROPÄISCHE PATENTSCHRIFT

(11) **EP 1 915 451 B1**
(45) Veröffentlichungstag und Bekanntmachung des Hinweises auf die Patenterteilung: **30.05.2012**
(21) Anmeldenummer: 06764286.8
(22) Anmeldetag: 01.08.2006
(51) Int. Cl.: C12N 15/82, C11B 1/02, C12N 9/02, C07K 14/415

(54) **VERFAHREN ZUR HERSTELLUNG VON ARACHIDONSÄURE UND/ODER EICOSAPENTAENSÄURE IN TRANSGENEN NUTZPFLANZEN**
METHOD FOR PRODUCING ARACHIDONIC ACID AND/OR EICOSAPENTAENOIC ACID IN USEFUL TRANSGENIC PLANTS
PROCEDE DE PRODUCTION D'ACIDE ARACHIDONIQUE ET/OU D'ACIDE EICOSAPENTANOIQUE DANS DES PLANTES UTILES TRANSGENIQUES

(30) Priorität: 09.08.2005 DE 102005038036
(43) Veröffentlichungstag der Anmeldung: 30.04.2008
(73) Patentinhaber: BASF Plant Science GmbH, 67056 Ludwigshafen (DE)
(72) Erfinder: CIRPUS, Petra, 68163 Mannheim (DE); BAUER, Jörg, 67117 Limburgerhof (DE); QIU, Xiao, Saskatoon Sk. S7N 3S5 (CA); WU, Guohai, Saskatoon Sk. S7N 3S5 (CA); DATLA, Nagamani, Saskatoon Sk. S7V 1B6 (CA); TRUKSA, Martin, Saskatoon Sk., S7N 0W9 (CA)
(86) Internationale Anmeldenummer: PCT/EP2006/064922
(87) Internationale Veröffentlichungsnummer: WO 2007/017419

(56) Entgegenhaltungen:
- WO-A2-03/093482
- WO-A2-2005/012316
- WO-A2-2005/083093
- WO-A2-2005/118814
- WO-A2-2006/100241
- WU G ET AL: "STEPWISE ENGINEERING TO PRODUCE HIGH YIELDS OF VERY LONG-CHAIN POLYUNSATURATED FATTY ACIDS IN PLANTS" NATURE BIOTECHNOLOGY, NATURE PUBLISHING GROUP, NEW YORK, NY, US, Bd. 23, Nr. 8, 1. August 2005 (2005-08-01), Seiten 1013-1017, XP007900180 ISSN: 1087-0156
- ABBADI A ET AL: "Biosynthesis of very-long-chain polyunsaturated fatty acids in transgenic oilseeds: Constraints on their accumulation" PLANT CELL, AMERICAN SOCIETY OF PLANT PHYSIOLOGISTS, ROCKVILLE, MD, US, Bd. 16, Nr. 10, Oktober 2004 (2004-10), Seiten 2734-2748, XP002334261 ISSN: 1040-4651
- DREXLER H ET AL: "Metabolic engineering of fatty acids for breeding of new oilseed crops: strategies, problems and first results" JOURNAL OF PLANT PHYSIOLOGY, FISCHER, STUTTGART, DE, Bd. 160, Nr. 7, 2003, Seiten 779-802, XP004955255 ISSN: 0176-1617
- DATABASE UniProt [Online] 1. Dezember 2001 (2001-12-01), "Delta-6 fatty acid desaturase" XP002416319 gefunden im EBI Database accession no. Q944W4 & HONG HAIPING ET AL: "High-level production of gamma-linolenic acid in Brassica juncea using a DELTA6 desaturase from Pythium irregulare" PLANT PHYSIOLOGY, AMERICAN SOCIETY OF PLANT PHYSIOLOGISTS, ROCKVILLE, MD, US, Bd. 129, Nr. 1, Mai 2002 (2002-05), Seiten 354-362, XP002207405 ISSN: 0032-0889
- QIU XIAO ET AL: "Identification of a DELTA4 fatty acid desaturase from Thraustochytrium sp. involved in the biosynthesis of docosahexanoic acid by heterologous expression in Saccharomyces cerevisiae and Brassica juncea" JOURNAL OF BIOLOGICAL CHEMISTRY, AMERICAN SOCIETY OF BIOLOCHEMICAL BIOLOGISTS, BIRMINGHAM,, US, Bd. 276, Nr. 34, 24. August 2001 (2001-08-24), Seiten 31561-31566, XP002207404 ISSN: 0021-9258
- DATABASE UniProt [Online] 21. Dezember 2004 (2004-12-21), "Polyunsaturated fatty acid elongase 1" XP002416320 gefunden im EBI Database accession no. Q5SE76 & MEYER A ET AL: "NOVEL FATTY ACID ELONGASES AND THEIR USE FOR THE RECONSTITUTION OF DOCOSAHEXAENOIC ACID BIOSYNTHESIS" JOURNAL OF LIPID RESEARCH, BETHESDA, MD, US, Bd. 45, Nr. 10, Oktober 2004 (2004-10), Seiten 1899-1909, XP009046591 ISSN: 0022-2275
- TRUKSA MARTIN ET AL: "Metabolic engineering of plants to produce very long-chain polyunsaturated fatty acids." TRANSGENIC RESEARCH APR 2006, Bd. 15, Nr. 2, April 2006 (2006-04), Seiten 131-137, XP002416460 ISSN: 0962-8819

### SEQUENCE LISTING

<110> BASF Plant Science GmbH
<120> Verfahren zur Herstellung von Arachidonsäure und/oder Eicosapentaensäure in transgenen Nutzpflanzen
<130> PF56991
<140> 20050640
   <141> 2005-08-08
<160> 12
<170> PatentIn version 3.1
<210> 1
   <211> 1380
   <212> DNA
   <213> Pythium irregulare
<220>
   <221> CDS
   <222> (1)..(1380)
   <223> Delta-6-Desaturase
<400> 1
   atg gtg gac ctc aag cct gga gtg aag cgc ctg gtg agc tgg aag gag      48
   Met Val Asp Leu Lys Pro Gly Val Lys Arg Leu Val Ser Trp Lys Glu
   1               5                   10                  15
   atc cgc gag cac gcg acg ccc gcg acc gcg tgg atc gtg att cac cac      96
   Ile Arg Glu His Ala Thr Pro Ala Thr Ala Trp Ile Val Ile His His
               20                  25                  30
   aag gtc tac gac atc tcc aag tgg gac tcg cac ccg ggt ggc tcc gtg     144
   Lys Val Tyr Asp Ile Ser Lys Trp Asp Ser His Pro Gly Gly Ser Val
           35                  40                  45
   atg ctc acg cag gcc ggc gag gac gcc acg gac gcc ttc gcg gtc ttc     192
   Met Leu Thr Gln Ala Gly Glu Asp Ala Thr Asp Ala Phe Ala Val Phe
       50                  55                  60
   cac ccg tcc tcg gcg ctc aag ctg ctc gag cag ttc tac gtc ggc gac     240
   His Pro Ser Ser Ala Leu Lys Leu Leu Glu Gln Phe Tyr Val Gly Asp
   65                  70                  75                  80
   gtg gac gaa acc tcc aag gcc gag atc gag ggg gag ccg gcg agc gac     288
   Val Asp Glu Thr Ser Lys Ala Glu Ile Glu Gly Glu Pro Ala Ser Asp
                   85                  90                  95
   gag gag cgc gcg cgc cgc gag cgc atc aac gag ttc atc gcg tcc tac     336
   Glu Glu Arg Ala Arg Arg Glu Arg Ile Asn Glu Phe Ile Ala Ser Tyr
               100                 105                 110
   cgc cgt ctg cgc gtc aag gtc aag ggc atg ggg ctc tac gac gcc agc     384
   Arg Arg Leu Arg Val Lys Val Lys Gly Met Gly Leu Tyr Asp Ala Ser
           115                 120                 125
   gcg ctc tac tac gcg tgg aag ctc gtg agc acg ttc ggc atc gcg gtg     432
   Ala Leu Tyr Tyr Ala Trp Lys Leu Val Ser Thr Phe Gly Ile Ala Val
       130                 135                 140
   ctc tcg atg gcg atc tgc ttc ttc ttc aac agt ttc gcc atg tac atg      480
   Leu Ser Met Ala Ile Cys Phe Phe Phe Asn Ser Phe Ala Met Tyr Met
   145                 150                 155                 160
   gtc gcc ggc gtg att atg ggg ctc ttc tac cag cag tcc gga tgg ctg      528
   Val Ala Gly Val Ile Met Gly Leu Phe Tyr Gln Gln Ser Gly Trp Leu
                   165                 170                 175
   gcg cac gac ttc ttg cac aac cag gtg tgc gag aac cgc acg ctc ggc      576
   Ala His Asp Phe Leu His Asn Gln Val Cys Glu Asn Arg Thr Leu Gly
               180                 185                 190
   aac ctt atc ggc tgc ctc gtg ggc aac gcc tgg cag ggc ttc agc atg      624
   Asn Leu Ile Gly Cys Leu Val Gly Asn Ala Trp Gln Gly Phe Ser Met
           195                 200                 205
   cag tgg tgg aag aac aag cac aac ctg cac cac gcg gtg ccg aac ctg      672
   Gln Trp Trp Lys Asn Lys His Asn Leu His His Ala Val Pro Asn Leu
       210                 215                 220
   cac agc gcc aag gac gag ggc ttc atc ggc gac ccg gac atc gac acc      720
   His Ser Ala Lys Asp Glu Gly Phe Ile Gly Asp Pro Asp Ile Asp Thr
   225                 230                 235                 240
   atg ccg ctg ctg gcg tgg tct aag gag atg gcg cgc aag gcg ttc gag      768
   Met Pro Leu Leu Ala Trp Ser Lys Glu Met Ala Arg Lys Ala Phe Glu
                   245                 250                 255
   tcg gcg cac ggc ccg ttc ttc atc cgc aac cag gcg ttc cta tac ttc      816
   Ser Ala His Gly Pro Phe Phe Ile Arg Asn Gln Ala Phe Leu Tyr Phe
               260                 265                 270
   ccg ctg ctg ctg ctc gcg cgc ctg agc tgg ctc gcg cag tcg ttc ttc      864
   Pro Leu Leu Leu Leu Ala Arg Leu Ser Trp Leu Ala Gln Ser Phe Phe
           275                 280                 285
   tac gtg ttc acc gag ttc tcg ttc ggc atc ttc gac aag gtc gag ttc      912
   Tyr Val Phe Thr Glu Phe Ser Phe Gly Ile Phe Asp Lys Val Glu Phe
       290                 295                 300
   gac gga ccg gag aag gcg ggt ctg atc gtg cac tac atc tgg cag ctc      960
   Asp Gly Pro Glu Lys Ala Gly Leu Ile Val His Tyr Ile Trp Gln Leu
   305                 310                 315                 320
   gcg atc ccg tac ttc tgc aac atg agc ctg ttt gag ggc gtg gca tac     1008
   Ala Ile Pro Tyr Phe Cys Asn Met Ser Leu Phe Glu Gly Val Ala Tyr
                   325                 330                 335
   ttc ctc atg ggc cag gcg tcc tgc ggc ttg ctc ctg gcg ctg gtg ttc     1056
   Phe Leu Met Gly Gln Ala Ser Cys Gly Leu Leu Leu Ala Leu Val Phe
               340                 345                 350
   agt att ggc cac aac ggc atg tcg gtg tac gag cgc gaa acc aag ccg     1104
   Ser Ile Gly His Asn Gly Met Ser Val Tyr Glu Arg Glu Thr Lys Pro
           355                 360                 365
   gac ttc tgg cag ctg cag gtg acc acg acg cgc aac atc cgc gcg tcg     1152
   Asp Phe Trp Gln Leu Gln Val Thr Thr Thr Arg Asn Ile Arg Ala Ser
               370                 375                 380
   gta ttc atg gac tgg ttc acc ggt ggc ttg aac tac cag atc gac cat      1200
   Val Phe Met Asp Trp Phe Thr Gly Gly Leu Asn Tyr Gln Ile Asp His
   385                 390                 395                 400
   cac ctg ttc ccg ctc gtg ccg cgc cac aac ttg cca aag gtc aac gtg      1248
   His Leu Phe Pro Leu Val Pro Arg His Asn Leu Pro Lys Val Asn Val
                   405                 410                 415
   ctc atc aag tcg cta tgc aag gag ttc gac atc ccg ttc cac gag acc      1296
   Leu Ile Lys Ser Leu Cys Lys Glu Phe Asp Ile Pro Phe His Glu Thr
               420                 425                 430
   ggc ttc tgg gag ggc atc tac gag gtc gtg gac cac ctg gcg gac atc      1344
   Gly Phe Trp Glu Gly Ile Tyr Glu Val Val Asp His Leu Ala Asp Ile
           435                 440                 445
   agc aag gaa ttt atc acc gag ttc cca gcg atg taa                      1380
   Ser Lys Glu Phe Ile Thr Glu Phe Pro Ala Met
       450                 455
<210> 2
   <211> 459
   <212> PRT
   <213> Pythium irregulare
<400> 2
   Met Val Asp Leu Lys Pro Gly Val Lys Arg Leu Val Ser Trp Lys Glu
   1               5                   10                  15
   Ile Arg Glu His Ala Thr Pro Ala Thr Ala Trp Ile Val Ile His His
               20                  25                  30
   Lys Val Tyr Asp Ile Ser Lys Trp Asp Ser His Pro Gly Gly Ser Val
           35                  40                  45
   Met Leu Thr Gln Ala Gly Glu Asp Ala Thr Asp Ala Phe Ala Val Phe
       50                  55                  60
   His Pro Ser Ser Ala Leu Lys Leu Leu Glu Gln Phe Tyr Val Gly Asp
   65                  70                  75                  80
   Val Asp Glu Thr Ser Lys Ala Glu Ile Glu Gly Glu Pro Ala Ser Asp
                   85                  90                  95
   Glu Glu Arg Ala Arg Arg Glu Arg Ile Asn Glu Phe Ile Ala Ser Tyr
               100                 105                 110
   Arg Arg Leu Arg Val Lys Val Lys Gly Met Gly Leu Tyr Asp Ala Ser
           115                 120                 125
   Ala Leu Tyr Tyr Ala Trp Lys Leu Val Ser Thr Phe Gly Ile Ala Val
       130                 135                 140
   Leu Ser Met Ala Ile Cys Phe Phe Phe Asn Ser Phe Ala Met Tyr Met
   145                 150                 155                 160
   Val Ala Gly Val Ile Met Gly Leu Phe Tyr Gln Gln Ser Gly Trp Leu
                   165                 170                 175
   Ala His Asp Phe Leu His Asn Gln Val Cys Glu Asn Arg Thr Leu Gly
               180                 185                 190
   Asn Leu Ile Gly Cys Leu Val Gly Asn Ala Trp Gln Gly Phe Ser Met
           195                 200                 205
   Gln Trp Trp Lys Asn Lys His Asn Leu His His Ala Val Pro Asn Leu
       210                 215                 220
   His Ser Ala Lys Asp Glu Gly Phe Ile Gly Asp Pro Asp Ile Asp Thr
   225                 230                 235                 240
   Met Pro Leu Leu Ala Trp Ser Lys Glu Met Ala Arg Lys Ala Phe Glu
                   245                 250                 255
   Ser Ala His Gly Pro Phe Phe Ile Arg Asn Gln Ala Phe Leu Tyr Phe
               260                 265                 270
   Pro Leu Leu Leu Leu Ala Arg Leu Ser Trp Leu Ala Gln Ser Phe Phe
           275                 280                 285
   Tyr Val Phe Thr Glu Phe Ser Phe Gly Ile Phe Asp Lys Val Glu Phe
       290                 295                 300
   Asp Gly Pro Glu Lys Ala Gly Leu Ile Val His Tyr Ile Trp Gln Leu
   305                 310                 315                 320
   Ala Ile Pro Tyr Phe Cys Asn Met Ser Leu Phe Glu Gly Val Ala Tyr
                   325                 330                 335
   Phe Leu Met Gly Gln Ala Ser Cys Gly Leu Leu Leu Ala Leu Val Phe
               340                 345                 350
   Ser Ile Gly His Asn Gly Met Ser Val Tyr Glu Arg Glu Thr Lys Pro
           355                 360                 365
   Asp Phe Trp Gln Leu Gln Val Thr Thr Thr Arg Asn Ile Arg Ala Ser
       370                 375                 380
   Val Phe Met Asp Trp Phe Thr Gly Gly Leu Asn Tyr Gln Ile Asp His
   385                 390                 395                 400
   His Leu Phe Pro Leu Val Pro Arg His Asn Leu Pro Lys Val Asn Val
                   405                 410                 415
   Leu Ile Lys Ser Leu Cys Lys Glu Phe Asp Ile Pro Phe His Glu Thr
               420                 425                 430
   Gly Phe Trp Glu Gly Ile Tyr Glu Val Val Asp His Leu Ala Asp Ile
           435                 440                 445
   Ser Lys Glu Phe Ile Thr Glu Phe Pro Ala Met
       450                 455
<210> 3
   <211> 831
   <212> DNA
   <213> Traustochytrium sp.
<220>
   <221> CDS
   <222> (1)..(831)
   <223> Delta-6-Elongase
<400> 3
   atg gac gtc gtc gag cag caa tgg cgc cgc ttc gtg gac gcc gtg gac      48
   Met Asp Val Val Glu Gln Gln Trp Arg Arg Phe Val Asp Ala Val Asp
   1               5                   10                  15
   aac gga atc gtg gag ttc atg gag cat gag aag ccc aac aag ctg aac      96
   Asn Gly Ile Val Glu Phe Met Glu His Glu Lys Pro Asn Lys Leu Asn
               20                  25                  30
   gag ggc aag ctc ttc acc tcg acc gag gag atg atg gcg ctt atc gtc     144
   Glu Gly Lys Leu Phe Thr Ser Thr Glu Glu Met Met Ala Leu Ile Val
           35                  40                  45
   ggc tac ctg gcg ttc gtg gtc ctc ggg tcc gcc ttc atg aag gcc ttt     192
   Gly Tyr Leu Ala Phe Val Val Leu Gly Ser Ala Phe Met Lys Ala Phe
       50                  55                  60
   gtc gat aag cct ttc gag ctc aag ttc ctc aag ctc gtg cac aac atc     240
   Val Asp Lys Pro Phe Glu Leu Lys Phe Leu Lys Leu Val His Asn Ile
   65                  70                  75                  80
   ttc ctc acc ggt ctg tcc atg tac atg gcc acc gag tgc gcg cgc cag     288
   Phe Leu Thr Gly Leu Ser Met Tyr Met Ala Thr Glu Cys Ala Arg Gln
                   85                  90                  95
   gca tac ctc ggc ggc tac aag ctc ttt ggc aac ccg atg gag aag ggc     336
   Ala Tyr Leu Gly Gly Tyr Lys Leu Phe Gly Asn Pro Met Glu Lys Gly
               100                 105                 110
   acc gag tcg cac gcc ccg ggc atg gcc aac atc atc tac atc ttc tac       384
   Thr Glu Ser His Ala Pro Gly Met Ala Asn Ile Ile Tyr Ile Phe Tyr
           115                 120                 125
   gtg agc aag ttc ctc gaa ttc ctc gac acc gtc ttc atg atc ctc ggc       432
   Val Ser Lys Phe Leu Glu Phe Leu Asp Thr Val Phe Met Ile Leu Gly
       130                 135                 140
   aag aag tgg aag cag ctc agc ttt ctc cac gtc tac cac cac gcg agc       480
   Lys Lys Trp Lys Gln Leu Ser Phe Leu His Val Tyr His His Ala Ser
   145                 150                 155                 160
   atc agc ttc atc tgg ggc atc atc gcc cgc ttc gcg ccc ggt ggc gac       528
   Ile Ser Phe Ile Trp Gly Ile Ile Ala Arg Phe Ala Pro Gly Gly Asp
                   165                 170                 175
   gcc tac ttc tct acc atc ctc aac agc agc gtg cat gtc gtg ctc tac       576
   Ala Tyr Phe Ser Thr Ile Leu Asn Ser Ser Val His Val Val Leu Tyr
               180                 185                 190
   ggc tac tac gcc tcg acc acc ctc ggc tac acc ttc atg cgc ccg ctg       624
   Gly Tyr Tyr Ala Ser Thr Thr Leu Gly Tyr Thr Phe Met Arg Pro Leu
           195                 200                 205
   cgc ccg tac att acc acc att cag ctc acg cag ttc atg gcc atg gtc       672
   Arg Pro Tyr Ile Thr Thr Ile Gln Leu Thr Gln Phe Met Ala Met Val
       210                 215                 220
   gtc cag tcc gtc tat gac tac tac aac ccc tgc gac tac ccg cag ccc       720
   Val Gln Ser Val Tyr Asp Tyr Tyr Asn Pro Cys Asp Tyr Pro Gln Pro
   225                 230                 235                 240
   ctc gtc aag ctg ctc ttc tgg tac atg ctc acc atg ctc ggc ctc ttc       768
   Leu Val Lys Leu Leu Phe Trp Tyr Met Leu Thr Met Leu Gly Leu Phe
                   245                 250                 255
   ggc aac ttc ttc gtg cag cag tac ctc aag ccc aag gcg ccc aag aag       816
   Gly Asn Phe Phe Val Gln Gln Tyr Leu Lys Pro Lys Ala Pro Lys Lys
               260                 265                 270
   cag aag acc atc taa                                                   831
   Gln Lys Thr Ile
           275
<210> 4
   <211> 276
   <212> PRT
   <213> Traustochytrium sp.
<400> 4
   Met Asp Val Val Glu Gln Gln Trp Arg Arg Phe Val Asp Ala Val Asp
   1               5                   10                  15
   Asn Gly Ile Val Glu Phe Met Glu His Glu Lys Pro Asn Lys Leu Asn
               20                  25                  30
   Glu Gly Lys Leu Phe Thr Ser Thr Glu Glu Met Met Ala Leu Ile Val
           35                  40                  45
   Gly Tyr Leu Ala Phe Val Val Leu Gly Ser Ala Phe Met Lys Ala Phe
       50                  55                  60
   Val Asp Lys Pro Phe Glu Leu Lys Phe Leu Lys Leu Val His Asn Ile
   65                  70                  75                  80
   Phe Leu Thr Gly Leu Ser Met Tyr Met Ala Thr Glu Cys Ala Arg Gln
                   85                  90                  95
   Ala Tyr Leu Gly Gly Tyr Lys Leu Phe Gly Asn Pro Met Glu Lys Gly
               100                 105                 110
   Thr Glu Ser His Ala Pro Gly Met Ala Asn Ile Ile Tyr Ile Phe Tyr
           115                 120                 125
   Val Ser Lys Phe Leu Glu Phe Leu Asp Thr Val Phe Met Ile Leu Gly
       130                 135                 140
   Lys Lys Trp Lys Gln Leu Ser Phe Leu His Val Tyr His His Ala Ser
   145                 150                 155                 160
   Ile Ser Phe Ile Trp Gly Ile Ile Ala Arg Phe Ala Pro Gly Gly Asp
                   165                 170                 175
   Ala Tyr Phe Ser Thr Ile Leu Asn Ser Ser Val His Val Val Leu Tyr
               180                 185                 190
   Gly Tyr Tyr Ala Ser Thr Thr Leu Gly Tyr Thr Phe Met Arg Pro Leu
           195                 200                 205
   Arg Pro Tyr Ile Thr Thr Ile Gln Leu Thr Gln Phe Met Ala Met Val
       210                 215                 220
   Val Gln Ser Val Tyr Asp Tyr Tyr Asn Pro Cys Asp Tyr Pro Gln Pro
   225                 230                 235                 240
   Leu Val Lys Leu Leu Phe Trp Tyr Met Leu Thr Met Leu Gly Leu Phe
                   245                 250                 255
   Gly Asn Phe Phe Val Gln Gln Tyr Leu Lys Pro Lys Ala Pro Lys Lys
               260                 265                 270
   Gln Lys Thr Ile
           275
<210> 5
   <211> 1320
   <212> DNA
   <213> Traustochytrium sp.
<220>
   <221> CDS
   <222> (1)..(1320)
   <223> Delta-5-Desaturase
<400> 5
   atg ggc aag ggc agc gag ggc cgc agc gcg gcg cgc gag atg acg gcc      48
   Met Gly Lys Gly Ser Glu Gly Arg Ser Ala Ala Arg Glu Met Thr Ala
   1               5                   10                  15
   gag gcg aac ggc gac aag cgg aaa acg att ctg atc gag ggc gtc ctg      96
   Glu Ala Asn Gly Asp Lys Arg Lys Thr Ile Leu Ile Glu Gly Val Leu
               20                  25                  30
   tac gac gcg acg aac ttt aag cac ccg ggc ggt tcg atc atc aac ttc     144
   Tyr Asp Ala Thr Asn Phe Lys His Pro Gly Gly Ser Ile Ile Asn Phe
           35                  40                  45
   ttg acc gag ggc gag gcc ggc gtg gac gcg acg cag gcg tac cgc gag     192
   Leu Thr Glu Gly Glu Ala Gly Val Asp Ala Thr Gln Ala Tyr Arg Glu
       50                  55                  60
   ttt cat cag cgg tcc ggc aag gcc gac aag tac ctc aag tcg ctg ccg     240
   Phe His Gln Arg Ser Gly Lys Ala Asp Lys Tyr Leu Lys Ser Leu Pro
   65                  70                  75                  80
   aag ctg gat gcg tcc aag gtg gag tcg cgg ttc tcg gcc aaa gag cag     288
   Lys Leu Asp Ala Ser Lys Val Glu Ser Arg Phe Ser Ala Lys Glu Gln
                   85                  90                  95
   gcg cgg cgc gac gcc atg acg cgc gac tac gcg gcc ttt cgc gag gag     336
   Ala Arg Arg Asp Ala Met Thr Arg Asp Tyr Ala Ala Phe Arg Glu Glu
               100                 105                 110
   ctc gtc gcc gag ggg tac ttt gac ccg tcg atc ccg cac atg att tac     384
   Leu Val Ala Glu Gly Tyr Phe Asp Pro Ser Ile Pro His Met Ile Tyr
           115                 120                 125
   cgc gtc gtg gag atc gtg gcg ctc ttc gcg ctc tcg ttc tgg ctc atg     432
   Arg Val Val Glu Ile Val Ala Leu Phe Ala Leu Ser Phe Trp Leu Met
       130                 135                 140
   tcc aag gcc tcg ccc acc tcg ctc gtg ctg ggc gtg gtg atg aac ggc     480
   Ser Lys Ala Ser Pro Thr Ser Leu Val Leu Gly Val Val Met Asn Gly
   145                 150                 155                 160
   att gcg cag ggc cgc tgc ggc tgg gtc atg cac gag atg ggc cac ggg     528
   Ile Ala Gln Gly Arg Cys Gly Trp Val Met His Glu Met Gly His Gly
                   165                 170                 175
   tcg ttc acg ggc gtc atc tgg ctc gac gac cgg atg tgc gag ttc ttc      576
   Ser Phe Thr Gly Val Ile Trp Leu Asp Asp Arg Met Cys Glu Phe Phe
                   180                 185                 190
   tac ggc gtc ggc tgc ggc atg agc ggg cac tac tgg aag aac cag cac      624
   Tyr Gly Val Gly Cys Gly Met Ser Gly His Tyr Trp Lys Asn Gln His
               195                 200                 205
   agc aag cac cac gcc gcg ccc aac cgc ctc gag cac gat gtc gat ctc      672
   Ser Lys His His Ala Ala Pro Asn Arg Leu Glu His Asp Val Asp Leu
       210                 215                 220
   aac acg ctg ccc ctg gtc gcc ttt aac gag cgc gtc gtg cgc aag gtc      720
   Asn Thr Leu Pro Leu Val Ala Phe Asn Glu Arg Val Val Arg Lys Val
   225                 230                 235                 240
   aag ccg gga tcg ctg ctg gcg ctc tgg ctg cgc gtg cag gcg tac ctc      768
   Lys Pro Gly Ser Leu Leu Ala Leu Trp Leu Arg Val Gln Ala Tyr Leu
                   245                 250                 255
   ttt gcg ccc gtc tcg tgc ctg ctc atc ggc ctt ggc tgg acg ctc tac      816
   Phe Ala Pro Val Ser Cys Leu Leu Ile Gly Leu Gly Trp Thr Leu Tyr
               260                 265                 270
   ctg cac ccg cgc tac atg ctg cgc acc aag cgg cac atg gag ttc gtc      864
   Leu His Pro Arg Tyr Met Leu Arg Thr Lys Arg His Met Glu Phe Val
           275                 280                 285
   tgg atc ttc gcg cgc tac att ggc tgg ttc tcg ctc atg ggc gct ctc      912
   Trp Ile Phe Ala Arg Tyr Ile Gly Trp Phe Ser Leu Met Gly Ala Leu
       290                 295                 300
   ggc tac tcg ccg ggc acc tcg gtc ggg atg tac ctg tgc tcg ttc ggc      960
   Gly Tyr Ser Pro Gly Thr Ser Val Gly Met Tyr Leu Cys Ser Phe Gly
   305                 310                 315                 320
   ctc ggc tgc att tac att ttc ctg cag ttc gcc gtc agc cac acg cac     1008
   Leu Gly Cys Ile Tyr Ile Phe Leu Gln Phe Ala Val Ser His Thr His
                   325                 330                 335
   ctg ccg gtg acc aac ccg gag gac cag ctg cac tgg ctc gag tac gcg     1056
   Leu Pro Val Thr Asn Pro Glu Asp Gln Leu His Trp Leu Glu Tyr Ala
               340                 345                 350
   gcc gac cac acg gtg aac att agc acc aag tcc tgg ctc gtc acg tgg     1104
   Ala Asp His Thr Val Asn Ile Ser Thr Lys Ser Trp Leu Val Thr Trp
           355                 360                 365
   tgg atg tcg aac ctg aac ttt cag atc gag cac cac ctc ttc ccc acg     1152
   Trp Met Ser Asn Leu Asn Phe Gln Ile Glu His His Leu Phe Pro Thr
       370                 375                 380
   gcg ccg cag ttc cgc ttc aag gaa atc agt cct cgc gtc gag gcc ctc     1200
   Ala Pro Gln Phe Arg Phe Lys Glu Ile Ser Pro Arg Val Glu Ala Leu
   385                 390                 395                 400
   ttc aag cgc cac aac ctc ccg tac tac gac ctg ccc tac acg agc gcg     1248
   Phe Lys Arg His Asn Leu Pro Tyr Tyr Asp Leu Pro Tyr Thr Ser Ala
                   405                 410                 415
   gtc tcg acc acc ttt gcc aat ctt tat tcc gtc ggc cac tcg gtc ggc     1296
   Val Ser Thr Thr Phe Ala Asn Leu Tyr Ser Val Gly His Ser Val Gly
               420                 425                 430
   gcc gac acc aag aag cag gac tga                                     1320
   Ala Asp Thr Lys Lys Gln Asp
           435
<210> 6
   <211> 439
   <212> PRT
   <213> Traustochytrium sp.
<400> 6
   Met Gly Lys Gly Ser Glu Gly Arg Ser Ala Ala Arg Glu Met Thr Ala
   1               5                   10                  15
   Glu Ala Asn Gly Asp Lys Arg Lys Thr Ile Leu Ile Glu Gly Val Leu
               20                  25                  30
   Tyr Asp Ala Thr Asn Phe Lys His Pro Gly Gly Ser Ile Ile Asn Phe
           35                  40                  45
   Leu Thr Glu Gly Glu Ala Gly Val Asp Ala Thr Gln Ala Tyr Arg Glu
       50                  55                  60
   Phe His Gln Arg Ser Gly Lys Ala Asp Lys Tyr Leu Lys Ser Leu Pro
   65                  70                  75                  80
   Lys Leu Asp Ala Ser Lys Val Glu Ser Arg Phe Ser Ala Lys Glu Gln
                   85                  90                  95
   Ala Arg Arg Asp Ala Met Thr Arg Asp Tyr Ala Ala Phe Arg Glu Glu
               100                 105                 110
   Leu Val Ala Glu Gly Tyr Phe Asp Pro Ser Ile Pro His Met Ile Tyr
           115                 120                 125
   Arg Val Val Glu Ile Val Ala Leu Phe Ala Leu Ser Phe Trp Leu Met
       130                 135                 140
   Ser Lys Ala Ser Pro Thr Ser Leu Val Leu Gly Val Val Met Asn Gly
   145                 150                 155                 160
   Ile Ala Gln Gly Arg Cys Gly Trp Val Met His Glu Met Gly His Gly
                   165                 170                 175
   Ser Phe Thr Gly Val Ile Trp Leu Asp Asp Arg Met Cys Glu Phe Phe
               180                 185                 190
   Tyr Gly Val Gly Cys Gly Met Ser Gly His Tyr Trp Lys Asn Gln His
           195                 200                 205
   Ser Lys His His Ala Ala Pro Asn Arg Leu Glu His Asp Val Asp Leu
       210                 215                 220
   Asn Thr Leu Pro Leu Val Ala Phe Asn Glu Arg Val Val Arg Lys Val
   225                 230                 235                 240
   Lys Pro Gly Ser Leu Leu Ala Leu Trp Leu Arg Val Gln Ala Tyr Leu
                   245                 250                 255
   Phe Ala Pro Val Ser Cys Leu Leu Ile Gly Leu Gly Trp Thr Leu Tyr
               260                 265                 270
   Leu His Pro Arg Tyr Met Leu Arg Thr Lys Arg His Met Glu Phe Val
           275                 280                 285
   Trp Ile Phe Ala Arg Tyr Ile Gly Trp Phe Ser Leu Met Gly Ala Leu
       290                 295                 300
   Gly Tyr Ser Pro Gly Thr Ser Val Gly Met Tyr Leu Cys Ser Phe Gly
   305                 310                 315                 320
   Leu Gly Cys Ile Tyr Ile Phe Leu Gln Phe Ala Val Ser His Thr His
                   325                 330                 335
   Leu Pro Val Thr Asn Pro Glu Asp Gln Leu His Trp Leu Glu Tyr Ala
               340                 345                 350
   Ala Asp His Thr Val Asn Ile Ser Thr Lys Ser Trp Leu Val Thr Trp
           355                 360                 365
   Trp Met Ser Asn Leu Asn Phe Gln Ile Glu His His Leu Phe Pro Thr
       370                 375                 380
   Ala Pro Gln Phe Arg Phe Lys Glu Ile Ser Pro Arg Val Glu Ala Leu
   385                 390                 395                 400
   Phe Lys Arg His Asn Leu Pro Tyr Tyr Asp Leu Pro Tyr Thr Ser Ala
                   405                 410                 415
   Val Ser Thr Thr Phe Ala Asn Leu Tyr Ser Val Gly His Ser Val Gly
               420                 425                 430
   Ala Asp Thr Lys Lys Gln Asp
           435
<210> 7
   <211> 819
   <212> DNA
   <213> Talassiosira pseudonana
<220>
   <221> CDS
   <222> (1)..(819)
   <223> Delta-6-Elongase
<400> 7
   atg gac gcc tac aac gct gca atg gat aag atc ggt gcc gcc atc atc      48
   Met Asp Ala Tyr Asn Ala Ala Met Asp Lys Ile Gly Ala Ala Ile Ile
   1               5                   10                  15
   gat tgg tct gat ccc gat gga aag ttc cgt gcc gat aga gag gac tgg      96
   Asp Trp Ser Asp Pro Asp Gly Lys Phe Arg Ala Asp Arg Glu Asp Trp
               20                  25                  30
   tgg ctc tgc gac ttc cgt agc gcc atc acc atc gcc ctc atc tac atc      144
   Trp Leu Cys Asp Phe Arg Ser Ala Ile Thr Ile Ala Leu Ile Tyr Ile
           35                  40                  45
   gcc ttc gtc atc ctc ggt tcc gcc gtc atg caa tcc ctc ccc gca atg      192
   Ala Phe Val Ile Leu Gly Ser Ala Val Met Gln Ser Leu Pro Ala Met
       50                  55                  60
   gat ccc tac ccc atc aaa ttc ctc tac aac gtc tcc caa atc ttc ctt      240
   Asp Pro Tyr Pro Ile Lys Phe Leu Tyr Asn Val Ser Gln Ile Phe Leu
   65                  70                  75                  80
   tgt gcc tac atg act gtc gag gcg gga ttt ttg gcc tac cgc aat gga      288
   Cys Ala Tyr Met Thr Val Glu Ala Gly Phe Leu Ala Tyr Arg Asn Gly
                   85                  90                  95
   tat acc gtc atg cct tgc aat cat ttc aat gtg aat gat cct ccc gtg      336
   Tyr Thr Val Met Pro Cys Asn His Phe Asn Val Asn Asp Pro Pro Val
               100                 105                 110
   gcg aat ctt ctt tgg ttg ttt tat att tcc aag gtg tgg gac ttt tgg      384
   Ala Asn Leu Leu Trp Leu Phe Tyr Ile Ser Lys Val Trp Asp Phe Trp
           115                 120                 125
   gat acc att ttc att gtg ttg ggg aag aag tgg cgt caa tta tct ttc      432
   Asp Thr Ile Phe Ile Val Leu Gly Lys Lys Trp Arg Gln Leu Ser Phe
       130                 135                 140
   ttg cat gta tac cat cac acc acc atc ttt cta ttc tat tgg ctg aat      480
   Leu His Val Tyr His His Thr Thr Ile Phe Leu Phe Tyr Trp Leu Asn
   145                 150                 155                 160
   gcc aat gtc ttg tac gat ggt gac atc ttc ctt acc atc ttg ctc aat      528
   Ala Asn Val Leu Tyr Asp Gly Asp Ile Phe Leu Thr Ile Leu Leu Asn
                   165                 170                 175
   gga ttc atc cac acg gtg atg tac acg tat tac ttc atc tgt atg cat      576
   Gly Phe Ile His Thr Val Met Tyr Thr Tyr Tyr Phe Ile Cys Met His
               180                 185                 190
   acc aaa gat tcc aag acg ggc aag agt ctt cct ata tgg tgg aag tcg      624
   Thr Lys Asp Ser Lys Thr Gly Lys Ser Leu Pro Ile Trp Trp Lys Ser
           195                 200                 205
   agt ttg acg gcg ttt cag ttg ttg caa ttc act atc atg atg agt cag      672
   Ser Leu Thr Ala Phe Gln Leu Leu Gln Phe Thr Ile Met Met Ser Gln
       210                 215                 220
   gct acc tac ctt gtc ttc cac ggg tgt gat aag gtg tcg ctt cgt atc      720
   Ala Thr Tyr Leu Val Phe His Gly Cys Asp Lys Val Ser Leu Arg Ile
   225                 230                 235                 240
   acg att gtg tac ttt gtg tcc ctt ttg agt ttg ttc ttc ctt ttt gct      768
   Thr Ile Val Tyr Phe Val Ser Leu Leu Ser Leu Phe Phe Leu Phe Ala
                   245                 250                 255
   cag ttc ttt gtg caa tca tac atg gca ccc aaa aag aag aag agt gct      816
   Gln Phe Phe Val Gln Ser Tyr Met Ala Pro Lys Lys Lys Lys Ser Ala
               260                 265                 270
   tag                                                                  819
<210> 8
   <211> 272
   <212> PRT
   <213> Talassiosira pseudonana
<400> 8
   Met Asp Ala Tyr Asn Ala Ala Met Asp Lys Ile Gly Ala Ala Ile Ile
   1               5                   10                  15
   Asp Trp Ser Asp Pro Asp Gly Lys Phe Arg Ala Asp Arg Glu Asp Trp
               20                  25                  30
   Trp Leu Cys Asp Phe Arg Ser Ala Ile Thr Ile Ala Leu Ile Tyr Ile
           35                  40                  45
   Ala Phe Val Ile Leu Gly Ser Ala Val Met Gln Ser Leu Pro Ala Met
       50                  55                  60
   Asp Pro Tyr Pro Ile Lys Phe Leu Tyr Asn Val Ser Gln Ile Phe Leu
   65                  70                  75                  80
   Cys Ala Tyr Met Thr Val Glu Ala Gly Phe Leu Ala Tyr Arg Asn Gly
                   85                  90                  95
   Tyr Thr Val Met Pro Cys Asn His Phe Asn Val Asn Asp Pro Pro Val
               100                 105                 110
   Ala Asn Leu Leu Trp Leu Phe Tyr Ile Ser Lys Val Trp Asp Phe Trp
           115                 120                 125
   Asp Thr Ile Phe Ile Val Leu Gly Lys Lys Trp Arg Gln Leu Ser Phe
       130                 135                 140
   Leu His Val Tyr His His Thr Thr Ile Phe Leu Phe Tyr Trp Leu Asn
   145                 150                 155                 160
   Ala Asn Val Leu Tyr Asp Gly Asp Ile Phe Leu Thr Ile Leu Leu Asn
                   165                 170                 175
   Gly Phe Ile His Thr Val Met Tyr Thr Tyr Tyr Phe Ile Cys Met His
               180                 185                 190
   Thr Lys Asp Ser Lys Thr Gly Lys Ser Leu Pro Ile Trp Trp Lys Ser
           195                 200                 205
   Ser Leu Thr Ala Phe Gln Leu Leu Gln Phe Thr Ile Met Met Ser Gln
       210                 215                 220
   Ala Thr Tyr Leu Val Phe His Gly Cys Asp Lys Val Ser Leu Arg Ile
   225                 230                 235                 240
   Thr Ile Val Tyr Phe Val Ser Leu Leu Ser Leu Phe Phe Leu Phe Ala
                   245                 250                 255
   Gln Phe Phe Val Gln Ser Tyr Met Ala Pro Lys Lys Lys Lys Ser Ala
               260                 265                 270
<210> 9
   <211> 1086
   <212> DNA
   <213> Phytophthora infestans
<220>
   <221> CDS
   <222> (1)..(1086)
   <223> Omega-3-Desaturase
<400> 9
   atg gcg acg aag gag gcg tat gtg ttc ccc act ctg acg gag atc aag      48
   Met Ala Thr Lys Glu Ala Tyr Val Phe Pro Thr Leu Thr Glu Ile Lys
   1               5                   10                  15
   cgg tcg cta cct aaa gac tgt ttc gag gct tcg gtg cct ctg tcg ctc      96
   Arg Ser Leu Pro Lys Asp Cys Phe Glu Ala Ser Val Pro Leu Ser Leu
               20                  25                  30
   tac tac acc gtg cgt tgt ctg gtg atc gcg gtg gct cta acc ttc ggt      144
   Tyr Tyr Thr Val Arg Cys Leu Val Ile Ala Val Ala Leu Thr Phe Gly
           35                  40                  45
   ctc aac tac gct cgc gct ctg ccc gag gtc gag agc ttc tgg gct ctg      192
   Leu Asn Tyr Ala Arg Ala Leu Pro Glu Val Glu Ser Phe Trp Ala Leu
       50                  55                  60
   gac gcc gca ctc tgc acg ggc tac atc ttg ctg cag ggc atc gtg ttc      240
   Asp Ala Ala Leu Cys Thr Gly Tyr Ile Leu Leu Gln Gly Ile Val Phe
   65                  70                  75                  80
   tgg ggc ttc ttc acg gtg ggc cac gat gcc ggc cac ggc gcc ttc tcg      288
   Trp Gly Phe Phe Thr Val Gly His Asp Ala Gly His Gly Ala Phe Ser
                   85                  90                  95
   cgc tac cac ctg ctt aac ttc gtg gtg ggc act ttc atg cac tcg ctc      336
   Arg Tyr His Leu Leu Asn Phe Val Val Gly Thr Phe Met His Ser Leu
               100                 105                 110
   atc ctc acg ccc ttc gag tcg tgg aag ctc acg cac cgt cac cac cac      384
   Ile Leu Thr Pro Phe Glu Ser Trp Lys Leu Thr His Arg His His His
           115                 120                 125
   aag aac acg ggc aac att gac cgt gac gag gtc ttc tac ccg caa cgc      432
   Lys Asn Thr Gly Asn Ile Asp Arg Asp Glu Val Phe Tyr Pro Gln Arg
       130                 135                 140
   aag gcc gac gac cac ccg ctg tct cgc aac ctg att ctg gcg ctc ggg      480
   Lys Ala Asp Asp His Pro Leu Ser Arg Asn Leu Ile Leu Ala Leu Gly
   145                 150                 155                 160
   gca gcg tgg ctc gcc tat ttg gtc gag ggc ttc cct cct cgt aag gtc      528
   Ala Ala Trp Leu Ala Tyr Leu Val Glu Gly Phe Pro Pro Arg Lys Val
                   165                 170                 175
   aac cac ttc aac ccg ttc gag cct ctg ttc gtg cgt cag gtg tca gct      576
   Asn His Phe Asn Pro Phe Glu Pro Leu Phe Val Arg Gln Val Ser Ala
               180                 185                 190
   gtg gta atc tct ctt ctc gcc cac ttc ttc gtg gcc gga ctc tcc atc      624
   Val Val Ile Ser Leu Leu Ala His Phe Phe Val Ala Gly Leu Ser Ile
           195                 200                 205
   tat ctg agc ctc cag ctg ggc ctt aag acg atg gca atc tac tac tat      672
   Tyr Leu Ser Leu Gln Leu Gly Leu Lys Thr Met Ala Ile Tyr Tyr Tyr
       210                 215                 220
   gga cct gtt ttt gtg ttc ggc agc atg ctg gtc att acc acc ttc cta      720
   Gly Pro Val Phe Val Phe Gly Ser Met Leu Val Ile Thr Thr Phe Leu
   225                 230                 235                 240
   cac cac aat gat gag gag acc cca tgg tac gcc gac tcg gag tgg acg      768
   His His Asn Asp Glu Glu Thr Pro Trp Tyr Ala Asp Ser Glu Trp Thr
                   245                 250                 255
   tac gtc aag ggc aac ctc tcg tcc gtg gac cga tcg tac ggc gcg ctc      816
   Tyr Val Lys Gly Asn Leu Ser Ser Val Asp Arg Ser Tyr Gly Ala Leu
               260                 265                 270
   att gac aac ctg agc cac aac atc ggc acg cac cag atc cac cac ctt      864
   Ile Asp Asn Leu Ser His Asn Ile Gly Thr His Gln Ile His His Leu
       275                 280                 285
   ttc cct atc att ccg cac tac aaa ctc aag aaa gcc act gcg gcc ttc      912
   Phe Pro Ile Ile Pro His Tyr Lys Leu Lys Lys Ala Thr Ala Ala Phe
       290                 295                 300
   cac cag gct ttc cct gag ctc gtg cgc aag agc gac gag cca att atc      960
   His Gln Ala Phe Pro Glu Leu Val Arg Lys Ser Asp Glu Pro Ile Ile
   305                 310                 315                 320
   aag gct ttc ttc cgg gtt gga cgt ctc tac gca aac tac ggc gtt gtg     1008
   Lys Ala Phe Phe Arg Val Gly Arg Leu Tyr Ala Asn Tyr Gly Val Val
                   325                 330                 335
   gac cag gag gcg aag ctc ttc acg cta aag gaa gcc aag gcg gcg acc     1056
   Asp Gln Glu Ala Lys Leu Phe Thr Leu Lys Glu Ala Lys Ala Ala Thr
               340                 345                 350
   gag gcg gcg gcc aag acc aag tcc acg taa                             1086
   Glu Ala Ala Ala Lys Thr Lys Ser Thr
       355                 360
<210> 10
   <211> 361
   <212> PRT
   <213> Phytophthora infestans
<400> 10
   Met Ala Thr Lys Glu Ala Tyr Val Phe Pro Thr Leu Thr Glu Ile Lys
   1               5                   10                  15
   Arg Ser Leu Pro Lys Asp Cys Phe Glu Ala Ser Val Pro Leu Ser Leu
               20                  25                  30
   Tyr Tyr Thr Val Arg Cys Leu Val Ile Ala Val Ala Leu Thr Phe Gly
           35                  40                  45
   Leu Asn Tyr Ala Arg Ala Leu Pro Glu Val Glu Ser Phe Trp Ala Leu
       50                  55                  60
   Asp Ala Ala Leu Cys Thr Gly Tyr Ile Leu Leu Gln Gly Ile Val Phe
   65                  70                  75                  80
   Trp Gly Phe Phe Thr Val Gly His Asp Ala Gly His Gly Ala Phe Ser
                   85                  90                  95
   Arg Tyr His Leu Leu Asn Phe Val Val Gly Thr Phe Met His Ser Leu
               100                 105                 110
   Ile Leu Thr Pro Phe Glu Ser Trp Lys Leu Thr His Arg His His His
           115                 120                 125
   Lys Asn Thr Gly Asn Ile Asp Arg Asp Glu Val Phe Tyr Pro Gln Arg
   130 135 140
   Lys Ala Asp Asp His Pro Leu Ser Arg Asn Leu Ile Leu Ala Leu Gly
   145 150 155 160
   Ala Ala Trp Leu Ala Tyr Leu Val Glu Gly Phe Pro Pro Arg Lys Val
   165 170 175
   Asn His Phe Asn Pro Phe Glu Pro Leu Phe Val Arg Gln Val Ser Ala
   180 185 190
   Val Val Ile Ser Leu Leu Ala His Phe Phe Val Ala Gly Leu Ser Ile
   195 200 205
   Tyr Leu Ser Leu Gln Leu Gly Leu Lys Thr Met Ala Ile Tyr Tyr Tyr
   210 215 220
   Gly Pro Val Phe Val Phe Gly Ser Met Leu Val Ile Thr Thr Phe Leu
   225 230 235 240
   His His Asn Asp Glu Glu Thr Pro Trp Tyr Ala Asp Ser Glu Trp Thr
   245 250 255
   Tyr Val Lys Gly Asn Leu Ser Ser Val Asp Arg Ser Tyr Gly Ala Leu
   260 265 270
   Ile Asp Asn Leu Ser His Asn Ile Gly Thr His Gln Ile His His Leu
   275 280 285
   Phe Pro Ile Ile Pro His Tyr Lys Leu Lys Lys Ala Thr Ala Ala Phe
   290 295 300
   His Gln Ala Phe Pro Glu Leu Val Arg Lys Ser Asp Glu Pro Ile Ile
   305 310 315 320
   Lys Ala Phe Phe Arg Val Gly Arg Leu Tyr Ala Asn Tyr Gly Val Val
   325 330 335
   Asp Gln Glu Ala Lys Leu Phe Thr Leu Lys Glu Ala Lys Ala Ala Thr
   340 345 350
   Glu Ala Ala Ala Lys Thr Lys Ser Thr
   355 360
<210> 11
   <211> 755
   <212> DNA
   <213> 35S-Virus
<220>
   <221> promoter
   <222> (1)..(755)
   <223>
<400> 11
   cctgcaggtc aacatggtgg agcacgacac acttgtctac tccaaaaata tcaaagatac     60
   agtctcagaa gaccaaaggg caattgagac ttttcaacaa agggtaatat ccggaaacct    120
   cctcggattc cattgcccag ctatctgtca ctttattgtg aagatagtgg aaaaggaagg    180
   tggctcctac aaatgccatc attgcgataa aggaaaggcc atcgttgaag atgcctctgc    240
   cgacagtggt cccaaagatg gacccccacc cacgaggagc atcgtggaaa aagaagacgt    300
   tccaaccacg tcttcaaagc aagtggattg atgtgataac atggtggagc acgacacact    360
   tgtctactcc aaaaatatca aagatacagt ctcagaagac caaagggcaa ttgagacttt    420
   tcaacaaagg gtaatatccg gaaacctcct cggattccat tgcccagcta tctgtcactt    480
   tattgtgaag atagtggaaa aggaaggtgg ctcctacaaa tgccatcatt gcgataaagg    540
   aaaggccatc gttgaagatg cctctgccga cagtggtccc aaagatggac ccccacccac    600
   gaggagcatc gtggaaaaag aagacgttcc aaccacgtct tcaaagcaag tggattgatg    660
   tgatatctcc actgacgtaa gggatgacgc acaatcccac tatccttcgc aagacccttc    720
   ctctatataa ggaagttcat ttcatttgga gagga                               755
<210> 12
   <211> 211
   <212> DNA
   <213> 35S-Virus
<220>
   <221> terminator
   <222> (1)..(211)
   <223>
<400> 12
   agtccgcaaa aatcaccagt ctctctctac aaatctatct ctctctattt ttctccagaa     60
   taatgtgtga gtagttccca gataagggaa ttagggttct tatagggttt cgctcatgtg    120
   ttgagcatat aagaaaccct tagtatgtat ttgtatttgt aaaatacttc tatcaataaa    180
   atttctaatt cctaaaacca aaatccagtg a                                   211

## Beschreibung

Die vorliegende Erfindung betrifft ein Verfahren zur Herstellung von Eicosapentaensäure oder Arachidonsäure und Eicosapentaensäure im vegetativen Gewebe von transgenen Nutzpflanzen, indem Nukleinsäuren in die Pflanze eingebracht werden, die für Polypeptide mit Δ-6-Desaturase-, Δ-6-Elongase- und A-5-Desaturaseaktivität codieren. Vorteilhaft wird in den Nutzpflanzen weiterhin ein Gen, das für eine ω-3-Desaturase codiert, exprimiert. In einer weiteren vorteilhaften Ausführungsform des Verfahrens können weitere Nukleinsäuresequenzen, die für Polypeptide der Biosynthese des Fettsäure- oder Lipidstoffwechels codieren, in den Pflanzen exprimiert werden. Besonders vorteilhaft sind hierfür die Nukleinsäuresequenzen, die für eine Δ-8-Desaturase- ,Δ-12-Desaturase-, eine Δ-15-Desaturase-, Δ-4-Desaturase-, Δ-9-Elongase- und/oder Δ-5-Elongaseaktivität codieren.

Fettsäuren und Triacylglyceride haben eine Vielzahl von Anwendungen in der Lebensmittelindustrie, der Tierernährung, der Kosmetik und im Pharmabereich. Je nachdem, ob es sich um freie gesättigte und ungesättigte Fettsäuren oder um Triacylglyceride mit einem erhöhten Gehalt an gesättigten oder ungesättigten Fettsäuren handelt, sind sie für die unterschiedlichsten Anwendungen geeignet. Mehrfach ungesättigte w-3-Fettsäuren und w-6-Fettsäuren sind ein wichtiger Bestandteile in der tierischen und menschlichen Nahrung.

Mehrfach ungesättigte langkettige w-3-Fettsäuren wie Eicosapentaensäure (= EPA, C20:^{Δ5,8,11,14,17}) oder Docosahexaensäure (= DHA, C22:6^{Δ4,7,10,13,16,19}) sind wichtige Komponenten der menschlichen Ernährung aufgrund ihrer verschiedenen Rollen in der Gesundheit, die Aspekte wie die Entwicklung des kindlichen Gehirns, der Funktionalität des Auges, der Synthese von Hormonen und anderer Signalstoffe, sowie die Vorbeugung von Herz-Kreislauf-Beschwerden, Krebs und Diabetes umfassen (Poulos, A Lipids 30:1-14,1995; Horrocks, LA und Yeo YK Pharmacol Res 40:211-225, 1999). Es besteht aus diesem Grund ein Bedarf an der Produktion mehrfach ungesättigter langkettiger Fettsäuren.

Aufgrund der heute üblichen Zusammensetzung der menschlichen Nahrung ist ein Zusatz von mehrfach ungesättigten w-3-Fettsäuren, die bevorzugt in Fischölen vorkommen, zur Nahrung besonders wichtig. So werden beispielsweise mehrfach ungesättigte Fettsäuren wie Docosahexaensäure (= DHA, C22:6^{Δ4,7,10,13,16,19}) oder Eisosapentaensäure (= EPA, C20:5^{Δ5,8,11,14,17}) Babynahrung zur Erhöhung des Nährwertes zugesetzt.

Im folgenden werden mehrfach ungesättigte Fettsäuren als PUFA, PUFAs, LCPUFA oder LCPUFAs bezeichnet (**p**oly **u**nsaturated **f**atty **a**cids, **PUFA**, mehrfach ungesättigte Fettsäuren; **l**ong **c**hain **p**oly **u**nsaturated **f**atty **a**cids, **LCPUFA**, langkettige mehrfach ungesättigte Fettsäuren).

Hauptsächlich werden die verschiedenen Fettsäuren und Triglyceride aus Mikroorganismen wie Mortierella oder Schizochytrium oder aus Öl-produzierenden Pflanzen wie Soja, Raps, Algen wie Crypthecodinium oder Phaeodactylum und weiteren gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung hergestellt. Sehr langkettige mehrfach ungesättigte Fettsäuren wie EPA, Dihomo-γ-linolensäure (C20:3^{Δ8,11,14}) oder Arachidonsäure (= ARA, C20:4^{Δ5,8,11,14}) werden in Ölfruchtpflanzen wie Raps, Soja, Sonnenblume, Färbersaflor nicht synthetisiert. Übliche natürliche Quellen für diese Fettsäuren sind Fische wie Hering, Lachs, Sardine, Goldbarsch, Aal, Karpfen, Forelle, Heilbutt, Makrele, Zander oder Thunfisch oder Algen.

Je nach Anwendungszweck werden Öle mit gesättigten oder ungesättigten Fettsäuren bevorzugt. So werden z.B. in der humanen Ernährung Lipide mit ungesättigten Fettsäuren speziell mehrfach ungesättigten Fettsäuren bevorzugt. Den mehrfach ungesättigten ω-3-Fettsäuren wird dabei ein positiver Effekt auf den Cholesterinspiegel im Blut und damit auf die Möglichkeit der Prävention einer Herzerkrankung zugeschrieben. Durch Zugabe dieser w-3-Fettsäuren zur Nahrung kann das Risiko einer Herzerkrankung, eines Schlaganfalls oder von Bluthochdruck deutlich verringert werden. Auch entzündliche speziell chronisch entzündliche Prozesse im Rahmen immunologischer Erkrankungen wie rheumatroider Arthritis lassen sich durch w-3-Fettsäuren positiv beeinflussen. Sie werden deshalb Lebensmitteln speziell diätischen Lebensmitteln zugegeben oder finden in Medikamenten Anwendung.

ω-3- und w-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, den Thromoxanen und Leukotrienen, die sich von der Arachidonsäure und der Eicosapentaensäure ableiten. Eicosanoide (sog. PG₂-Serie), die aus w-6-Fettsäuren gebildet werden fördern in der Regel Entzündungsreaktionen, während Eicosanoide (sog. PG₃-Serie) aus w-3-Fettsäuren geringe oder keine entzündungsfördernde Wirkung haben.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase in WO 94/11516 wird eine Δ-12-Desaturase beansprucht. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturasen werden in WO 93/06712, US 5,614,393, US5614393, WO 96/21022, WO00/21557 und WO 99/27111 beschrieben und auch die Anwendung zur Produktion in transgenen Organismen beschrieben wie in WO98/46763 WO98/46764, WO9846765. Dabei wird auch die Expression verschiedener Desaturasen wie in WO99/64616 oder WO98/46776 und Bildung polyungesättigter Fettsäuren beschrieben und beansprucht.

Die polyungesättigten Fettsäuren können entsprechend ihrem Desaturierungsmuster in zwei große Klassen, in w-6- oder w-3-Fettsäuren eingeteilt werden, die metabolisch und funktionell unterschiedlich Aktivitäten haben. Die Synthese der w-6- oder w-3-Fettsäuren Arachidonsäure und EPA über verschiedene Biosynthesewege in Mikroorganismen wie Hefen werden beispielsweise in WO0159128, WO0012720, WO02077213 und WO0208401 beschrieben.

Als Ausgangsprodukt für den ω-6-Stoffwechselweg fungiert die Fettsäure Linolsäure (18:2^{Δ9,12}), während der w-3-Weg über Linolensäure (18:3^{Δ9,12,15}) abläuft. Linolensäure wird dabei durch Aktivität einer ω-3-Desaturase gebildet (Tocher et al. 1998, Prog. Lipid Res. 37, 73-117 ; Domergue et al. 2002, Eur. J. Biochem. 269, 4105-4113).

Säugetiere und damit auch der Mensch verfügen über keine entsprechende Desaturaseaktivität (Δ-12- und ω-3-Desaturase) und müssen diese Fettsäuren (essentielle Fettsäuren) über die Nahrung aufnehmen. Über die Abfolge von Desaturase- und Elongase-Reaktionen werden dann aus diesen Vorstufen die physiologisch wichtigen polyungesättigten Fettsäuren Arachidonsäure (= ARA, 20:4^{Δ5,8,11,14}), eine ω-6-Fettsäure, und die ω-3-Fettsäure Eicosapentaensäure (= EPA, 20:5^{Δ5,8,11,14,17}) synthetisiert. Die Applikation von ω-3-Fettsäuren zeigt dabei die wie oben beschrieben therapeutische Wirkung bei der Behandlung von Herz-Kreislaufkrankheiten (Shimikawa 2001, World Rev. Nutr. Diet. 88, 100-108), Entzündungen (Calder 2002, Proc. Nutr. Soc. 61, 345-358) und Arthridis (Cleland und James 2000, J. Rheumatol. 27, 2305-2307).

Die bisher bekannten Verfahren zur Herstellung von Arachidonsäure und/oder Eicosapenatensäure besitzen einige Nachteile. In der Regel werden beide Fettsäuren als ein Gemisch in den Verfahren erhalten. Das einzige bekannte Verfahren zur Herstellung von Arachidonsäure mit geringen Anteilen an EPA ist ein pilzliches, fermentatives Verfahren. Dies ist eine Ölquelle, die aus ernährungsphysiologischer Sicht suboptimal ist, da sie Fettsäuren enthält, die in der menschlichen Nahrung sonst nicht vorkommen. Als weitere Arachidonsäurequelle kommen Eilipide in Frage. Diese enthalten allerdings hohe Phospholipidanteile sowie Cholesterin, die in für eine breite Verwendung in Nahrungsmitteln eher nachteilig sind.

Höhere Pflanzen enthalten mehrfach ungesättigte Fettsäuren wie Linolsäure (C18:2) und Linolensäure (C18:3). ARA und/oder EPA kommen im Samenöl höherer Pflanzen gar nicht oder nur in Spuren vor (E. Ucciani: Nouveau Dictionnaire des Huiles Végétales. Technique & Documentation - Lavoisier, 1995. ISBN: 2-7430-0009-0). Es wäre jedoch vorteilhaft, in höheren Pflanzen, bevorzugt in Ölsaaten wie Raps, Lein, Sonnenblume und Soja, LCPUFAs herzustellen, da auf diese Weise große Mengen qualitativ hochwertiger LCPUFAs für die Lebensmittelindustrie, die Tierernährung und für pharmazeutische Zwecke kostengünstig gewonnen werden können.

Erste transgene Pflanzen, die Gene kodierend für Enzyme der LCPUFA-Biosynthese enthalten und exprimieren und LCPUFAs produzieren wurden beispielsweise in DE 102 19 203 (Verfahren zur Herstellung mehrfach ungesättigter Fettsäuren in Pflanzen) erstmals beschrieben. Diese Pflanzen produzieren allerdings LCPUFAs in Mengen, die für eine Aufarbeitung der in den Pflanzen enthaltenen Öle noch weiter optimiert werden müssen. Wu et al. gelang es, in Samen von Brassica juncea bis zu 25% Arachidonsäure und bis zu 15% Eicosapentaensäure jeweils bezogen auf den Gesamtgehalt an Fettsäuren in den Samen zu produzieren (G. Wu et al., Stepwise engineering to produce high yields of very long-chain polyunsaturated fatty acids in plants, Nature Biotechnology 23, 1013-1017 (2005)).

Allerdings sind Samen als Tierfutter oder Nahrungsmittel ungeeignet. Zudem werden bei einer ausschließlichen Produktion der Fettsäuren in den Samen die meisten Teile der Pflanzen ungenutzt verworfen.

Um eine weitere Anreicherung der Nahrung und/oder des Futters mit diesen mehrfach ungesättigten Fettsäuren zu ermöglichen, besteht daher nach wie vor ein großer Bedarf an einem einfachen, kostengünstigen Verfahren zur Herstellung von Arachidonsäure und/oder Eicosapentaensäure in Nutzpflanzen.

Es bestand daher die Aufgabe ein einfaches, kostengünstiges, wirtschaftliches Verfahren zur Herstellung von Arachidonsäure und/oder Eicosapentaensäure im vegetativen Gewebe von Nutzpflanzen zu entwickeln, dass die vorgenannten Nachteile nicht hat. Außderdem sollte ein solches Verfahren die Synthese der Fettsäuren preiswert in nahezu beliebigen Mengen ermöglichen. Neben den Wertprodukten Arachidonsäure und/oder Eicosapentaensäure sollten möglichst wenig andere PUFAs vorteilhaft nur entweder w-3- oder ω-6-Fettsäuren vorteilhaft nur ω-3-Fettsäuren enthalten.

Diese Aufgabe wurde durch das erfindungsgemäße Verfahren zur Herstellung von Eicosapentaensäure oder Arachidonsäure und Eicosapentaensäure in transgenen Nutzpflanzen mit einem Gehalt von mindestens 4 Gew.-% bezogen auf den Gesamtlipidgehalt der transgenen Nutzpflanze gelöst, dadurch gekennzeichnet, dass es folgende Verfahrensschritte umfasst:
a) Einbringen mindestens einer Nukleinsäuresequenz in die Nutzpflanze, die für eine Δ-6-Desaturase wie unten beschrieben codiert, und
b) Einbringen mindestens einer Nukleinsäuresequenz in die Nutzpflanze, die für eine Δ-6-Elongase wie unten beschrieben codiert, und
c) Einbringen mindestens einer Nukleinsäuresequenz in die Nutzpflanze, die für eine Δ-5-Desaturase wie unten beschrieben codiert, und
wobei die unter den Schritten (a) bis (c) genannten Sequenzen mit Hilfe mindestens eines konstitutiven CaMV/35S-Promotors, der die Expression der besagten Nukleinsäuresequenzen im vegetativen Gewebe der Pflanzen ermöglicht, und eines Terminators in den Pflanzen exprimiert werden.

Die vorgenannten Nukleinsäuresequenzen, die im erfindungsgemäßen Verfahren verwendet werden und für Polypeptide mit Δ-6-Desaturase-, Δ-6-Elongase-, und Δ-5-Desaturaseaktivität codieren, sind ausgewählt aus der Gruppe bestehend aus:
a) einer Nukleinsäuresequenz mit der in SEQ ID NO: 1, SEQ ID NO: 3, SEQ ID NO: 5 oder SEQ ID NO: 7 dargestellten Sequenz, oder
b) Nukleinsäuresequenzen, die sich als Ergebnis des degenerierten genetischen Codes von den in SEQ ID NO: 2, SEQ ID NO: 4, SEQ ID NO: 6 oder SEQ ID NO: 8 dargestellten Aminosäuresequenzen ableiten lassen, und
c) Derivate der in SEQ ID NO: 1, SEQ ID NO: 3, SEQ ID NO: 5 oder SEQ ID NO: 7 dargestellten Nukleinsäuresequenz, die für Polypeptide codieren, die auf Aminosäureebene mindestens 70% Identität mit SEQ ID NO: 2, SEQ ID NO: 4, SEQ ID NO: 6 oder SEQ ID NO: 8 aufweisen und eine Δ-6-Desaturase-, Δ-6-Elongase-, oder Δ-5-Desaturaseaktivität haben.

In einer bevorzugten Ausführungsform des Verfahrens wird in die Nutzpflanzen zusätzlich eine Nukleinsäuresequenz, die für eine ω-3-Desaturase codiert eingebracht. Diese ω-3-Desaturase codierende Nukleinsäuresequenz wird vorteilhaft ausgewählt aus der Gruppe bestehend aus:
a) einer Nukleinsäuresequenz mit der in SEQ ID NO: 9 dargestellten Sequenz, oder
b) Nukleinsäuresequenzen, die sich als Ergebnis des degenerierten genetischen Codes von den in SEQ ID NO: 10 dargestellten Aminosäuresequenzen ableiten lassen, oder
c) Derivate der in SEQ ID NO: 9 dargestellten Nukleinsäuresequenz, die für Polypeptide codieren, die auf Aminosäureebene mindestens 60% Identität mit SEQ ID NO: 10 aufweisen und eine ω-3-Desaturaseaktivität haben.

Vorteilhaft enthalten die im erfindungsgemäßen Verfahren hergestellten mehrfach ungesättigten Fettsäuren EPA oder ARA und EPA weitere LCPUFAS der w-3- oder w-6-Fettsäurereihe mit mindestens zwei vorteilhaft drei, vier oder fünf Doppelbindungen. Im Verfahren hergestellte Fettsäuren haben vorteilhaft 18- oder 20-C-Atome in der Fettsäurekette, bevorzugt enthalten die Fettsäuren 20 Kohlenstoffatome in der Fettsäurekette. Vorteilhaft werden gesättigte Fettsäuren mit den im Verfahren verwendeten Nukleinsäuren, die für Δ-6-Desaturasen, Δ-6-Elongasen oder Δ-5-Desaturasen und/oder ω-3-Desaturasen codieren, wenig oder gar nicht vorteilhaft gar nicht umgesetzt. Unter wenig ist zu verstehen, das im Vergleich zu mehrfach ungesättigten Fettsäuren die gesättigten Fettsäuren mit weniger als 5 % der Aktivität, vorteilhaft weniger als 3 %, besonders vorteilhaft mit weniger als 2 %, ganz besonders bevorzugt mit weniger als 1; 0,5; 0,25 oder 0,125 % umgesetzt werden. Diese neben den im Verfahren hergestellten Fettsäuren EPA oder ARA und EPA können als einzelne Fettsäuren zusätzlich im Verfahren hergestellt werden oder in einem Fettsäuregemisch vorliegen.

Vorteilhaft werden die vorgenannten Nukleinsäuresequenzen, die für Δ-6-Desaturasen, Δ-6-Elongasen oder Δ-5-Desaturasen und/oder ω-3-Desaturasen codieren, in Kombination mit weiteren Genen des Fettsäure- und/oder Lipidstoffwechsels wie z.B. Nukleinsäuresequenzen, die für Polypeptide mit Δ-12-Desaturase-, Δ-9-Elongase-, Δ-8-Desaturase-, Δ-5-Elongase- und/oder Δ-4-Desaturaseaktivität codieren, exprimiert.

Die im erfindungsgemäßen Verfahren verwendeten Nukleinsäuren werden im vegetativen Gewebe (= somatischem Gewebe) exprimiert. Unter vegetativen Gewebe ist im Sinne dieser Erfindung zu verstehen, dass das Gewebe dadurch gekennzeichnet ist, das es sich durch mitotische Teilungen vermehrt. Derartiges Gewebe entsteht auch durch asexuelle Fortpflanzung (= Apomixis) und Vermehrung. Von Vermehrung spricht man dann, wenn sich die Zahl der Individuen in aufeinander folgenden Generationen erhöht. Diese durch asexuelle Vermehrung entstandenen Individuen sind mit ihren Eltern weitestgehend identisch. Beispiele für derartige Gewebe sind Blatt, Blüte, Wurzel, Stengel, oberirdische oder unterirdische Ausläufer (Seitensprosse, Stolonen), Rhizome, Knospen, Knollen wie Wurzelknollen oder Ausläuferknollen, Zwiebel, Brutkörper, Brutknospen, Bulbillen oder Turione. Derartige Gewebe können auch durch unechte, echte oder durch den Mensch verursachte Viviparie entstehen. Aber auch Samen, die durch Agamospermie, wie sie für Asteraceae, Poaceae oder Rosaceae typisch sind, entstanden sind, gehören zu den vegetativen Geweben, in denen vorteilhaft die Expression stattfindet. Zu einem geringeren Teil oder gar nicht werden die im erfindungsgemäßen Verfahren verwendeten Nukleinsäuren im generativen Gewebe (Keimbahngewebe) exprimiert. Beispiele für derartige Gewebe sind Gewebe, die durch geschlechtliche Fortpflanzung, d.h. meiotische Zellteilungen entstehen, wie z.B. Samen, die durch geschlechtliche Prozesse entstanden sind. Unter zu einem geringen Teil ist zu verstehen, das im Vergleich zum vegetativen Gewebe die Expression gemessen auf RNA und/oder Proteinebene weniger als 5 %, vorteilhaft weniger als 3 %, besonders vorteilhaft mit weniger als 2 %, ganz besonders bevorzugt weniger als 1; 0,5; 0,25 oder 0,125 % beträgt. Unter generative Gewebe ist im Sinne dieser Erfindung zu verstehen, dass das Gewebe sich durch meiotische Teilung bildet.

Die im Verfahren hergestellten mehrfach ungesättigten Fettsäuren sind vorteilhaft in Phospholipiden und/oder Triacylglyceriden gebunden, können aber auch als freie Fettsäuren oder aber gebunden in Form anderer Fettsäureester in den Organismen vorkommen. Dabei können sie als "Reinprodukte" oder aber vorteilhaft in Form von Mischungen verschiedener Fettsäuren oder Mischungen unterschiedlicher Phospholipide wie Phosphatidylglycol, Phosphatidylcholin, Phosphatidylethanolamin und/oder Phosphatidylserin und/oder Triacylglyceride, Monoacylglyceride und/oder Diacylglyceride vorliegen. Vorteilhaft liegen die im Verfahren hergestellten LCPUFAS EPA oder ARA und EPA im Phosphatidylcholin und/oder Phosphatidylethanolamin und/oder in den Triacylglyceriden vor. Die Triacylglyceride können außerdem noch weitere Fettsäuren enthalten wie kurzkettige Fettsäuren mit 4 bis 6 C-Atomen, mittelkettige Fettsäuren mit 8 bis 12 C-Atomen oder langkettigen Fettsäuren mit 14 bis 24 C-Atomen, bevorzugt enthalten sie langkettige Fettsäuren besonders bevorzugt sind die langkettigen Fettsäuren LCPUFAs von C₁₈- oder C₂₀-Fettsäuren.

Im erfindungsgemäßen Verfahren werden vorteilhaft Fettsäureester mit mehrfach ungesättigten C₁₈-, und/oder C₂₀-Fettsäuremolekülen mit mindestens zwei Doppelbindungen im Fettsäureester, vorteilhaft mit mindestens drei, vier oder fünf Doppelbindungen im Fettsäureester, besonders vorteilhaft mit vier oder fünf Doppelbindungen im Fettsäureester hergestellt und führen vorteilhaft zur Synthese von Linolsäure (= LA, C18:2^{Δ9,12}), y-Linolensäure (= GLA, C18:3^{Δ6,9,12}), Stearidonsäure (= SDA, C18:4 ^{Δ6,9,12,15}), Dihomo-y-Linolensäure (= DGLA, 20:3^{Δ8,11,14}), Arachidonsäure (ARA, C20:4 ^{Δ5,8,11,14}) oder Eicosapentaensäure (EPA, C20:5^{Δ5,8,11,14,17}) oder deren Mischungen, bevorzugt EPA oder ARA und EPA. Ganz besonders bevorzugt wird die w-3-Fettsäure EPA hergestellt.

Die Fettsäureester mit mehrfach ungesättigten C₁₈- und/oder C₂₀-Fettsäuremolekülen können aus den Nutzpflanzen, die für die Herstellung der Fettsäureester verwendet wurden, in Form eines Öls oder Lipids beispielsweise in Form von Verbindungen wie Sphingolipide, Phosphoglyceride, Lipide, Glycolipide wie Glycosphingolipide, Phospholipide wie Phosphatidylethanolamin, Phosphatidylcholin, Phosphatidylserin, Phosphatidylglycerol, Phosphatidylinositol oder Diphosphatidylglycerol, Monoacylglyceride, Diacylglyceride, Triacylglyceride oder sonstige Fettsäureester wie die AcetylCoenzymA-Ester, die die mehrfach ungesättigten Fettsäuren mit mindestens zwei, drei oder vier bevorzugt drei oder vier Doppelbindungen enthalten, isoliert werden, vorteilhaft werden sie in der Form ihrer Diacylglyceride, Triacylglyceride und/oder in Form des Phosphatidylester isoliert, besonders bevorzugt in der Form der Triacylglyceride, Phosphatidylcholin und/oder Phosphatidylserin. Neben diesen Estern sind die mehrfach ungesättigten Fettsäuren auch als freie Fettsäuren oder gebunden in anderen Verbindungen in den Pflanzen enthalten. In der Regel liegen die verschiedenen vorgenannten Verbindungen (Fettsäureester und frei Fettsäuren) in den Organismen in einer ungefähren Verteilung von 80 bis 90 Gew.-% Triglyceride, 2 bis 5 Gew.-% Diglyceride, 5 bis 10 Gew.-% Monoglyceride, 1 bis 5 Gew.-% freie Fettsäuren, 2 bis 8 Gew.-% Phospholipide vor, wobei sich die Summe der verschiedenen Verbindungen zu 100 Gew.-% ergänzt.

Im erfindungsgemäßen Verfahren werden die hergestellten LCPUFAs mit einem Gehalt von mindestens 4 Gew.-%, vorteilhaft von mindestens 5, 6, 7, 8,9 oder 10 Gew.-%, bevorzugt von mindestens 11, 12, 13, 14 oder 15 Gew.-%, besonders bevorzugt von mindestens 16, 17, 18, 19, oder 20 Gew.-%, ganz besonders bevorzugt von mindestens 25, 30, 35 oder 40 Gew.-% bezogen auf die gesamten Fettsäuren in den transgenen Pflanze hergestellt. Dabei sind die im erfindungsgemäßen Verfahren hergestellten Fettsäuren EPA oder ARA und EPA mit einem Gehalt von mindestens 10 Gew.-% bevorzugt von mindestens 11, 12, 13, 14 oder 15 Gew.-%, besonders bevorzugt von mindestens 16, 17, 18, 19, oder 20 Gew.-%, ganz besonders bevorzugt von mindestens 25, 26, 27, 28, 29, 30 oder 31 Gew.-%, am meisten bevorzugt von mindestens 32, 33, 34, 35, 36, 37, 38, 39, 40, 41, 42, 43, 44 oder 45 Gew.-% bezogen auf die gesamten Fettsäuren in den Triacylglyceriden und/oder Phosphatidylglyceriden vorteilhaft im Phosphatidylcholin und/oder Phosphatidylserin enthalten.

Vorteilhaft werden die Fettsäuren in gebundener Form hergestellt. Mit Hilfe der im erfindungsgemäßen Verfahren verwendeten Nukleinsäuren lassen sich diese ungesättigten Fettsäuren an sn1-, sn2- und/oder sn3-Position der vorteilhaft hergestellten Triglyceride bringen. Vorteilhaft sind mindestens 11 % der Triacylglyceride doppelt substituiert, das heißt an sn1- und sn2- oder sn2- und sn3-Position substituiert. Auch dreifach substituierte Triacylglyceride sind nachweisbar. Da im erfindungsgemäßen Verfahren von den Ausgangsverbindungen Linolsäure (C18:2) bzw. Linolensäure (C18:3) mehrere Reaktionsschritte durchlaufen werden, fallen die Endprodukte des Verfahrens wie beispielsweise Eicosapentaensäure (EPA) oder Arachidonsäure (ARA) und Eicosapentaensäure (EPA) nicht als absolute Reinprodukte an, es sind immer auch Spuren oder größere Mengen der Vorstufen im Endprodukt enthalten. Sind in der Ausgangspflanze beispielsweise sowohl Linolsäure als auch Linolensäure vorhanden, so liegen die Endprodukte wie EPA oder ARA und EPA als Mischungen vor. Die Vorstufen sollten vorteilhaft nicht mehr als 20 Gew.-%, bevorzugt nicht mehr als 15 Gew.-%, besonders bevorzugt nicht als 10 Gew.-%, ganz besonders bevorzugt nicht mehr als 5 Gew.-% bezogen auf die Menge des jeweilige Endprodukts betragen. Vorteilhaft werden in einer transgenen Pflanze als Endprodukte nur ARA oder EPA im erfindungsgemäßen Verfahren gebunden oder als freie Säuren hergestellt.

Fettsäureester bzw. Fettsäuregemische, die nach dem erfindungsgemäßen Verfahren hergestellt wurden, enthalten vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureester bzw. Fettsäuregemische bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopentendodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure), Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. In einer weiteren bevorzugten Form der Erfindung kommen diese vorgenannten Fettsäuren bezogen auf die Gesamtfettsäuren zu weniger als 0,9%; 0,8%; 0,7%; 0,6%; oder 0,5%, besonders bevorzugt zu weniger als 0,4%; 0,3%; 0,2%; 0,1% vor. Vorteilhaft enthalten die nach dem erfindungsgemäßen Verfahren hergestellten Fettsäureester bzw. Fettsäuregemische weniger als 0,1 % bezogen auf die Gesamtfettsäuren und/oder keine Buttersäure, kein Cholesterin, keine Clupanodonsäure (= Docosapentaensäure, C22:5^{Δ4,8,12,15,21}) sowie keine Nisinsäure (Tetracosahexaensäure, C23:6^{Δ3,8,12,15,18,21}),

Durch die im erfindungsgemäßen Verfahren verwendeten Nukleinsäuresequenzen kann eine Steigerung der Ausbeute an mehrfach ungesättigten Fettsäuren von mindestens 50 %, vorteilhaft von mindestens 80 %, besonders vorteilhaft von mindestens 100 %, ganz besonders vorteilhaft von mindestens 150 % gegenüber den nicht transgenen Nutzpflanzen siehe Beispiele erreicht werden.

Auch chemisch reine mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus den Pflanzen in bekannter Weise beispielsweise über Extraktion, Destillation, Kristallisation, Chromatographie oder Kombinationen dieser Methoden isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Für das erfindungsgemäße Verfahren sind prinzipiell alle dicotylen oder monokotylen Nutzpflanzen geeignet. Unter Nutzpflanzen sind Pflanzen zu verstehen, die der Nahrungsproduktion für Mensch und Tier, der Produktion von Genussmitteln, Fasern und Pharmazeutika dienen wie Getreide z.B. Mais, Reis, Weizen, Gerste, Hirse, Hafer, Rogen, Buchweizen; wie Knollen z.B. Kartoffel, Maniok, Batate, Yams etc.; wie Zuckerpflanzen z.B. Zuckerrohr oder Zuckerrübe; wie Hülsenfrüchte z.B. Bohnen, Erbsen, Saubohne etc.; wie Öl- und Fettfrüchte z.B. Sojabohne, Raps, Sonnenblume, Färberdiestel, Lein, Camelina etc., um nur einige zu nennen. Vorteilhafte Pflanzen sind ausgewählt aus der Gruppe der Pflanzenfamilien bestehend aus den Familien der *Aceraceae, Actinidiaceae, Anacardiaceae, Apiaceae, Arecaceae, Asteraceae, Arecaceae, Betulaceae, Boraginaceae, Brassicaceae, Bromeliaceae, Cannabaceae, Cannaceae, Caprifoliaceae, Chenopodiaceae, Convolvulaceae, Cucurbitaceae, Dioscoreaceae, Elaeagnaceae, Ericaceae, Euphorbiaceae, Fabaceae, Fagaceae, Grossulariaceae, Juglandaceae, Lauraceae, Liliaceae, Linaceae, Malvaceae, Moraceae, Musaceae, Oleaceae, Oxalidaceae, Papaveraceae, Poaceae, Polygonaceae, Punicaceae, Rosaceae, Rubiaceae, Rutaceae, Scrophulariaceae, Solanaceae, Sterculiaceae* und *Valerianaceae.*

Beispielhaft seien die folgenden Pflanzen genannt ausgewählt aus der Gruppe: Anacardiaceae wie die Gattungen Pistacia, Mangifera, Anacardium z.B. die Gattung und Arten *Pistacia vera* [Pistazie], *Mangifer indica* [Mango] oder *Anacardium occiden*tale [Cashew], Asteraceae wie die Gattungen Calendula, Carthamus, Centaurea, Cichorium, Cynara, Helianthus, Lactuca, Locusta, Tagetes, Valeriana z.B. die Gattung und Arten Calendula officinalis [Garten-Ringelblume], Carthamus tinctorius [Färberdistel, safflower], *Centaurea cyanus* [Kornblume], *Cichorium intybus* [Wegwarte], Cynara scolymus [Artichoke], *Helianthus annus* [Sonnenblume], *Lactuca sativa, Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta* [Salat], *Tagetes lucida,* Tagetes *erecta* oder *Tagetes tenuifolia* [Studentenblume], Apiaceae wie die Gattung Daucus z.B. die Gattung und Art *Daucus carota* [Karotte], Betulaceae wie die Gattung Corylus z.B. die Gattungen und Arten *Corylus avellana* oder *Corylus colurna* [Haselnuss], Boraginaceae wie die Gattung Borago z.B. die Gattung und Art *Borago officinalis* [Borretsch], Brassicaceae wie die Gattungen Brassica, Camelina, Melanosinapis, Sinapis, Arabadopsis z.B. die Gattungen und Arten *Brassica napus, Brassica rapa* ssp. [Raps], *Sinapis arvensis Brassica juncea, Brassica juncea var. juncea, Brassica juncea var. crispifolia, Brassica juncea var. foliosa, Brassica nigra, Brassica sinapioides,* Camelina sativa, *Melanosinapis communis* [Senf], *Brassica oleracea* [Futterrübe] oder Arabidopsis thaliana, Bromeliaceae wie die Gattungen Anana, Bromelia (Ananas) z.B. die Gattungen und Arten *Anana comosus, Ananas ananas* oder *Bromelia* comosa [Ananas], Caricaceae wie die Gattung Carica wie die Gattung und Art *Carica papaya* [Papaya], Cannabaceae wie die Gattung Cannabis wie die Gattung und Art *Cannabis sative* [Hanf], Convolvulaceae wie die Gattungen Ipomea, Convolvulus z.B. die Gattungen und Arten *Ipomoea batatus, Ipomoea pandurata, Convolvulus batatas, Convolvulus tiliaceus, Ipomoea fastigiata, Ipomoea tiliacea, Ipomoea triloba* oder *Convolvulus panduratus* [Süßkartoffel, Batate], Chenopodiaceae wie die Gattung Beta wie die Gattungen und Arten *Beta vulgaris, Beta vulgaris var. altissima, Beta vulgaris var. vulgaris, Beta maritima, Beta vulgaris var. perennis, Beta vulgaris var. conditiva* oder *Beta vulgaris var. esculenta* [Zuckerrübe], Cucurbitaceae wie die Gattung Cucubita z.B. die Gattungen und Arten *Cucurbita maxima, Cucurbita mixta, Cucurbita pepo* oder *Cucurbita moschata* [Kürbis], Elaeagnaceae wie die Gattung Elaeagnus z.B. die Gattung und Art *Olea europaea* [Olive], Ericaceae wie die Gattung Kalmia z.B. die Gattungen und Arten *Kalmia latifolia, Kalmia angustifolia, Kalmia microphylia, Kalmia polifolia, Kalmia occidentatis, Cistus chamaerhodendros* oder *Kalmia lucida* [Berglorbeer], Euphorbiaceae wie die Gattungen Manihot, Janipha, Jatropha, Ricinus z.B. die Gattungen und Arten *Manihot utilissima, Janipha manihot,, Jatropha manihot., Manihot aipil, Manihot dulcis, Manihot manihot, Manihot melanobasis, Manihot esculenta* [Manihot] oder *Ricinus communis* [Rizinus], Fabaceae wie die Gattungen Pisum, Albizia, Cathormion, Feuillea, Inga, Pithecolobium, Acacia, Mimosa, Medicajo, Glycine, Dolichos, Phaseolus, Soja z.B. die Gattungen und Arten *Pisum sativum, Pisum arvense, Pisum humile* [Erbse], *Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [Seidenbaum], *Medicago sativa, Medicago falcata, Medicago varia* [Alfalfa] *Glycine max Dolichos soja; Glycine gracilis, Glycine hispida, Phaseolus max, Soja hispida* oder *Soja max* [Sojabohne], Geraniaceae wie die Gattungen Pelargonium, Cocos, Oleum z.B. die Gattungen und Arten Cocos *nucifera, Pelargonium grossularioides* oder *Oleum cocois* [Kokusnuss], Gramineae wie die Gattung Saccharum z.B. die Gattung und Art Saccharum officinarum, Juglandaceae wie die Gattungen Juglans, Wallia z.B. die Gattungen und Arten *Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* oder *Wallia nigra* [Walnuss], Lauraceae Wie die Gattungen Persea, Laurus z.B. die Gattungen und Arten *Laurus nobilis* [Lorbeer], *Persea americana, Persea gratissima* oder *Persea persea* [Avocado], Leguminosae wie die Gattung Arachis z.B. die Gattung und Art Arachis *hypogaea* [Erdnuss], Linaceae wie die Gattungen Linum, Adenolinum z.B. die Gattungen und Arten *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* oder *Linum trigynum* [Lein], Lythrarieae wie die Gattung Punica z.B. die Gattung und Art *Punica granatum* [Granatapfel], Malvaceae wie die Gattung Gossypium z.B. die Gattungen und Arten *Gossypium hirsutum, Gossypium arboreum, Gossypium barbadense, Gossypium herbaceum* oder *Gossypium thurberi* [Baumwolle], Musaceae wie die Gattung Musa z.B. die Gattungen und Arten *Musa nana, Musa acuminata, Musa paradisiaca, Musa* spp. [Banane], Onagraceae wie die Gattungen Camissonia, Oenothera z.B. die Gattungen und Arten *Oenothera biennis* oder *Camissonia brevipes* [Nachtkerze], Palmae wie die Gattung Elacis z.B. die Gattung und Art *Elaeis guineensis* [Ölpalme], Papaveraceae wie die Gattung Papaver z.B. die Gattungen und Arten *Papaver orientale, Papaver rhoeas, Papaver dubium* [Mohn], Pedaliaceae wie die Gattung Sesamum z.B. die Gattung und Art *Sesamum indicum* [Sesam], Piperaceae wie die Gattungen Piper, Artanthe, Peperomia, Steffensia z.B. die Gattungen und Arten *Piper aduncum, Piper amalago, Piper angustifolium, Piper auritum, Piper betel, Piper cubeba, Piper longum, Piper nigrum, Piper retrofractum, Artanthe* adunca, *Artanthe elongata, Peperomia elongata, Piper elongatum, Steffensia elongata.* [Cayennepfeffer], Poaceae wie die Gattungen Hordeum, Secale, Avena, Sorghum, Andropogon, Holcus, Panicum, Oryza, Zea (Mais), Triticum z.B. die Gattungen und Arten *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [Gerste], *Secale cereale* [Roggen], Avena sativa, *Avena fatua, Avena byzantina, Avena fatua var. sativa, Avena hybrida* [Hafer], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum, Panicum militaceum [Hirse], Oryza sativa, Oryza latifolia* [Reis], *Zea mays* [Mais] *Triticum aestivum, Triticum durum, Triticum turgidum, Triticum hybernum, Triticum macha, Triticum sativum* oder *Triticum vulgare* [Weizen], Porphyridiaceae wie die Gattungen Chroothece, Flintiella, Petrovanella, Porphyridium, Rhodella, Rhodosorus, Vanhoeffenia z.B. die Gattung und Art *Porphyridium cruentum,* Proteaceae wie die Gattung Macadamia z.B. die Gattung und Art *Macadamia intergrifolia* [Macadamia], Rubiaceae wie die Gattung Coffea z.B. die Gattungen und Arten Cofea spp., *Coffea arabica, Coffea canephora* oder *Coffea liberica* [Kaffee], Scrophulariaceae wie die Gattung Verbascum z.B. die Gattungen und Arten *Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* oder *Verbascum thapsus [Königskerze],* Solanaceae wie die Gattungen Capsicum, Nicotiana, Solanum, Lycopersicon z.B. die Gattungen und Arten *Capsicum annuum, Capsicum annuum var. glabriusculum, Capsicum frutescens* [Pfeffer], *Capsicum annuum* [Paprika], *Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [Tabak], *Solanum tuberosum* [Kartoffel], *Solanum melongena* [Aubergine] *Lycopersicon esculentum, Lycopersicon lycopersicum., Lycopersicon pyriforme, Solanum integrifolium* oder *Solanum lycopersicum* [Tomate], Sterculiaceae wie die Gattung Theobroma z.B. die Gattung und Art *Theobroma* cacao [Kakao] oder Theaceae wie die Gattung Camellia z.B. die Gattung und Art *Camellia sinensis* [Tee].

In einer vorteilhaften Ausführungsform des Verfahren werden als Nutzpflanzen Ölfruchtpflanzen verwendet, die große Mengen an Lipidverbindungen enthalten, wie Erdnuss, Raps, Canola, Sonnenblume, Saflor (Carthamus tinctoria), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme, Kokosnuss oder Walnuss) oder Feldfrüchte, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte. Vorteilhafte erfindungsgemäße Pflanzen sind Ölfruchtpflanzen, wie Erdnuss, Raps, Canola, Sonnenblume, Saflor , Mohn, Senf, Hanf, Rhizinus, Olive, Calendula, Punica, Nachtkerze, Kürbis, Lein, Soja, Borretsch, Bäume (Ölpalme, Kokosnuss). Besonders bevorzugt sind C18:2- und/oder C18:3-Fettsäure reiche Pflanzen wie Sonnenblume, Färberdistel, Tabak, Königskerze, Sesam, Baumwolle, Kürbis, Mohn, Nachtkerze, Walnuss, Lein, Hanf, Distel oder Färberdistel. Ganz besonders bevorzugt sind Pflanzen wie Färberdistel, Sonnenblume, Mohn, Nachtkerze, Walnuss, Lein oder Hanf.

Für das erfindungsgemäße beschriebene Verfahren ist es vorteilhaft in die Pflanzen zusätzlich zu den unter Verfahrensschritt (a) bis (c) eingebrachten Nukleinsäuren sowie den ggf. eingebrachten Nukleinsäuresequenzen, die für die ω-3-Desaturasen codieren, zusätzlich weitere Nukleinsäuren einzubringen, die für Enzyme des Fettsäure- oder Lipidstoffwechsels codieren.

Im Prinzip können alle Gene des Fettsäure- oder Lipidstoffwechsels vorteilhaft in Kombination mit den im erfinderungsgemäßen Verfahren verwendeten Nukleinsäuresequenzen, die für die Δ-6-Elongase(n), Δ-6-Desaturase(n), Δ-5-Desaturase(n) und/oder Δ-3-Desaturase(n) [im Sinne dieser Anmeldung soll der Plural den Singular und umgekehrt beinhalten] codieren, verwendet werden; vorteilhaft werden Gene des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferasen, Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) in Kombination mit der Δ-6-Elongase, Δ-6-Desaturase, Δ-5-Desaturase und/oder ω-3-Desaturase verwendet. Besonders bevorzugt werden Gene ausgewählt aus der Gruppe der Δ-4-Desaturasen, Δ-8-Desatuasen, Δ-9-Desaturasen, Δ-12-Desaturasen, Δ-5-Elongase oder Δ-9-Elongasen in Kombination mit den vorgenannten Genen für die Δ-6-Elongase, Δ-6-Desaturase, Δ-5-Desaturase und/oder ω-3-Desaturase verwendet, wobei einzelne Gene oder mehrere Gene in Kombination verwendet werden können.

Die im erfindungsgemäße Verfahren verwendete ω-3-Desaturase sollte vorteilhaft eine Verschiebung vom ω-6-Biosyntheseweg zum ω-3-Biosyntheseweg ermöglichen dies führt vorteilhaft zu einer Verschiebung von C_{18:2}- zu C_{18:3}-Fettsäuren. Durch diese Eigenschaften der ω-3-Desaturase ist es vorteilhaft möglich das Fettsäurespektrum innerhalb eines Organismus vorteilhaft innerhalb einer Pflanze oder einem Pilz von den w-6-Fettsäuren zu den w-3-Fettsäuren hin zu verschieben. Weiterhin ist vorteilhaft, dass die ω-3-Desaturase eine breite Palette von Phospholipiden wie Phosphatidylcholin (= PC), Phosphatidylinositol (= PIS) oder Phosphatidylethanolamin (= PE) umsetzt. Schließlich lassen sich auch Desaturierungsprodukte in den Neutrallipiden (= NL), das heißt in den Triglyceriden finden.

Durch die enzymatische Aktivität der im erfindungsgemäßen Verfahren verwendeten Nukleinsäuren, die für Polypeptide mit Δ-6-Elongase, Δ-6-Desaturase, Δ-5-Desaturase, und/oder ω-3-Desaturaseaktivität codieren, vorteilhaft in Kombination mit Nukleinsäuresequenzen, die für Polypeptide des Fettsäure- oder Lipidstoffwechsels wie weiteren Polypeptiden mit Δ-4-, Δ-5-, Δ-6-, Δ-8-, Δ-12-Desaturase- oder Δ-5-, Δ-6-oder Δ-9-Elongaseaktivität codieren, können unterschiedlichste mehrfach ungesättigte Fettsäuren im erfindungsgemäßen Verfahren hergestellt werden. Je nach Auswahl der für das erfindungsgemäße Verfahren verwendeten Nutzpflanzen lassen sich Mischungen der verschiedenen mehrfach ungesättigten Fettsäuren oder einzelne mehrfach ungesättigte Fettsäuren wie EPA oder ARA und EPA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA oder, die sich von C18:3-Fettsäuren ableiten, wie SDA, ETA oder EPA. Liegt in der für das Verfahren verwendeten Pflanze als ungesättigte Fettsäure nur Linolsäure (= LA, C18:2^{Δ9,12}) vor, so können als Produkte des Verfahrens nur GLA, DGLA und ARA entstehen, die als freie Fettsäuren oder gebunden vorliegen können. Ist in der im Verfahren verwendeten Pflanze als ungesättigte Fettsäure nur α-Linolensäure (= ALA, C18:3^{Δ9,12,15}) beispielsweise wie in Lein, so können als Produkte des Verfahrens nur SDA, ETA und/oder EPA entstehen, die wie oben beschrieben als freie Fettsäuren oder gebunden vorliegen können. Durch Modifikation der Aktivität der im Verfahren verwendeten und an der Synthese beteiligten Enzyme Δ-6-Elongase, Δ-6-Desaturase, Δ-5-Desaturase und/oder ω-3-Desaturase vorteilhaft in Kombination mit weiteren Genen des Lipid- oder Fettsäurestoffwechsels lassen sich gezielt in den Pflanzen nur einzelne Produkte herstellten. Vorteilhaft werden nur EPA oder ARA und EPA synthetisiert, abhängig von der in im Organismus bzw. in der Pflanze vorliegenden Fettsäure, die als Ausgangssubstanz für die Synthese dient. Da es sich um Biosyntheseketten handelt, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, vorteilhaft weniger als 15 Gew.-%, besonders vorteilhaft weniger als 10 Gew.-%, ganz besonders vorteilhaft weniger als 5, 4, 3, 2 oder 1 Gew.-% bezogen auf das Endprodukt EPA oder ARA und EPA.

Neben der Produktion der Ausgangsfettsäuren für das erfindungsgemäße Verfahren direkt in der Pflanze können die Fettsäuren prinzipiell auch von außen gefüttert werden. Aus Kostengründen ist die Produktion in der Pflanze bevorzugt. Bevorzugt Substrate der ω-3-Desaturase sind die Linolsäure (C18:2^{Δ9,12}), die y-linolensäure (C18:3^{Δ6,9,12}), die Eicosadiensäure (C20:2^{Δ11,14}), die Dihomo-y-linolensäure (C20:3^{Δ8,11,14}) und die Arachidonsäure (C20:4^{Δ5,8,11,14}).

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft die Menge an Ausgangsprodukt für die Fettsäuresynthese zu steigern, dies kann beispielsweise durch das Einbringen einer Nukleinsäure in den Organismus, die für ein Polypeptid mit Δ-12-Desaturase codiert, erreicht werden. Dies ist besonders vorteilhaft in Nutzpflanzen, die Ölsäure enthalten wie Öl-produzierende Pflanzen wie Pflanzen der Familie der Brassicaceae wie der Gattung Brassica z.B. Raps; der Familie der Elaeagnaceae wie die Gattung Elaeagnus z.B. die Gattung und Art *Olea europaea* oder der Familie Fabaceae wie der Gattung Glycine z.B. die Gattung und Art *Glycine max*, die einen hohen Ölsäuregehalt aufweisen. Da diese Organismen nur einen geringen Gehalt an Linolsäure aufweisen (Mikoklajczak et al., Journal of the American Oil Chemical Society, 38, 1961, 678 - 681) ist die Verwendung der genannten Δ-12-Desaturasen zur Herstellung des Ausgangsprodukts Linolsäure vorteilhaft.

Im erfindungsgemäßen Verfahren verwendeten Nukleinsäuren stammen vorteilhaft aus Pflanzen wie Algen beispielsweise Algen der Familie der Prasinophyceae wie aus den Gattungen Heteromastix, Mammella, Mantoniella, Micromonas, Nephroselmis, Ostreococcus, Prasinocladus, Prasinococcus, Pseudoscourfielda, Pycnococcus, Pyramimonas, Scherffelia oder Tetraselmis wie den Gattungen und Arten Heteromastix longifillis, Mamiella gilva, Mantoniella squamata, Micromonas pusilla, Nephroselmis olivacea, Nephroselmis pyriformis, Nephroselmis rotunda, Ostreococcus tauri, Ostreococcus sp. Prasinocladus ascus, Prasinocladus lubricus, Pycnococcus provasolii, Pyramimonas amylifera, Pyramimonas disomata, Pyramimonas obovata, Pyramimonas orientalis, Pyramimonas parkeae, Pyramimonas spinifera, Pyramimonas sp., Tetraselmis apiculata, Tetraselmis carteriaformis, Tetraselmis chui, Tetraselmis convolutae, Tetraselmis desikacharyi, Tetraselmis gracilis, Tetraselmis hazeni, Tetraselmis impellucida, Tetraselmis inconspicua, Tetraselmis levis, Tetraselmis maculata, Tetraselmis marina, Tetraselmis striata, Tetraselmis subcordiformis, Tetraselmis suecica, Tetraselmis tetrabrachia, Tetraselmis tetrathele, Teträselmis verrucosa, Tetraselmis verrucosa fo. rubens oder Tetraselmis sp. oder aus Algen der Familie Euglenaceae wie aus den Gattungen Ascoglena, Astasia, Colacium, Cyclidiopsis, Euglena, Euglenopsis, Hyalophacus, Khawkinea, Lepocinclis, Phacus, Strombomonas oder Trachelomonas wie die Gattungen und Art Euglena acus, Euglena geniculata, Euglena gracilis, Euglena mixocylindracea, Euglena rostrifera, Euglena viridis, Colacium stentorium, Trachelomonas cylindrica oder Trachelomonas volvocina. Vorteilhaft stammen die verwendeten Nukleinsäuren aus Algen der Gattungen Euglena, Mantoniella oder Ostreococcus.

Weitere vorteilhafte Pflanzen sind Algen wie Isochrysis oder Crypthecodinium, Algen/ Diatomeen wie Thalassiosira oder Phaeodactylum, Moose wie Physcomitrella oder Ceratodon oder höheren Pflanzen wie den Primulaceae wie Aleuritia, Calendula stellata, Osteospermum spinescens oder Osteospermum hyoseroides, Mikroorganismen wie Pilzen wie Aspergillus, Thraustochytrium, Phytophthora, Entomophthora, Mucor oder Mortierella, Bakterien wie Shewanella, Hefen oder Tieren wie Nematoden wie Caenorhabditis, Insekten, Fröschen, Seegurken oder Fischen. Vorteilhaft stammen die erfindungsgemäßen isolierten Nukleinsäuresequenzen aus einem Tier aus der Ordnung der Vertebraten. Bevorzugt stammen die Nukleinsäuresequenzen aus der Klasse der Vertebrata; Euteleostomi, Actinopterygii; Neopterygii; Teleostei; Euteleostei, Protacanthopterygii, Salmoniformes; Salmonidae bzw. Oncorhynchus oder Vertebrata, Amphibia, Anura, Pipidae, Xenopus oder Evertebrata wie Protochordata, Tunicata, Holothuroidea, Cionidae wie Amaroucium constellatum, Botryllus schlosseri, Ciona intestinalis, Molgula citrina, Molgula manhattensis, Perophora viridis oder Styela partita. Besonders vorteilhaft stammen die Nukleinsäuren aus Pilzen, Tieren oder aus Pflanzen wie Algen oder Moosen, bevorzugt aus der Ordnung der Salmoniformes wie der Familie der Salmonidae wie der Gattung Salmo beispielsweise aus den Gattungen und Arten Oncorhynchus mykiss, Trutta trutta oder Salmo trutta fario, aus Algen wie den Gattungen Mantoniella oder Ostreococcus oder aus den Diatomeen wie den Gattungen Thalassiosira oder Phaeodactylum oder aus Algen wie Crypthecodinium.

Vorteilhaft werden im erfindungsgemäßen Verfahren die vorgenannten Nukleinsäuresequenzen oder deren Derivat oder Homologe, die für Polypeptide codieren, die noch die enzymatische Aktivität der durch Nukleinsäuresequenzen codierten Proteine besitzen. Diese Sequenzen werden einzeln oder in Kombination mit den für die Δ-12-Desaturase, Δ-4-Desaturase, Δ-5-Desaturase, Δ-6-Desaturase, Δ-5-Elongase, Δ-6-Elongase und/oder ω-3-Desaturase codierenden Nukleinsäuresquenzen in Expressionskonstrukte cloniert und zum Einbringen und zur Expression in Organismen verwendet. Diese Expressionskonstrukte ermöglichen durch ihre Konstruktion eine vorteilhafte optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer Zelle oder einer ganzen Pflanze, die die im Verfahren verwendeten Nukleinsäuresequenzen, die für eine Δ-6-Desaturase, Δ-6-Elongase, Δ-5-Desaturase und/oder ω-3-Desaturase codieren, enthält, wobei die Zelle und/oder die Nutzpflanze noch weitere Nukleinsäuresequenzen des Lipid- oder Fettsäurestoffwechsels enthalten kann. Diese im Verfahren bevorzugt verwendeten Nukleinsäuresequenzen werden zur Expression vorteilhaft in mindestens ein Genkonstrukt und/oder einen Vektor wie nachfolgend beschrieben, allein oder in Kombination mit weiteren Nukleinsäuresequenzen, die für Proteine des Fettsäure- oder Lipidsstoffwechsels codieren, eingebaut und schließlich in die Zelle oder Pflanze transformiert wird. Bei einer weiteren bevorzugten Ausführungsform umfasst dieses Verfahren ferner den Schritt des Gewinnens der Öle, Lipide oder freien Fettsäuren aus den Nutzpflanzen. Die so hergestellte Zelle oder die so hergestellte Nutzpflanze ist vorteilhaft eine Zelle einer Öl-produzierenden Pflanze, Gemüse-, Salat-, oder Zierpflanze oder die Pflanze selbst wie oben ausgeführt.

Unter Anzucht ist beispielsweise die Kultivierung im Falle von Pflanzenzellen, -gewebe oder -organe auf oder in einem Nährmedium oder der ganzen Pflanze auf bzw. in einem Substrat beispielsweise in Hydrokultur, Blumentopferde oder auf einem Ackerboden zu verstehen.

"Transgen" bzw. "Rekombinant" im Sinne der Erfindung bedeutet bezüglich zum Beispiel einer Nukleinsäuresequenz, einer Expressionskassette (= Genkonstrukt) oder einem Vektor enthaltend die im erfindungsgemäßen Verfahren verwendeten Nukleinsäuresequenzen oder einer mit den im erfindungsgemäßen Verfahren verwendeten Nukleinsäuresequenzen, Expressionskassette oder Vektor transformierten Pflanze alle solche durch gentechnische Methoden zustandegekommenen Konstruktionen, in denen sich entweder
a) die Nukleinsäuresequenz, oder
b) eine mit der Nukleinsäuresequenz funktionell verknüpfte genetische Kontrollsequenz, zum Beispiel ein Promotor, oder
c) (a) und (b)

sich nicht in ihrer natürlichen, genetischen Umgebung befinden oder durch gentechnische Methoden modifiziert wurden, wobei die Modifikation beispielhaft eine Substitution, Addition, Deletion, Inversion oder Insertion eines oder mehrerer Nukleotidreste sein kann. Natürliche genetische Umgebung meint den natürlichen genomischen bzw. chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des natürlichen Promotors der im erfindungsgemäßen Verfahren verwendeten Nukleinsäuresequenz, die für Proteine mit entsprechender A-6-Desaturase-, Δ-6-Elongase-, Δ-5-Desaturase- und/oder ω-3-Desaturaseaktivität codiert, vorteilhaft in Kombination mit Nukleinsäuresequenzen, die für Proteine mit Δ-12-Desaturase-, Δ-4-Desaturase-, Δ-8-Desaturase-, Δ-9-Elongase-, und/oder Δ-5-Elongaseaktivität codieren, wird zu einer transgenen Expressionskassette, wenn diese durch nicht-natürliche, synthetische ("künstliche") Verfahren wie beispielsweise einer Mutagenisierung geändert wird. Entsprechende Verfahren sind beispielsweise beschrieben in US 5,565.350 oder WO 00/15815.

Unter transgener Pflanze im Sinne der Erfindung ist wie vorgenannt zu verstehen, dass die im Verfahren verwendeten Nukleinsäuren nicht an ihrer natürlichen Stelle im Genom der Pflanze sind, dabei können die Nukleinsäuren homolog oder heterolog exprimiert werden. Transgen bedeutet aber auch wie genannt, dass die erfindungsgemäßen Nukleinsäuren an ihrem natürlichen Platz im Genom der Pflanze sind, dass jedoch die Sequenz gegenüber der natürlichen Sequenz verändert wurde und/oder das die Regulationssequenzen, der natürlichen Sequenzen verändert wurden. Bevorzugt ist unter transgen die Expression der im erfindungsgemäßen Verfahren verwendeten Nukleinsäuren an nicht natürlicher Stelle im Genom zu verstehen, das heißt eine homologe oder bevorzugt heterologe Expression der Nukleinsäuren liegt vor. Bevorzugte transgene Organismen sind Nutzpflanzen wie Öl-produzierende Pflanzen, Gemüse-, Salat- oder Zierpflanzen, die vorteilhaft ausgewählt sind aus der Gruppe der Pflanzenfamilien bestehend aus den Familien der *Aceraceae, Actinidiaceae, Anacardiaceae, Apiaceae, Arecaceae, Asteraceae, Arecaceae, Betulaceae, Boraginaceae, Brassicaceae, Bromeliaceae, Cannabaceae, Cannaceae, Caprifoliaceae, Chenopodiaceae, Convolvulaceae, Cucurbitaceae, Dioscoreaceae, Elaeagnaceae, Ericaceae, Euphorbiaceae, Fabaceae, Fagaceae, Grossulariaceae, Juglandaceae, Lauraceae, Liliaceae, Linaceae, Malvaceae, Moraceae, Musaceae, Oleaceae, Oxalidaceae, Papaveraceae, Poaceae, Polygonaceae, Punicaceae, Rosaceae, Rubiaceae, Rutaceae, Scrophulariaceae, Solanaceae, Sterculiaceae* und *Valerianaceae.*

Als Wirtspflanzen für die im erfindungsgemäßen Verfahren verwendeten Nukleinsäuren, die Expressionskassette oder den Vektor eignen sich prinzipiell vorteilhaft alle Nutzpflanzen, die in der Lage sind Fettsäuren speziell ungesättigte Fettsäuren zu synthetisieren bzw. für die Expression rekombinanter Gene geeignet sind. Beispielhaft seien an dieser Stelle Pflanzen wie Arabidopsis, Asteraceae wie Calendula oder Nutzpflanzen wie Soja, Erdnuss, Rizinus, Sonnenblume, Mais, Baumwolle, Flachs, Raps, Kokosnuss, Ölpalme, FärberSaflor (Carthamus tinctorius) oder Kakaobohne genannt, Weitere vorteilhafte Pflanzen sind an andere Stelle dieser Anmeldung aufgeführt.

Für die Herstellung der transgenen Nutzpflanze werden in der Regel als Zwischenwirte Mikroorganismen verwendet. Derartige nutzbare Zwischenwirtszellen werden in: Goeddel, Gene Expression Technology: Methods in Enzymology 185, Academic Press, San Diego, CA (1990) genannt.

Vorteilhaft verwendbare Expressionsstämme für diesen Zweck sind z.B. solche, die eine geringere Proteaseaktivität aufweisen. Sie werden z.B. in: Gottesman, S., Gene Expression Technology: Methods in Enzymology 185, Academic Press, San Diego, California (1990) 119-128 beschrieben.

Transgene Pflanzen, die die im erfindungsgemäßen Verfahren synthetisierten mehrfach ungesättigten Fettsäuren enthalten, können vorteilhaft direkt vermarktet werden ohne dass die synthetisierten Öle, Lipide oder Fettsäuren isoliert werden müssen. Diese Form der Vermarktung ist besonders vorteilhaft.

Unter Pflanzen im erfindungsgemäßen Verfahren sind , wie oben beschrieben, ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, die sich von der transgenen Pflanze abgeleiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe. Die im erfindungsgemäßen Verfahren hergestellten Verbindungen können aber auch aus den Pflanzen in Form ihrer Öle, Fett, Lipide und/oder freien Fettsäuren isoliert werden. Durch dieses Verfahren hergestellte mehrfach ungesättigten Fettsäuren lassen sich durch Ernten der Pflanzen oder Pflanzenzellen entweder aus der Kultur, in der sie wachsen, oder vom Feld ernten. Dies kann über Pressen oder Extraktion der Pflanzenteile bevorzugt der Pflanzensamen erfolgen. Dabei können die Öle, Fette, Lipide und/oder freien Fettsäuren durch sogenanntes kalt schlagen oder kalt pressen ohne Zuführung von Wärme durch Pressen gewonnen werden. Damit sich die Pflanzenteile speziell die Samen leichter aufschließen lassen, werden sie vorher zerkleinert, gedämpft oder geröstet. Die so vorbehandelten Samen können anschließend gepresst werden oder mit Lösungsmittel wie warmen Hexan extrahiert werden. Anschließend wird das Lösungsmittel wieder entfernt. Auf diese Weise können mehr als 96 % der im Verfahren hergestellten Verbindungen isoliert werden. Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen werden die Produkte einer Bleichung mit beispielsweise Bleicherde oder Aktivkohle unterzogen. Zum Schluss wird das Produkt noch beispielsweise mit Wasserdampf noch desodoriert.

Vorzugsweise sind die durch dieses Verfahren produzierten PUFAs bzw. LCPUFAs C₁₈-, und/oder C₂₀- Fettsäuremoleküle vorteilhaft C₂₀-Fettsäuremoleküle mit mindestens zwei Doppelbindungen im Fettsäuremolekül, vorzugsweise drei, vier oder fünf Doppelbindungen. Diese C₁₈- und/oder C₂₀-Fettsäuremoleküle lassen sich aus der Pflanze in Form eines Öls, Lipids oder einer freien Fettsäure isolieren. Geeignete transgene Pflanzen sind beispielsweise die vorstehend erwähnten.

Öle, Lipide oder Fettsäuren oder Fraktionen davon, die durch das oben beschriebene Verfahren hergestellt worden sind, lassen sich zur Herstellung von Futtermitteln, Nahrungsmitteln, Kosmetika oder Pharmazeutika verwenden.

Diese so gewonnenen Öle, Lipide oder Fettsäuren enthalten wie oben beschrieben vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Pflanzen. Als vorteilhafte mehrfach ungesättigte Fettsäure sind in den Fettsäureester bzw. Fettsäuregemische wie Phosphatidylfettsäureester oder Triacylglyceridester bevorzugt mindestens 10; 11; 12; 13; 14; 15; 16; 17; 18; 19 oder 20 Gew.% bezogen auf den Gesamtfettsäuregehalt an Arachidonsäure und/oder mindestens 20; 21; 22; 23; 24 oder 25; vorteilhaft mindestens 26, 27, 28, 29 oder 30, besonders vorteilhaft mindestens 31, 32, 33, 34, 35, 36; 37; 38; 39 oder 40, ganz besonders vorteilhaft mindestens 41, 42, 43, 44, 45 Gew.% oder mehr bezogen auf den Gesamtfettsäuregehalt an Eicosapentaensäure enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopentendodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11 t13c-Octadecatriensäure),Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. In einer weiteren bevorzugten Form der Erfindung kommen diese vorgenannten Fettsäuren bezogen auf die Gesamtfettsäuren zu weniger als 0,9%; 0,8%; 0,7%; 0,6%; oder 0,5%, besonders bevorzugt zu weniger als 0,4%; 0,3%; 0,2%; 0,1 % vor. Vorteilhaft enthalten die nach dem erfindungsgemäßen Verfahren hergestellten Fettsäureester bzw. Fettsäuregemische weniger als 0,1 % bezogen auf die Gesamtfettsäuren und/oder keine Butterbuttersäure, kein Cholesterin, keine Clupanodonsäure (= Docosapentaensäure, C22:5^{Δ4,8,12,15,21}) sowie keine Nisinsäure (Tetracosahexaensäure, C23:6^{Δ3,8,12,15,18,21})_{.}

Die im erfindungsgemäßen Verfahren gewonnenen Öle, Lipide, Fettsäuren oder Fettsäuregemische können in der dem Fachmann bekannten Weise zur Abmischung mit anderen Ölen, Lipiden, Fettsäuren oder Fettsäuregemischen tierischen Ursprungs wie z.B. Fischölen verwendet werden. Auch diese so hergestellten Öle, Lipide, Fettsäuren oder Fettsäuregemische, die aus pflanzlichen und tierischen Bestandteilen bestehen, können zur Herstellung von Futtermitteln, Nahrungsmitteln, Kosmetika oder Pharmazeutika verwendet werden.

Unter dem Begriff "Öl", "Lipid" oder "Fett" wird ein Fettsäuregemisch verstanden, das ungesättigte, gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, γ-Linolensäure, Dihomo-γ-linolensäure, Arachidonsäure, α-Linolensäure, Stearidonsäure, Eicosatetraensäure oder Eicosapentaensäure hat. Vorzugsweise ist der Anteil an ungesättigten veresterten Fettsäuren ungefähr 30 %, mehr bevorzugt ist ein Anteil von 50 %, noch mehr bevorzugt ist ein Anteil von 60 %, 70 %, 80 % oder mehr. Zur Bestimmung kann z.B. der Anteil an Fettsäure nach Überführung der Fettsäuren in die Methylestern durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach Ausgangspflanze der Anteil der verschiedenen Fettsäuren in dem Öl oder Fett schwanken.

Bei den im Verfahren hergestellten mehrfach ungesättigte Fettsäuren mit vorteilhaft mindestens zwei, drei, vier oder fünf, besonders vorteilhaft mit vier oder fünf Doppelbindungen handelt es sich wie oben beschrieben vorteilhaft um Fettsäureester beispielsweise um Sphingolipidester, Phosphoglyceridester, Lipidester, Glycolipidester, Phospholipidester, Monoacylglycerinester, Diacylglycerinester, Triacylglycerinester oder sonstige Fettsäureester, bevorzugt handelt es sich um Phospholipidester und/oder Triacylglycerinester.

Aus den so im erfindungsgemäßen Verfahren hergestellten mehrfach ungesättigte Fettsäureestern mit vorteilhaft mindestens drei, vier oder fünf Doppelbindungen lassen sich die enthaltenden mehrfach ungesättigten Fettsäuren beispielsweise über eine Alkalibehandlung beispielsweise wäßrige KOH oder NaOH oder saure Hydrolyse vorteilhaft in Gegenwart eines Alkohols wie Methanol oder Ethanol oder über eine enzymatische Abspaltung freisetzen und isolieren über beispielsweise Phasentrennung und anschließender Ansäuerung über z.B. H₂SO₄. Die Freisetzung der Fettsäuren kann auch direkt ohne die vorhergehend beschriebene Aufarbeitung erfolgen.

Die im Verfahren verwendeten Nukleinsäuren können nach Einbringung in eine Pflanze oder Pflanzenzelle entweder auf einem separaten Plasmid liegen oder vorteilhaft in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression in die Organismen vorteilhaft zur multiparallelen samenspezifischen Expression von Genen in die Pflanzen gebracht.

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Moose enthalten PUFAs in Membranlipiden während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft für das erfindungsgemäße Verfahren und damit zur Modifikation des Lipid- und PUFA-Produktionssystems in einer Pflanze, wie einer Nutzpflanze wie einer Ölfruchtpflanze, beispielsweise Raps, Canola, Lein, Hanf, Soja, Sonnenblumen, Borretsch. Sie sind deshalb vorteilhaft im erfindungsgemäßen Verfahren verwendbar.

Als Substrate der im erfindungsgemäßen Verfahren verwendeten Nukleinsäuren, die für Polypeptide mit Δ-6-Desaturase-, Δ-6-Elongase-, Δ-5-Desaturase- und/oder ω-3-Desaturase-Aktivität codieren, und/oder den weiteren verwendeten Nukleinsäuren wie den Nukleinsäuren, die für Polypeptide des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophosphotipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetytenase(n), Lipoxygenase(n), Triacylglycerol-Lipase(n), Allenoxid-Synthase(n), Hydroperoxid-Lyase(n) oder Fettsäure-Elongase(n) codieren eignen sich vorteilhaft C₁₆-, C₁₈- oder C₂₀-Fettsäuren. Bevorzugt werden die im Verfahren als Substrate umgesetzten Fettsäuren in Form ihrer Acyl-CoA-Ester und/oder ihrer Phospholipid-Ester umgesetzt.

Zur Herstellung der erfindungsgemäßen langkettigen PUFAs müssen die gesättigten, einfach ungesättigten C₁₆-Fettsäuren und/oder mehrfach ungesättigten C₁₈-Fettsäuren zunächst je nach Substrat durch die enzymatische Aktivität einer Desaturase und/oder Elongase zunächst desaturiert und/oder elongiert oder nur desaturiert und anschließend über eine Elongase um mindestens zwei Kohlenstoffatome verlängert werden. Nach einer Elongationsrunde führt diese Enzymaktivität entweder ausgehend von C₁₆-Fettsäuren zu C₁₈-Fettsäuren oder ausgehend von C₁₈-Fettsäuren zu C₂₀-Fettsäuren, und nach zwei Elongationsrunden ausgehend von C₁₆-Fettsäuren zu C₂₀-Fettsäuren. Die Aktivität der erfindungsgemäßen Verfahren verwendeten Desaturasen und Elongasen führt vorzugsweise zu C₁₈- und/oder C₂₀-Fettsäuren vorteilhaft mit mindestens zwei Doppelbindungen im Fettsäuremolekül, vorzugsweise mit drei, vier oder fünf Doppelbindungen, besonders bevorzugt zu C₂₀-Fettsäuren mit mindestens vier Doppelbindungen im Fettsäuremolekül. Besonders bevorzugt als Produkte des erfindungsgemäßen Verfahrens sind Dihomo-γ-linolensäure, Arachidonsäure und/oder Eicosapentaensäure. Die C₁₈-Fettsäuren mit mindestens zwei Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.

Der bevorzugte Biosyntheseort von Fettsäuren, Ölen, Lipiden oder Fette in den vorteilhaft verwendeten Pflanzen ist beispielsweise im allgemeinen der Samen oder Zellschichten des Samens, so dass eine samenspezifische Expression der im Verfahren verwendeten Nukleinsäuren sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann. Gemäß des erfinderischen Verfahrens findet die im Synthese im vegetativen (somatischen) Gewebe statt.

Durch das erfindungsgemäße Verfahren können die hergestellten mehrfach ungesättigten Fettsäuren in den im Verfahren verwendeten Pflanzen prinzipiell auf zwei Arten erhöht werden. Es kann vorteilhaft der Pool an freien mehrfach ungesättigten Fettsäuren und/oder der Anteil der über das Verfahren hergestellten veresterten mehrfach ungesättigten Fettsäuren erhöht werden. Vorteilhaft wird durch das erfindungsgemäße Verfahren der Pool an veresterten mehrfach ungesättigten Fettsäuren in den transgenen Pflanzen erhöht, vorteilhaft in Form der Phosphatidylester und/oder Triacylester.

Die im Verfahren gewonnenen Fettsäuren eignen sich auch als Ausgangsmaterial für die chemische Synthese von weiteren Wertprodukten. Sie können beispielsweise in Kombination miteinander oder allein zur Herstellung von Pharmaka, Nahrungsmittel, Tierfutter oder Kosmetika verwendet werden.

Vorteilhafte im Verfahren verwendete Nukleinsäuresequenzen, die für Polypeptide mit Δ-6-Desaturaseaktivität codieren, sind isolierte Nukleinsäuresequenzen ausgewählt aus der Gruppe:
a) einer Nukleinsäuresequenz mit der in SEQ ID NO: 1 dargestellten Sequenz,
b) Nukleinsäuresequenzen, die sich als Ergebnis des degenerierten genetischen Codes von der in SEQ ID NO: 2 dargestellten Aminosäuresequenz ableiten lassen, oder
c) Derivate der in SEQ ID NO: 1 dargestellten Nukleinsäuresequenz, die für Polypeptide codieren, die auf Aminosäureebene mindestens 70% Identität mit SEQ ID NO: 2 aufweisen und eine Δ-6-Desaturaseaktivität haben.

Vorteilhafte im Verfahren verwendete Nukleinsäuresequenzen, die für Polypeptide mit Δ-6-Elongaseaktivität codieren, sind isolierte Nukleinsäuresequenzen ausgewählt aus der Gruppe:
a) einer Nukleinsäuresequenz mit der in SEQ ID NO: 3 oder 7 dargestellten Sequenz,
b) Nukleinsäuresequenzen, die sich als Ergebnis des degenerierten genetischen Codes von der in SEQ ID NO: 4 oder 8 dargestellten Aminosäuresequenz ableiten lassen, oder
c) Derivate der in SEQ ID NO: 3 oder 7 dargestellten Nukleinsäuresequenz, die für Polypeptide codieren, die auf Aminosäureebene mindestens 70% Identität mit SEQ ID NO: 4 oder 8 aufweisen und eine Δ-6-Elongaseaktivität haben.

Vorteilhafte im Verfahren verwendete Nukleinsäuresequenzen, die für Polypeptide mit Δ-5-Desaturaseaktivität codieren, sind isolierte Nukleinsäuresequenzen ausgewählt aus der Gruppe:
a) einer Nukleinsäuresequenz mit der in SEQ ID NO: 5 dargestellten Sequenz,
b) Nukleinsäuresequenzen, die sich als Ergebnis des degenerierten genetischen Codes von der in SEQ ID NO: 6 dargestellten Aminosäuresequenz ableiten lassen, oder
c) Derivate der in SEQ ID NO: 5 dargestellten Nukleinsäuresequenz, die für Polypeptide codieren, die auf Aminosäureebene mindestens 70% Identität mit SEQ ID NO: 6 aufweisen und eine Δ-5-Desaturaseaktivität haben.

Vorteilhaft werden in Kombination mit den vorgenannten Nukleinsäuren, die für Δ-6-Desaturasen, Δ-6-Elonasen und/oder Δ-5-Desaturasen codieren, weitere Nukleinsäuresequenzen verwendete, die für Polypeptide mit ω-3-Desaturaseaktivität codieren, sind isolierte Nukleinsäuresequenzen ausgewählt aus der Gruppe:
a) einer Nukleinsäuresequenz mit der in SEQ ID NO: 9 dargestellten Sequenz,
b) Nukleinsäuresequenzen, die sich als Ergebnis des degenerierten genetischen Codes von der in SEQ ID NO: 10 dargestellten Aminosäuresequenz ableiten lassen, oder
c) Derivate der in SEQ ID NO: 9 dargestellten Nukleinsäuresequenz, die für Polypeptide codieren, die auf Aminosäureebene mindestens 60 % Identität mit SEQ ID NO: 10 aufweisen und eine ω-3-Desaturaseaktivität haben.

Vorteilhaft werden die vorgenannten Nukleinsäuresequenzen zur Expression in Genkonstrukte eingebracht, wobei die Nukleinsäuren funktionsfähig mit einem oder mehreren Regulationssignalen verbunden ist. Zusätzlich können weitere Biosynthesegene des Fettsäure- oder Lipidstoffwechsels enthält ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) im Genkonstrukt enthalten sein. Vorteilhaft sind zusätzlich Biosynthesegene des Fettsäure- oder Lipidstoffwechsels ausgewählt aus der Gruppe der Δ-4-Desaturase, Δ-8-Desatuase, Δ-9-Desaturase, Δ-12-Desaturase, Δ-5-Elongase, Δ-9-Elongase und/oder Δ-15-Desaturase enthalten.

Vorteilhaft stammen alle die im erfindungsgemäßen Verfahren verwendeten Nukleinsäuresequenzen aus einem eukaryontischen Organismus wie einer Pflanze, einem Mikroorganismus oder einem Tier. Bevorzugt stammen die Nukleinsäuresequenzen aus der Ordnung Salmoniformes, Xenopus oder Ciona, Algen wie Mantoniella, Crypthecodinium, Euglena oder Ostreococcus, Pilzen wie der Gattung Phytophtora oder von Diatomeen wie den Gattungen Thalassiosira oder Phaeodactylum.

Die im Verfahren verwendeten Nukleinsäuresequenzen, die für Proteine mit w-3-Desaturase-, Δ-5-Desaturase-, Δ-6-Desaturase- und/oder Δ-6-Elongase-Aktivität codieren, werden vorteilhaft allein oder bevorzugt in Kombination in einer Expressionskassette (= Nukleinsäurekonstrukt), die die Expression der Nukleinsäuren in einer Pflanze ermöglicht, eingebracht. Es kann im Nukleinsäurekonstrukt mehr als eine Nukleinsäuresequenz einer enzymatischen Aktivität wie z.B. einer Δ-12-Desaturase, Δ-4-Desaturase, Δ-5-Desaturase, Δ-8-Desaturase, Δ-6-Desaturase, Δ-5-Elongase, Δ-6-Elongase, Δ-9-Elongase und/oder ω-3-Desaturase enthalten sein.

Zum Einbringen werden die im Verfahren verwendeten Nukleinsäuren vorteilhaft einer Amplifikation und Ligation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden in Anlehnung an die zu amplifizierende Sequenz gewählt. Zweckmäßigerweise sollten die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert. Beispielsweise kann die Analyse nach gelelektrophoretischer Auftrennung hinsichtlich Qualität und Quantität erfolgen. Im Anschluss kann das Amplifikat nach einem Standardprotokoll gereinigt werden (z.B. Qiagen). Ein Aliquot des gereinigten Amplifikats steht dann für die nachfolgende Klonierung zur Verfügung. Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilze gewährleisten, und die stabile Transformation von Pflanzen ermöglichen. Zu nennen sind insbesondere verschiedene für die T-DNA-vermittelte Transformation geeignete, binäre und co-integrierte Vektorsysteme. Derartige Vektorsysteme sind in der Regel dadurch gekennzeichnet, dass sie zumindest die für die Agrobakterium-vermittelte Transformation benötigten vir-Gene sowie die T-DNA begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in E.-coli als auch in Agrobacterium zu replizieren. Zu diesen binären Vektoren gehören Vektoren der Serien pBIB-HYG, pPZP, pBecks, pGreen. Erfindungsgemäß bevorzugt verwendet werden Bin19, pBI101, pBinAR, pGPTV und pCAMBIA. Eine Übersicht über binäre Vektoren und ihre Verwendung gibt Hellens et al, Trends in Plant Science (2000) 5, 446-451. Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung wird das enzymatisch geschnittenen und erforderlichenfalls gereinigten Amplifikat mit ähnlich präparierten Vektorfragmenten mit Einsatz von Ligase kloniert. Dabei kann ein bestimmtes Nukleinsäurekonstrukt bzw. Vektor- oder Plasmidkonstrukt einen oder auch mehrere kodogene Genabschnitte aufweisen. Vorzugsweise sind die kodogenen Genabschnitte in diesen Konstrukten mit regulatorischen Sequenzen funktional verknüpft. Zu den regulatorischen Sequenzen gehören insbesondere pflanzliche Sequenzen wie die oben beschriebenen Promotoren und Terminatoren. Die Konstrukte lassen sich vorteilhafterweise in Mikroorganismen, insbesondere Escherichia coli und Agrobacterium tumefaciens, unter selektiven Bedingungen stabil propagieren und ermöglichen so einen Transfer von heterologer DNA in Pflanzen.

Unter der vorteilhaften Verwendung von Klonierungsvektoren können die im Verfahren verwendeten Nukleinsäuren, die erfinderischen Nukleinsäuren und Nukleinsäurekonstrukte in Mikroorganismen und danach vorteilhaft Pflanzen eingebracht werden und damit bei der Pflanzentransformation verwendet werden, wie denjenigen, die veröffentlicht sind in und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225)). Die im Verfahren verwendeten Nukleinsäuren, Nukleinsäurekonstrukte und/oder Vektoren lassen sich damit zur gentechnologischen Veränderung eines breiten Spektrums an Pflanzen verwenden, so dass diese bessere und/oder effizientere Produzenten von PUFAs werden.

Durch das Einbringen eines ω-3-Desaturase-, Δ-6-Desaturase-, Δ-6-Elongase- und Δ-5-Desaturase-Genes in eine Pflanze allein oder in Kombination mit anderen Genen kann nicht nur den Biosynthesefluss zum Endprodukt erhöht, sondern auch die entsprechende Triacylglycerin- und/oder Phosphatidylester-Zusammensetzung erhöht oder de novo geschaffen werden. Ebenso kann die Anzahl oder Aktivität anderer Gene, die am Import von Nährstoffen, die zur Biosynthese einer oder mehrerer Fettsäuren, Ölen, polaren und/oder neutralen Lipiden nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs, wie im folgenden beschrieben, weiter gesteigert wird. Durch Optimierung der Aktivität oder Erhöhung der Anzahl einer oder mehrerer ω-3-Desaturase-, Δ-6-Desaturase-, Δ-6-Elongase- und/oder Δ-5-Desaturase-Gene, die an der Biosynthese dieser Verbindungen beteiligt sind, oder durch Zerstören der Aktivität einer oder mehrerer Gene, die am Abbau dieser Verbindungen beteiligt sind, kann es möglich sein, die Ausbeute, Produktion und/oder Effizienz der Produktion von Fettsäure- und Lipidmolekülen aus Organismen und vorteilhaft aus Pflanzen zu steigern.

Die im erfindungsgemäßen Verfahren verwendeten Nukleinsäuremoleküle codieren für Proteine oder Teile von diesen, wobei die Proteine oder das einzelne Protein oder Teile davon eine Aminosäuresequenz enthält, die ausreichend homolog zu einer Aminosäuresequenz ist, die in den Sequenzen SEQ ID NO: 2, SEQ ID NO: 4, SEQ ID NO: 6, SEQ ID NO: 8 oder SEQ ID NO: 10 dargestellt ist, so dass die Proteine oder Teile davon noch eine ω-3-Desaturase-, Δ-6-Desaturase-, Δ-6-Elongase-und Δ-5-Desaturase-Aktivität aufweisen. Vorzugsweise haben die Proteine oder Teile davon, die von dem Nukleinsäuremolekül/den Nukleinsäuremolekülen kodiert wird/werden, noch seine/ihre wesentliche enzymatische Aktivität und die Fähigkeit, am Stoffwechsel von zum Aufbau von Zellmembranen oder Lipidkörperchen in Organismen vorteilhaft in Pflanzen notwendigen Verbindungen oder am Transport von Molekülen über diese Membranen teilzunehmen. Vorteilhaft sind die von den Nukleinsäuremolekülen kodierten Proteine zu mindestens etwa 70 %, 80 % oder 90 % und am stärksten bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr identisch zu den in SEQ ID NO: 2, SEQ ID NO: 4, SEQ ID NO: 6, SEQ ID NO: 8 oder SEQ ID NO: 10 dargestellten Aminosäuresequenzen. Im Sinne der Erfindung ist unter Homologie oder homolog, Identität oder identisch zu verstehen.

Die Homologie wurde über den gesamten Aminosäure- bzw. Nukleinsäuresequenzbereich berechnet. Für das Vergleichen verschiedener Sequenzen stehen dem Fachmann eine Reihe von Programmen, die auf verschiedenen Algorithmen beruhen zur Verfügung. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Diese Einstellungen wurden, falls nicht anders angegeben, immer als Standardeinstellungen für Sequenzvergleiche verwendet wurden.

Unter wesentlicher enzymatischer Aktivität der im erfindungsgemäßen Verfahren verwendeten ω-3-Desaturase, Δ-6-Desaturase, Δ-6-Elongase, und Δ-5-Desaturase ist zu verstehen, dass sie gegenüber den durch die Sequenz mit SEQ ID NO: 1, SEQ ID NO: 3, SEQ ID NO: 5, SEQ ID NO: 7 oder SEQ ID NO: 9 und deren Derivate codierten Proteinen/Enzymen im Vergleich noch mindestens eine enzymatische Aktivität von mindestens 10 %, bevorzugt 20 %, besonders bevorzugt 30 % und ganz besonders 40 % aufweisen und damit am Stoffwechsel von zum Aufbau von Fettsäuren, vorteilhaft Fettsäureester wie Phosphatidylester und/oder Triacylglyceridester in einer Pflanze oder Pflanzenzelle notwendigen Verbindungen oder am Transport von Molekülen über Membranen teilnehmen können, wobei C₁₈- oder C₂₀-Kohlenstoffketten im Fettsäuremolekül mit Doppelbindungen an mindestens zwei, vorteilhaft drei oder vier Stellen gemeint sind.

Vorteilhaft im Verfahren verwendbare Nukleinsäuren stammen aus Bakterien, Pilzen, Diatomeen, Tieren wie Caenorhabditis oder Oncorhynchus oder Pflanzen wie Algen oder Moosen wie den Gattungen Shewanella, Physcomitrella, Thraustochytrium, Fusarium, Phytophthora, Ceratodon, Pytium irregulare, Mantoniella, Ostreococcus, Isochrysis, Aleurita, Muscarioides, Mortierella, Borago, Phaeodactylum, Crypthecodinium, speziell aus den Gattungen und Arten Pytium irregulare, Oncorhynchus mykiss, Xenopus laevis, Ciona intestinalis, Thalassiosira pseudonona, Mantoniella squamata, Ostreococcus sp., Ostreococcus tauri, Euglena gracilis, Physcomitrella patens, Phytophtora infestans, Fusarium graminaeum, Cryptocodinium cohnii, Ceratodon purpureus, Isochrysis galbana, Aleurita farinosa, Thraustochytrium sp., Muscarioides viallii, Mortierella alpina, Borago officinalis, Phaeodactylum tricornutum, Caenorhabditis elegans oder besonders vorteilhaft aus Pytium irregulare, Thraustochytrium sp. oder Thalassiosira pseudonana.

Alternativ können zusätzlich im erfindungsgemäßen Verfahren Nukleotidsequenzen verwendet werden, die für eine Δ-12-Desaturase, Δ-9-Elongase, Δ-8-Desaturase, Δ-5-Elongase oder Δ-4-Desaturase codieren. Die im Verfahren verwendeten Nukleinsäuresequenzen werden vorteilhaft in einer Expressionskassette, die die Expression der Nukleinsäuren in Pflanzen ermöglicht, eingebracht.

Dabei werden die Nukleinsäuresequenzen, die für die A-12-Desaturase, ω-3-Desaturase, Δ-9-Elongase, Δ-6-Desaturase, Δ-8-Desaturase, Δ-6-Elongase, Δ-5-Desaturase, Δ-5-Elongase oder Δ-4-Desaturase codieren, mit einem oder mehreren Regulationssignalen vorteilhafterweise zur Erhöhung der Genexpression funktionell verknüpft. Diese regulatorischen Sequenzen sollen die gezielte Expression der Gene und der Proteinexpression ermöglichen. Dies kann beispielsweise je nach Pflanze bedeuten, dass das Gen erst nach Induktion exprimiert und/oder überexprimiert wird, oder dass es sofort exprimiert und/oder überexprimiert wird. Erfindungsgemäß werden Sequenzen für die Expression verwendet, die eine konstitutive Expression wie der CaMV35S-Promotor in möglichst vielen Geweben der Pflanze ermöglichen. Weitere geeignete Promotoren sind CaMV36S-, CaMV35Smas-, nos-, mas-, ubi-, stpt-, lea- oder Super-Promotoren. Die Expression erfolgt erfindungsgemäß im vegetativen Gewebe wie oben beschrieben mit Hilfe zumindest eines caMV35S-Promotors. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren vorteilhafte Terminatoren sind beispielweise virale Terminatoren wie der 35S-Terminator oder andere. Die im erfindungsgemäßen Verfahren vewendeten Enzyme bzw. Nukleinsäuren, die diese Enzyme kodieren, können in einer oder mehreren Kopien in der Expressionskassette (= Genkonstrukt) enthalten sein. Vorteilhaft liegt nur jeweils eine Kopie der Gene in der Expressionskassette vor. Dieses Genkonstrukt oder die Genkonstrukte können gleichzeitig oder nacheinander in die Pflanze eingebracht werden und zusammen im Wirtsorganismus exprimiert werden. Dabei kann das Genkonstrukt oder die Genkonstrukte in einem - oder mehreren Vektoren inseriert sein und frei in der Zelle vorliegen oder aber im Genom inseriert sein. Es ist vorteilhaft für die Insertion weiterer Gene in die Pflanze, wenn die zu exprimierenden Gene zusammen in einem Genkonstrukt vorliegen.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Vorteilhafte Regulationssequenzen für das neue Verfahren liegen in Promotoren vor, wie den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. Vorteilhaft werden im Verfahren konsitutive Promotoren vewendet. Erfindungsgemäß werden die genannten Sequenzen mit Hilfe mindestens eines konstitutiven CaMV35S-Promotors exprimiert. Aber auch induzierbare Promotoren, wie die in EP-A-O 388 186 (Benzylsulfonamidinduzierbar), Plant J. 2, 1992:397-404 (Gatz et al., Tetracyclin-induzierbar), EP-A-O 335 528 (Abzisinsäure-induzierbar) oder WO 93/21334 (Ethanol- oder Cyclohexenolinduzierbar) beschriebenen Promotoren können im Verfahren verwendet werden.. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylpyrophosphatamidotransferase-Promotor aus Glycine max (Genbank-Zugangsnr. U87999) oder der in EP-A-O 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind die oben genannten konstitutiven Promotoren CaMV35S, CaMV36S, CaMV35Smas, nos, mas, ubi, stpt, lea oder der Super-Promotor.

Es ist im Prinzip möglich, alle natürlichen vorteilhaft konstitutiven Promotoren mit ihren Regulationssequenzen, wie die oben genannten, für das neue Verfahren zu verwenden. Es ist ebenfalls möglich und vorteilhaft, zusätzlich oder alleine synthetische Promotoren zu verwenden.

Die Pflanzengenexpression lässt sich auch über einen chemisch induzierbaren Promotor ermöglichen (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

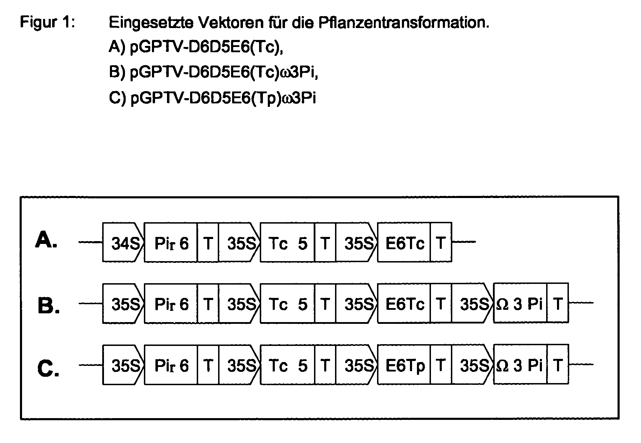

Um eine stabile Integration der Biosynthesegene in die transgene Pflanze über mehrere Generation sicherzustellen, sollte jede der im Verfahren verwendeten Nukleinsäuren, die für die ω-3-Desaturase, Δ-6-Desaturase, Δ-6-Elongase oder Δ-5-Desaturase codieren, unter der Kontrolle eines eigenen Promotors exprimiert werden. Dieser kann vorteilhaft gleich oder unterschiedliche sein. Vorteilhaft werden unterschiedliche Promotoren verwendet, da sich wiederholende Sequenzmotive zu Instabilität der T-DNA bzw. zu Rekombinationsereignissen führen können. Die Expressionskassette ist dabei vorteilhaft so aufgebaut, dass einem Promotor eine geeignete Schnittstelle zur Insertion der zu exprimierenden Nukleinsäure folgt vorteilhaft in einem Polylinker anschließend gegebenenfalls ein Terminator hinter dem Polylinker liegt. Diese Abfolge wiederholt sich mehrfach bevorzugt drei-; vier- oder fünfmal, so dass bis zu fünf Gene in einem Konstrukt zusammengeführt werden und so zur Expression in die transgene Pflanze eingebracht werden können. Vorteilhaft wiederholt sich die Abfolge bis zu viermal. Die Nukleinsäuresequenzen werden zur Expression über die geeignete Schnittstelle beispielsweise im Polylinker hinter den Promotor inseriert. Vorteilhaft hat jede Nukleinsäuresequenz ihren eigenen Promotor und gegebenenfalls ihren eigenen Terminator (siehe Figur 1). Es ist aber auch möglich mehrere Nukleinsäuresequenzen hinter einem Promotor und ggf. vor einem Terminator zu inserieren. Dabei ist die Insertionsstelle bzw. die Abfolge der inserierten Nukleinsäuren in der Expressionskassette nicht von entscheidender Bedeutung, das heißt eine Nukleinsäuresequenz kann an erster oder letzter Stelle in der Kassette inseriert sein, ohne dass dadurch die Expression wesentlich beeinflusst wird. Es können in der Expressionskassette in einer vorteilhaften Ausführungsform unterschiedliche Promotoren wie beispielsweise der USP-, LegB4 oder DC3-Promotor und unterschiedliche Terminatoren verwendet werden. Erfindungsgemäß wird mindestens der CaMV35S-Promotor verwendet (siehe Figur 1).

Wie oben beschrieben sollte die Transkription der eingebrachten Gene vorteilhaft durch geeignete Terminatoren am 3'-Ende der eingebrachten Biosynthesegene (hinter dem Stoppcodon) abgebrochen werden. Verwendet werden kann hier z.B. der OCS1 Terminator. Wie auch für die Promotoren, so sollten hier für jedes Gen unterschiedliche Terminatorsequenzen verwendet werden.

Das Genkonstrukt kann, wie oben beschrieben, auch weitere Gene umfassen, die in die Organismen eingebracht werden sollen. Es ist möglich und vorteilhaft, in die Wirtspflanzen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Weiterhin können vorteilhaft im Nukleinsäurekonstrukt bzw. Genkonstrukt weitere Biosynthesegene des Fettsäure- oder Lipidstoffwechsets enthalten sein oder aber diese Gene können auf einem weiteren oder mehreren weiteren Nukleinsäurekonstrukten liegen. Vorteilhaft werden als Biosynthesegene des Fettsäure- oder Lipidstoffwechsels ein Gen ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) oder deren Kombinationen verwendet. Besonders vorteilhafte Nukleinsäuresequenzen sind Biosynthesegene des Fettsäure- oder Lipidstoffwechsels ausgewählt aus der Gruppe der Acyl-CoA:Lysophospholipid-Acyltransferase, Δ-4-Desaturase, Δ-8-Desatuase, Δ-9-Desaturase, A-12-Desaturase, Δ-5-Elongase und/oder Δ-9-Elongase.

Dabei können die vorgenannten Nukleinsäuren bzw. Gene in Kombination mit anderen Elongasen und Desaturasen in Expressionskassetten, wie den vorgenannten, kloniert werden und zur Transformation von Pflanzen Mithilfe von Agrobakterium eingesetzt werden.

Der in dieser Beschreibung verwendete Begriff "Vektor" bedeutet ein Nukleinsäuremolekül, das eine andere Nukleinsäure transportieren kann, an welche es gebunden ist. Ein Vektortyp ist ein "Plasmid", was für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch diese anderen Expressionsvektorformen, wie virale Vektoren, die ähnliche Funktionen ausüben, umfassen. Ferner soll der Begriff Vektor auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, umfassen.

Die im Verfahren vorteilhaft verwendeten rekombinanten Expressionsvektoren umfassen die im Verfahren verwendeten Nukleinsäuresequenzen oder das oben beschriebene Genkonstrukt in einer Form, die sich zur Expression der verwendeten Nukleinsäuren in einer Wirtszelle eignen, was bedeutet, dass die rekombinanten Expressionsvektoren eine oder mehrere Regulationssequenzen, ausgewählt auf der Basis der zur Expression zu verwendenden Wirtszellen, die mit der zu exprimierenden Nukleinsäuresequenz funktionsfähig verbunden ist, umfasst. In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz möglich ist und sie aneinander gebunden sind, so dass beide Sequenzen die vorhergesagte, der Sequenz zugeschriebene Funktion erfüllen (z.B. in einem In-vitro-Transkriptions-lTranslationssystem oder in einer Wirtszelle, wenn der Vektor in die Wirtszelle eingebracht wird). Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem Ausmaß der Expression des gewünschten Proteins usw., abhängen kann.

Die verwendeten rekombinanten Expressionsvektoren können zur Expression der im Verfahren verwendeten Nukleinsäuresequenzen so gestaltet sein, dass sie in prokaryotischen Zwischenwirten transformiert werden können und schließlich nach Einbringung in die Pflanzen die Expression in diesen ermöglichen. Dies ist vorteilhaft, da häufig Zwischenschritte der Vektorkonstruktion der Einfachheithalber in Mikroorganismen durchgeführt werden. Beispielsweise können die ω-3-Desaturase-, Δ-6-Desaturase-, Δ-6-Elongase- und/oder Δ-5-Desaturase-Desaturase-Gene in bakteriellen Zellen, Insektenzellen (unter Verwendung von Baculovirus-Expressionsvektoren), Hefe- und anderen Pilzzellen (siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al., 1999, Marine Biotechnology.1, 3:239-251), Ciliaten, mit Vektoren nach einem Transformationsverfahren, wie beschrieben in WO 98/01572, sowie bevorzugt in Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird.

Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11 d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL21 (DE3) oder HMS174 (DE3) von einem residenten λ-Prophagen bereitgestellt, der ein T7 gn1-Gen unter der Transkriptionskontrolle des lacUV 5-Promotors birgt.

Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III113-B1, λgt11 or pBdCl, in Streptomyces pIJ101, pIJ364, pIJ702 oder pIJ361, in Bacillus pUB110, pC194 oder pBD214, in Corynebacterium pSA77 oder pAJ667.

Bei einer weiteren Ausführungsform ist der Expressionsvektor ein Hefe-Expressionsvektor. Beispiele für Vektoren zur Expression in der Hefe S. cerevisiae umfassen pYeDesaturasec1 (Baldari et al. (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise pAG-1, YEp6, YEp13 oder pEMBLYe23.

Alternativ können die im erfindungsgemäßen Verfahren verwendeten Nukleinsäuresequenzen in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol.. 3:2156-2165) und die pVL-Reihe (Lucklow und Summers (1989) Virology 170:31-39).

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Auch können die im Verfahren verwendeten Gen in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignate sind diejenigen, die aus Agrobacterium tumefaciens-T-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet.

Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711).

Die Pflanzengenexpression muss wie oben beschrieben funktionsfähig mit einem geeigneten Promotor verbunden sein, der die Genexpression auf rechtzeitige, zell- oder gewebespezifische Weise durchführt. Vorteilhaft nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alpha-Amylase-Promotor aus Kartoffel (WO 96/12814) oder der durch Wunden induzierbare pinll-Promotor (EP-A-0 375 091).

Insbesondere kann die multiparallele Expression der im Verfahren verwendeten w-3-Desaturasen, Δ-6-Desaturasen, Δ-6-Elongasen und/oder Δ-5-Desaturasen gewünscht sein. Die Einführung solcher Expressionskassetten kann über eine simultane Transformation mehrerer einzelner Expressionskonstrukte erfolgen oder bevorzugt durch Kombination mehrerer Expressionskassetten auf einem Konstrukt. Auch können mehrere Vektoren mit jeweils mehreren Expressionskassetten transformiert und auf die Wirtszelle übertragen werden.

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Vektor-DNA lässt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Der Begriff "Nukleinsäure(molekül)", wie hier verwendet, umfasst in einer vorteilhaften Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das im Verfahren vewendete isolierte w-3-Desaturase-, Δ-6-Desaturase-, Δ-6-Elongase- oder Δ-5-Desaturasemolekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt flankieren.

Die im Verfahren verwendeten Nukleinsäuremoleküle kann unter Verwendung molekularbiologischer Standardtechniken und der hier bereitgestellten Sequenzinformation isoliert werden. Auch kann Mithilfe von Vergleichsalgorithmen beispielsweise eine homologe Sequenz oder homologe, konservierte Sequenzbereiche auf DNA oder Aminosäureebene identifiziert werden. Diese können als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) zur Isolierung weiterer im Verfahren nützlicher Nukleinsäuresequenzen verwendet werden. Überdies lassen sich die im Verfahren verwendeten Nukleinsäuremoleküle oder Teile von diesen, durch Polymerasekettenreaktion isolieren, wobei Oligonukleotidprimer, die auf der Basis dieser Sequenz oder von Teilen davon, verwendet werden (z.B. kann ein Nukleinsäuremolekül, umfassend die vollständigen Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis einer der in SEQ ID NO: 1, SEQ ID NO: 3, SEQ ID NO: 5, SEQ ID NO: 7 oder SEQ ID NO: 9 gezeigten Sequenzen oder Mithilfe der in SEQ ID NO: 2, SEQ ID NO: 4, SEQ ID NO: 6, SEQ ID NO: 8 oder SEQ ID NO: 10 dargestellten Aminosäuresequenzen erstellen. Eine erfindungsgemäße Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Desaturase-Nukleotidsequenz entsprechen, können durch Standard-Syntheseverfahren, beispielsweise mit einem automatischen DNA-Synthesegerät, hergestellt werden.

Homologe der verwendeten ω-3-Desaturase-, Δ-6-Desaturase-, Δ-6-Elongase-, oder Δ-5-Desaturase-Nukleinsäuresequenzen mit der Sequenz SEQ ID NO: 1, SEQ ID NO: 3, SEQ ID NO: 5, SEQ ID NO: 7 oder SEQ ID NO: 9 bedeutet beispielsweise allelische Varianten mit mindestens etwa 70 oder 80 %, 90 % oder 95 % und noch stärker bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr Identität bzw. Homologie zu einer in SEQ ID NO: 1, SEQ ID NO: 3, SEQ ID NO: 5, SEQ ID NO: 7 oder SEQ ID NO: 9 gezeigten Nukleotidsequenzen oder ihren Homologen, Derivaten oder Analoga oder Teilen davon. Weiterhin sind isolierte Nukleinsäuremoleküle einer Nukleotidsequenz, die an eine der in SEQ ID NO: 1, SEQ ID NO: 3, SEQ ID NO: 5, SEQ ID NO: 7 oder SEQ ID NO: 9 gezeigten Nukleotidsequenzen oder einen Teil davon hybridisieren, z.B. unter stringenten Bedingungen hybridisiert. Unter einem Teil gemäß der Erfindung ist dabei zu verstehen, dass mindestens 25 Basenpaare (= bp), 50 bp, 75 bp, 100 bp, 125 bp oder 150 bp, bevorzugt mindestens 175 bp, 200 bp, 225 bp, 250 bp, 275 bp oder 300 bp, besonders bevorzugt 350 bp, 400 bp, 450 bp, 500 bp oder mehr Basenpaare für die Hybridisierung verwendet werden. Es kann auch vorteilhaft die Gesamtsequenz verwendet werden. Allelische Varianten umfassen insbesondere funktionelle Varianten, die sich durch Deletion, Insertion oder Substitution von Nukleotiden aus der in SEQ ID NO: 1, SEQ ID NO: 3, SEQ ID NO: 5, SEQ ID NO: 7 oder SEQ ID NO: 9 dargestellten Sequenz erhalten lassen, wobei aber die Absicht ist, dass die Enzymaktivität der davon herrührenden synthetisierten Proteine für die Insertion eines oder mehrerer Gene vorteilhafterweise beibehalten wird. Proteine, die noch die enzymatische Aktivität der ω-3-Desaturase, Δ-6-Desaturase, Δ-6-Eiongase oder Δ-5-Desaturase besitzen, das heißt deren Aktivität im wesentlichen nicht reduziert ist, bedeutet Proteine mit mindestens 10 %, vorzugsweise 20 %, besonders bevorzugt 30 %, ganz besonders bevorzugt 40 % der ursprünglichen Enzymaktivität, verglichen mit dem durch SEQ ID NO: 2, SEQ ID NO: 4, SEQ ID NO: 6, SEQ ID NO: 8 oder SEQ ID NO: 10 kodierten Protein. Die Homologie wurde über den gesamten Aminosäure- bzw. Nukleinsäuresequenzbereich berechnet. Für das Vergleichen verschiedener Sequenzen stehen dem Fachmann eine Reihe von Programmen, die auf verschiedenen Algorithmen beruhen zur Verfügung. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Die falls nicht anders angegeben als Standardeinstellungen immer für Sequenzvergleiche verwendet wurden.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe wie der Phosphatidylrest. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend wieder für die weitere Elongationen aus den Phospholipiden in den FettsäureCoA-Ester-Pool überführt werden. Dies ermöglichen Acyl-CoA:Lysophospholipid-Acyltransferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden.

Vorläufer für die PUFA-Biosynthese sind beispielsweise Ölsäure, Linol- und Linolensäure. Diese C₁₈-Kohlenstoff-Fettsäuren müssen zu den C₂₀-Fettsäuren verlängert werden, damit ARA und EPA erhalten wird. Mithilfe der im Verfahren verwendeten Desaturasen wie der w3-, Δ-6- und Δ-6-Desaturasen und/oder Δ-6-Elongasen können Eicosapentaensäure oder Arachidonsäure und Eicosapentaensäure hergestellt werden und anschließend für verschiedene Zwecke bei Nahrungsmittel-, Futter-, Kosmetik- oder pharmazeutischen Anwendungen verwendet werden. Mit den genannten Enzymen können C₂₀- Fettsäuren mit mindestens zwei vorteilhaft mindestens drei, vier oder fünf Doppelbindungen im Fettsäuremolekül, vorzugsweise C₂₀-Fettsäuren mit vorteilhaft vier oder fünf Doppelbindungen im Fettsäuremolekül hergestellt werden. Die Desaturierung kann vor oder nach Elongation der entsprechenden Fettsäure erfolgen. Daher führen die Produkte der Desaturaseaktivitäten und der möglichen weiteren Desaturierung und Elongation zu bevorzugten PUFAs mit höherem Desaturierungsgrad zu Fettsäuren wie γ-Linolensäure, Dihomo-γ-linolensäure, Arachidonsäure, Stearidonsäure, Eicosatetraensäure oder Eicosapentaensäure. Substrate der verwendeten Desaturasen und Elongasen im erfindungsgemäßen Verfahren sind C₁₆- oder C₁₈-Fettsäuren wie zum Beispiel Linolsäure, γ-Linolensäure, α-Linolensäure, Dihomo-γ-linolensäure, Eicosatetraensäure oder Stearidonsäure. Bevorzugte Substrate sind Ölsäure, Linolsäure, γ-Linolensäure und/oder α-Linolensäure. Die synthetisierten C₂₀-Fettsäuren mit mindestens zwei, drei, vier oder fünf Doppelbindungen in der Fettsäure fallen im erfindungsgemäßen Verfahren in Form der freien Fettsäure oder in vorteilhaft in Form ihrer Ester beispielsweise in Form ihrer Glyceride oder Phospholipide an.

Unter dem Begriff "Glycerid wird ein mit ein, zwei oder drei Carbonsäureresten verestertes Glycerin verstanden (Mono-, Di- oder Triglycerid). Unter "Glycerid" wird auch ein Gemisch an verschiedenen Glyceriden verstanden. Vorteilhaft handelt es sich beim Glycerid um das Triglycerid. Das Glycerid oder das Glyceridgemisch kann weitere Zusätze, z.B. freie Fettsäuren, Antioxidantien, Proteine, Kohlenhydrate, Vitamine und/oder andere Substanzen enthalten.

Unter einem "Glycerid" im Sinne des erfindungsgemäßen Verfahrens werden ferner vom Glycerin abgeleitete Derivate verstanden. Dazu zählen neben den oben beschriebenen Fettsäureglyceriden auch Glycerophospholipide und Glyceroglycolipide. Bevorzugt seien hier die Glycerophospholipide wie Lecithin (Phosphatidylcholin), Cardiolipin, Phosphatidylglycerin, Phosphatidylserin und Alkylacylglycerophospholipide beispielhaft genannt.

Ferner müssen Fettsäuren anschließend an verschiedene Modifikationsorte transportiert und in das vorteilhafte Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und -Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Die im Verfahren hergestellten PUFAs, umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden, beispielsweise können Katzen Arachidonsäure nicht mehr synthetisieren.

Unter Phospholipiden im Sinne der Erfindung sind zu verstehen Phosphatidylcholin, Phosphatidylethanolamin, Phosphatidylserin, Phosphatidylglycerin und/oder Phosphatidylinositol vorteilhafterweise Phosphatidylcholin und/oder Phosphatidylserin. Die Begriffe Produktion oder Produktivität sind im Fachgebiet bekannt und beinhalten die Produktivität innerhalb einer Pflanzenzelle oder einer Pflanze, das heißt den Gehalt an den gewünschten im Verfahren hergestellten Fettsäuren bezogen auf den Gehalt an allen Fettsäuren in dieser Zelle oder Pflanze. Der Begriff Effizienz der Produktion umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Die Begriffe Biosynthese oder Biosyntheseweg sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess. Die Begriffe Abbau oder Abbauweg sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und stark regulierten Prozess. Der Begriff Stoffwechsel ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Für das erfindungsgemäße Verfahren vorteilhafte Nukleinsäuremoleküle können auf der Grundlage ihrer Homologie zu den hier offenbarten ω-3-Desaturase-, Δ-6-Desaturase-, Δ-5-Desaturase-, und/oder Δ-6-Elongase-Nukleinsäuren unter Verwendung der Sequenzen oder eines Teils davon als Hybridisierungssonde gemäß Standard-Hybridisierungstechniken unter stringenten Hybridisierungsbedingungen isoliert werden. Dabei können beispielsweise isolierte Nukleinsäuremoleküle verwendet werden, die mindestens 15 Nukleotide lang sind und unter stringenten Bedingungen mit dem Nukleinsäuremolekülen, die eine Nukleotidsequenz der SEQ ID NO: 1, SEQ ID NO: 3, SEQ ID NO: 5, SEQ ID NO: 7 oder SEQ ID NO: 9 umfassen, hybridisieren. Es können auch Nukleinsäuren mindestens 25, 50, 100, 250 oder mehr Nukleotide verwendet werden. Der Begriff "hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, dass Sequenzen, die mindestens etwa 65 %, stärker bevorzugt mindestens etwa 70 % und noch stärker bevorzugt mindestens etwa 75 % oder stärker zueinander homolog sind, gewöhnlich aneinander hybridisiert bleiben. Diese stringenten Bedingungen sind dem Fachmann bekannt und lassen sich in Current Protocols in Molecular Biology, John Wiley & Sons, N. Y. (1989), 6.3.1-6.3.6., finden. Ein bevorzugtes, nicht einschränkendes Beispiel für stringente Hybridisierungsbedingungen sind Hybridisierungen in 6 x Natriumchlorid/Natriumcitrat (sodium chloride/sodiumcitrate = SSC) bei etwa 45°C, gefolgt von einem oder mehreren Waschschritten in 0,2 x SSC, 0,1 % SDS bei 50 bis 65°C. Dem Fachmann ist bekannt, dass diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheiden. Die Temperatur unterscheidet sich beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel im obengenannten Puffer vorliegt, zum Beispiel 50 % Formamid, ist die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise zwischen 30°C und 45°C. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt. Der Fachmann weiß, wie die erforderlichen Hybridisierungsbedingungen anhand von Lehrbüchern, wie dem vorstehend erwähnten oder aus den folgenden Lehrbüchern Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können.

Zur Bestimmung der prozentualen Homologie (= Identität) von zwei Aminosäuresequenzen (z.B. einer der Sequenzen der SEQ ID NO: 2, SEQ ID NO: 4, SEQ ID NO: 6, SEQ ID NO: 8 oder SEQ ID NO: 10) oder von zwei Nukleinsäuren (z.B. SEQ ID NO: 1, SEQ ID NO: 3, SEQ ID NO: 5, SEQ ID NO: 7 oder SEQ ID NO: 9) werden die Sequenzen zum Zweck des optimalen Vergleichs untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäurereste oder Nukleotide an den entsprechenden Aminosäurepositionen oder Nukleotidpositionen werden dann verglichen. Wenn eine Position in einer Sequenz durch den gleichen Aminosäurerest oder das gleiche Nukleotid wie die entsprechende Stelle in der anderen Sequenz belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an identischen Positionen, die den Sequenzen gemeinsam sind (d.h. % Homologie = Anzahl der identischen Positionen/Gesamtanzahl der Positionen x 100). Die Begriffe Homologie und Identität sind damit als Synonym anzusehen. Die verwendeten Programme bzw. Algorithmen sind oben beschrieben.

Ein isoliertes Nukleinsäuremolekül, das für eine im Verfahren verwendete ω-3-Desaturase, Δ-6-Desaturase, Δ-5-Desaturase und Δ-6-Elongase kodiert, die zu einer Proteinsequenz der SEQ ID NO: 2, SEQ ID NO: 4, SEQ ID NO: 6, SEQ ID NO: 8 oder SEQ ID NO: 10 homolog ist, kann durch Einbringen einer oder mehrerer Nukleotidsubstitutionen, -additionen oder -deletionen in eine Nukleotidsequenz der SEQ ID NO: 1, SEQ ID NO: 3, SEQ ID NO: 5, SEQ ID NO: 7 oder SEQ ID NO: 9 erzeugt werden, so dass eine oder mehrere Aminosäuresubstitutionen, -additionen oder -deletionen in das kodierte Protein eingebracht werden. Mutationen können in eine der Sequenzen der SEQ ID NO: 1, SEQ ID NO: 3, SEQ ID NO: 5, SEQ ID NO: 7 oder SEQ ID NO: 9 durch Standardtechniken, wie stellenspezifische Mutagenese und PCR-vermittelte Mutagenese, eingebracht werden. Vorzugsweise werden konservative Aminosäuresubstitutionen an einer oder mehreren der vorhergesagten nicht-essentiellen Aminosäureresten hergestellt. Bei einer "konservativen Aminosäuresubstitution" wird der Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), unpolaren Seitenketten, (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einer w-3-Desaturase, Δ-6-Desaturase, Δ-5-Desaturase oder Δ-6-Elongase wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der ω-3-Desaturase, Δ-6-Desaturase, Δ-5-Desaturase oder Δ-6-Elongase kodierenden Sequenz eingebracht werden, z.B. durch Sättigungsmutagenese, und die resultierenden Mutanten können nach der hier beschriebenen ω-3-Desaturase-, Δ-6-Desaturase-, Δ-5-Desaturase- oder Δ-6-Elongase-Aktivität durchmustert werden, um Mutanten zu identifizieren, die die w-3-Desaturase-, Δ-6-Desaturase-, Δ-5-Desaturase- oder Δ-6-Elongase-Aktivität beibehalten haben. Nach der Mutagenese kann das kodierte Protein rekombinant exprimiert werden, und die Aktivität des Proteins kann z.B. unter Verwendung der hier beschriebenen Tests bestimmt werden.

Diese Erfindung wird durch die nachstehenden Beispiele weiter veranschaulicht, die nicht als beschränkend aufgefasst werden sollten.

### Beispiele

### Beispiel 1: Allgemeine Klonierungsverfahren:

Die Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

### Beispiel 2: Sequenzanalyse rekombinanter DNA:

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA74, 5463-5467). Fragmente resultierend aus einer Polymerase Kettenreaktion wurden zur Vermeidung von Polymerasefehlern in zu exprimierenden Konstrukten sequenziert und überprüft.

### Beispiel 3: Klonierung von Expressionsplasmiden zur Expression in Pflanzen

Für die Transformation von Pflanzen wurden Transformationsvektoren auf Basis von pGPTV-35S, eines Plasmids basierend auf pBIN19-35S (Bevan M. (1984) Binary Agrobacterium vectors for plant transformation. Nucl. Acids Res. 18:203) erzeugt. Dazu wurde in einem pUC-Vektor eine Expressionskassette bestehend aus dem Promotor-Element CaMV35S (SEQ ID NO: 11) sowie dem 35S-Terminator (SEQ ID NO: 12; Franck,A., Guilley,H., Jonard,G., Richards,K. and Hirth,L. (1980) Nucleotide sequence of cauliflower mosaic virus DNA Cell 21 (1), 285-294) zusammengesetzt. Dabei wurde der Promotor über die Restriktionsschnittstellen *Sal*I/*Xba*I und der Terminator über *Bam*HI/*Sma*I eingesetzt. An den Terminator wurde dabei ein Polylinker mit der *Xho*I Schnittstelle angehängt (,triple ligation'). Das entstandene Plasmid pUC19-35S wurde dann für die Klonierung von PUFA Genen eingesetzt. Es wurden parallel die offene Leserahmen der d6Des(Pir, SEQ ID NO: 1), d5Des(Tc, SEQ ID NO: 5) und d6Elo(Tc, SEQ ID NO: 3) Sequenzen über EcoRV in pUC19-35S Vektoren eingefügt. Die entstandenen Plasmide pUC-D6, pUC-D5, pUC-E6(Tc) wurden für die Erstellung des binären Vektors pGPTV-35S_D6D5E6(Tc) verwendet. Dazu wurde der Vektor pGPTV mit dem Enzym *Sal*I, das Plasmid pUC-D6 mit *Sal*I/*Xho*I verdaut und die korrekten Fragmente ligiert. Das entstandene Plasmid pGPTV-D6 wurde anschliessend mit *Sal*I, das Plasmid pUC-D5 mit *Sal*I/*Xho*I verdaut und die korrekten Fragmente ligiert. Das entstandene Plasmid pGPTV-D6-D5 wurde dann ein weiteres Mal mit SalI verdaut, das Plasmid pUC-E6(Tc) mit *Sal*I/*Xho*I und die korrekten Fragmente ligiert. Aus diesen sequentiellen Klonierungsschritten entstand der binäre Vektor pGPTV-D6D5E6(Tc), der für die Transformation eingesetzt wurde.

In einer weiteren Ausführung wurde an Stelle der Sequenz d6Elo(Tc) die Sequenz von d6Elo(Tp) [SEQ ID NO: 7] in den Vektor pUC19-35S eingesetzt. Das entstandene Plasmid pUC- E6(Tp) wurde zur Herstellung des binären Vektors pGPTV-35S_D6D5E6(Tp) verwendet.

In einer weiteren Ausführung wurde der offene Leserahmen der ω3Des(Pi, SEQ ID NO: 9) in pUC19-35S kloniert. Das entstandene Plasmid pUC-ω3Pi wurde über *Sal*I/*Xho*I in die binären Vektoren pGPTV-D6D5E6(Tc) und pGPTV-D6D5E6(Tp) übertragen. Die entstandene Vektoren pGPTV-D6D5E5(Tc)ω3Pi und pGPTV-D6D5E5(Tp)ω3Pi wurde für die Pflanzentransformation eingesetzt.

Fig. 1 gibt einen Überblick über die erstellten Konstrukte.

Alle binären Vektoren wurden in E. coli DH5α-Zellen (Invitrogen) nach Herstellerangaben transformiert. Positive Klone wurden durch PCR identifiziert und Plasmid-DNA isoliert (Qiagen Dneasy).

Zusammensetzung des PCR-Ansatzes (50 µL):
5,00 µL Template cDNA
5,00 µL 10x Puffer (Advantage-Polymerase)+ 25mM MgCl₂
5,00 µL 2mM dNTP
1,25 µL je Primer (10 pmol/µL)
0,50 µl Advantage-Polymerase

Die Advantage-Polymerase von Clontech wird eingesetzt.

Die überprüften Vektoren wurden dann in *Agrobacterium tumefaciens* GC3101 durch Elektroporation transformiert und auf Agar-Platten mit 2% YEB Medium+Kanamycin plattiert. Kanamycin-tolerante Zellen wurden ausgewählt und für die Transformation von *Brassica juncea* und *Arabidopsis thaliana* eingesetzt bzw. können für die Transformation anderer Pflanzenarten eingesetzt werden.

### Beispiel 4: Erzeugung von transgenen Pflanzen

### a) Erzeugung transgener Rapspflanzen (Brasscia juncea, verändert nach Radke et al., Transformation and regenerationof Brassica rapa using Agrobacterium tumefaciens. Plant Cell Rep. 11, 499-505, 1992).

Zur Erzeugung transgener Rapspflanzen werden binäre Vektoren wie die unter Beispiel 3 erzeugten binären Plasmide/Vektoren in *Agrobacterium tumefaciens* GC3101 transformiert (Deblaere et al, 1984, Nucl. Acids. Res. 13, 4777-4788). Zur Transformation von Rapspflanzen (Var. Drakkar, NPZ Nordeutsche Pflanzenzucht, Hohenlieth, Deutschland), wird eine 1:50 Verdünnung einer Übernachtkultur einer positiv transformierten Agrobakterienkolonie in Murashige-Skoog Medium (Murashige und Skoog 1962 Physiol. Plant. 15, 473) mit 3 % Saccharose (3MS-Medium) benutzt. Petiolen oder Hypokotyledonen frisch gekeimter steriler Rapspflanzen (zu je ca. 1 cm²) wurden in einer Petrischale mit einer 1:50 Agrobakterienverdünnung für 5-10 Minuten inkubiert. Es folgt eine 3-tägige Co-Inkubation in Dunkelheit bei 25°C auf 3MS-Medium mit 0,8 % Bacto-Agar. Die Kultivierung wurde nach 3 Tagen mit 16 Stunden Licht/8 Stunden Dunkelheit weitergeführt und in wöchentlichem Rhythmus auf MS-Medium mit 500 mg/l Claforan (Cefotaxime-Natrium), 50 mg/l Kanamycin, 20 mikroM Benzylaminopurin (BAP) und 1,6 g/l Glukose weitergeführt. Wachsende Sprosse wurden auf MS-Medium mit 2 % Saccharose, 250 mg/l Claforan und 0,8 % Bacto-Agar überführt. Bildeten sich nach drei Wochen keine Wurzeln, so wurde als Wachstumshormon 2-Indolbuttersäure zum Bewurzeln zum Medium gegeben.

Regenerierte Sprosse wurden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf Expression der Desaturase- bzw. Elongase-Gene mittels Lipidanalysen untersucht wie beispielhaft in Qiu et al. 2001, J. Biol. Chem. 276, 31561-31566 beschrieben.

### b) Herstellung von transgenen Leinpflanzen

Die Herstellung von transgenen Leinpflanzen können zum Beispiel nach der Methode von Bell et al., 1999, In Vitro Cell. Dev. Biol.-Plant. 35(6):456-465 mittels particle bombartment erzeugt werden. Agrobakterien-vermittelte Transformationen können zum Beispiel nach Mlynarova et al. (1994), Plant Cell Report 13: 282-285 oder nach Drexler et al. (2003), Mol. Breeding 11, 149-158 hergestellt werden.

### Beispiel 5: Lipidextraktion aus Blättern von Brassica juncea und Arabidopsis thaliana

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22): 12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Zur Analyse von transgenen Brassica juncea bzw. Arabidopsis thaliana Pflanzen wurde das Pflanzenmaterial zunächst mechanisch durch Mörsern homogenisiert, um es einer Extraktion zugänglicher zu machen. Dann wurde 10 min auf 100°C erhitzt und nach dem Abkühlen auf Eis erneut sedimentiert. Das Zellsediment wurde mit 1 M methanolischer Schwefelsäure und 2 % Dimethoxypropan 1 h bei 90°C hydrolysiert und die Lipide transmethyliert. Die resultierenden Fettsäuremethylester (FAME) wurden in Petrolether extrahiert. Die extrahierten FAME wurden durch Gasflüssigkeitschromatographie mit einer Kapillarsäule (Chrompack, WCOT Fused Silica, CP-Wax-52 CB, 25 m, 0,32 mm) und einem Temperaturgradienten von 170°C auf 240°C in 20 min und 5 min bei 240°C analysiert. Die Identität der Fettsäuremethylester wurde durch Vergleich mit entsprechenden FAME-Standards (Sigma) bestätigt. Die Identität und die Position der Doppelbindung kann durch geeignete chemische Derivatisierung der FAME-Gemische z.B. zu 4,4-Dimethoxyoxazolin-Derivaten (Christie, 1998) mittels GC-MS weiter analysiert werden.

Analyse von Blattmaterial trangener *Brasscia juncea* Pflanzen mit dem Konstrukt pGPTV-D6D5E6(Tc):
Pflanzen, die wie in Beispiel 4 mit dem Plasmid pGPTV-D6D5E6(Tc) transformiert wurden, wurden auf veränderte Fettsäuren untersucht. Als Ausgangsmaterial wurden Blätter eingesetzt, die entsprechend obiger Beschreibung extrahiert und gaschromatographisch analysiert wurden.

In den transgenen Pflanzen konnte dabei die Entstehung neuer Fettsäuren gezeigt werden (Tab. 1, Vergleich zur Kontrolle). Die Tabelle 1 ist am Ende der Beschreibung wieder gegeben. Die Analyse zahlreicher unabhängiger transgener Linie zeigte, dass im Blattmaterial durch die Aktivität der eingesetzten Gene die Fettsäuren DGLA (γ18:3), SDA (18:4), DGLA (20:3d8,11,14), AA (20:4) und EPA (20:5) entstanden sind. Diese Fettsäuren sind in nicht transformierten Pflanzen (,Kontrolle') nicht vorhanden. Arachidonsäure (AA) und Eicosapentaensäure (EPA) stellen dabei wertvolle Fettsäuren für die menschliche Ernährung dar. Von diesen beiden wertvollen Fettsäuren sind bis zu 8,9% bzw. 4,8% vorhanden.

EPA stellt eine besonders wertvolle Fettsäure dar. Aus diesem Grund wurden Ansätze durchgeführt, um den Gehalt an EPA zu steigern. Ein erster Ansatz war die Verwendung einer ARA-spezifischen ω3-Desaturase, die ARA zu EPA umsetzt. In einem weiteren Ansatz wurde die Verwendung einer 18:4-spezifischen Elongase in Kombination mit der ω-Desaturase getestet.

Analyse von Blattmaterial trangener *Brasscia juncea* Pflanzen mit dem Konstrukt pGPTV-D6D5E6(Tc)ω3Pi:
Pflanzen, die wie in Beispiel 4 mit dem Plasmid pGPTV-D6D5E6(Tc)ω3Pi transformiert wurden, wurden auf veränderte Fettsäuren untersucht. Als Ausgangsmaterial wurden Blätter eingesetzt, die entsprechend obiger Beschreibung extrahiert und gaschromatographisch analysiert wurden.

In den transgenen Pflanzen konnte dabei die Entstehung neuer Fettsäuren gezeigt werden (Tab. 2, Vergleich zur Kontrolle). Tabelle 2 wird am Ende der Beschreibung wiedergegeben. Die Analyse zahlreicher unabhängiger transgener Linie zeigte, dass im Blattmaterial durch die Aktivität der eingesetzten Gene die Fettsäuren GLA (γ18:3), SDA (18:4), 20:4d8,11,14,17 und EPA (20:5) entstanden sind. Diese Fettsäuren sind in nicht transformierten Pflanzen ('Kontrolle') nicht vorhanden. Eicosapentaensäure (EPA) stellt dabei eine wertvolle Fettsäuren für die menschliche Ernährung dar. Von dieser wertvollen Fettsäuren sind bis zu 12,5% vorhanden. Der zusätzliche Einsatz von ω3Pi reduziert dabei deutlich den Gehalt an AA und verschiebt die Reaktion in Richtung des wertvolleren Produktes EPA.

Analyse von Blattmaterial trangener *Brasscia juncea* Pflanzen mit dem Konstrukt pGPTV-D6D5E6(Tp)ω3Pi:
Pflanzen, die wie in Beispiel 4 mit dem Plasmid pGPTV-D6D5E6(Tp)ω3Pi transformiert wurden, wurden auf veränderte Fettsäuren untersucht. Als Ausgangsmaterial wurden Blätter eingesetzt, die entsprechend obiger Beschreibung extrahiert und gaschromatographisch analysiert wurden.

In den transgenen Pflanzen konnte dabei die Entstehung neuer Fettsäuren gezeigt werden (Tab. 3, Vergleich zur Kontrolle). Tabelle 3 wird am Ende der Beschreibung wiedergegeben. Die Analyse zahlreicher unabhängiger transgener Linie zeigte, dass im Blattmaterial durch die Aktivität der eingesetzten Gene die Fettsäuren GLA (γ18:3), SDA (18:4), 20:4d8,11,14,17 und EPA (20:5) entstanden sind. Diese Fettsäuren sind in nicht transformierten Pflanzen (,Kontrolle') nicht vorhanden. Eicosapentaensäure (EPA) stellt dabei eine wertvolle Fettsäuren für die menschliche Ernährung dar. Von dieser wertvollen Fettsäuren sind bis zu 14,7% vorhanden. Der zusätzliche Einsatz von ω3Pi reduziert dabei deutlich den Gehalt an ARA und verschiebt die Reaktion in Richtung des wertvolleren Produktes EPA. Im Vergleich zum Konstrukt pGPTV-D6D5E6(Tc)ω3Pi konnten höhere Gehalte an EPA (20:5) erzielt werden.

Dabei konnte gezeigt werden, dass die Synthese der wertvollen Fettsäure EPA (20:5) bevorzugt in Blättern stattfindet (Tab. 4). In den anderen Pflanzenorganen Samen, Stengel und Blüte sind nur geringste Mengen an EPA und den Vorstufen nachzuweisen.

In einer weiteren Ausführung wurde untersucht ob die wertvolle Fettsäure EPA in einer bestimmten Lipidklasse bzw. bestimmten Lipidklassen angereichert wird. Dazu wurden die Gesamtlipid wie oben beschrieben aus Blättern extrahiert und dann aufgetrennt nach Neutral- und Polarlipiden mittels einer Silikat PrepSep Säule (Fisher Scientific, USA). Die Neutrallipid-Fraktion wurde in einem weiteren Schritt mittels Dünnschichtchromatographie (G-25, Machery-Nagel) die Triacylglyceride aufgetrennt (Hexan/Diethylether/Essigsäure 70:30:1, vol/vol/vol). Aliquots der Polarlipid-Fraktion wurden mit Chloroform/Methanol/Ammoniak (65/:25:4, vol/vol/vol/, Galaktolipide) bzw. mit Chloroform/Methanol/Ammoniak/Wasser (70:30:4:1, vol/vol/vol/vol, Phospholipide) aufgetrennt. Die individuellen Lipidklassen wurden nach Besprühen mit Primuline-Lösung (0,05% in 80% Aceton, Sigma) unter UV-Licht identifiziert und von der Dünnschichtplatte abgekrazt und für die gaschromatographische Analyse transmethyliert wie weiter oben beschrieben.

Tabelle 5 zeigt die Ergebnisse dieser Analyse in den verschiedenen Lipidklassen. Tabelle 5 wird am Ende der Beschreibung wiedergegeben. Dabei konnte eine deutliche Anreicherung von EPA in TAG, der Ölfraktion, festgestellt werden. Des Weiteren konnten in der Polarfraktion im Phosphatidylcholin (PC) sowie Phosphatidylserin eine Akkumulation beobachtet werden.

**Tabelle 1: Gaschromatographische Analyse der Fettsäuren aus Blattmaterial von Brassica juncea Pflanzen, die mit dem Plasmid pGPTV-D6D5E6(Tc) transformiert wurden. Die Messung zeigt den Prozentanteil der einzelnen Fettsäuren in den verschiedenen transgenen Linien.**

| **Fett-säure** | **16:0** | **16:1** | **16:3** | **18:0** | **18.1 (n-9)** | **18:1 (n-11)** | **18:2** | **18:3 (GLA)** | **18:3 (ALA)** | **18:4 (SDA)** | **DGLA** | **ARA** | **EPA** |
|---|---|---|---|---|---|---|---|---|---|---|---|---|---|
| **Kontrolle PGPTV-D6D5E6(Tc)** | 11.2 | 1.8 | 13.2 | 1.4 | 0.6 | 0.8 | 6.4 | 0.0 | 55.9 | 0.0 | 0.0 | 0.0 | 0.0 |
| **C23-1** | 13.5 | 1.4 | 10.8 | 2.5 | 1.5 | 1.2 | 6.8 | 9.1 | 31.2 | 7.1 | 0.3 | 2.4 | 1.7 |
| **C23-2** | 15.3 | 1.7 | 11.8 | 3.0 | 2.1 | 1.1 | 4.1 | 5.2 | 21.9 | 4.0 | 2.1 | 8.8 | 4.8 |
| **C23.3** | 14.7 | 1.4 | 11.4 | 2.5 | 1.5 | 0.9 | 6.1 | 10.4 | 26.5 | 7.4 | 0.4 | 2,8 | 1.8 |
| **C23-4** | 13.0 | 2.0 | 13.8 | 1.8 | 0.5 | 1.1 | 5.6 | 6.2 | 39.3 | 5.5 | 0.3 | 1.8 | 1.3 |
| **C23-5** | 18.9 | 2.8 | 10.4 | 3.2 | 1.9 | 1.5 | 4.2 | 6.0 | 18.9 | 3.4 | 1.7 | 8.9 | 4.2 |
| **C23-6** | 13.6 | 1.8 | 11.7 | 2.3 | 1.2 | 1.3 | 8.1 | 9.1 | 33.1 | 5.8 | 0.3 | 1.3 | 0.9 |
| **C23.7** | 14.5 | 1.2 | 10.8 | 3.1 | 1.7 | 1.3 | 7.8 | 9.7 | 27.4 | 6.0 | 0.5 | 3.3 | 2.2 |
| **C23-9** | 15.2 | 1.9 | 12.9 | 2.4 | 1.3 | 1.3 | 3.8 | 6.8 | 25.6 | 4.5 | 1.3 | 8.4 | 4.8 |

**Tabelle 2: Gaschromatographische Analyse der Fettsäuren aus Blattmaterial von Brassica juncea Pflanzen, die mit dem Plasmid pGPTV-D6D5E6(Tc)ω3Pi transformiert wurden. Die Messung zeigt den Prozentanteil der einzelnen Fettsäuren in der nicht transgenen Kontrolle (Wt) und den verschiedenen transgenen Linien.**

| **Fettsäure** | **16:0** | **16:1** | **16:2** | **16:3** | **18:0** | **18.1 (n-9)** | **18:1 (n-11)** | **18:2 LA** | **18:3 (GLA)** | **18:3 (ALA)** | **18:4 (SDA)** | **HGLA 20:3 c8,11,14** | **ARA** | **20:4 c8.1.14.17** | **EPA** |
|---|---|---|---|---|---|---|---|---|---|---|---|---|---|---|---|
| **Wt** | 12.73 | 2.14 | 0.49 | 14.34 | 1.07 | 0.93 | 0.70 | 11.79 | - | 52.28 | - | - | n.d. | - | - |
| **11-3** | 15.69 | 1.18 | 0.80 | 13.69 | 1.97 | 2.83 | 0.61 | 3.86 | 6.68 | 24.01 | 7.24 | - | n.d. | 1.91 | 12.48 |
| **11-5** | 13.51 | 1.67 | 0.59 | 14.23 | 1.49 | 1.41 | 0.75 | 5.01 | 5.53 | 34.04 | 5.74 | - | n.d. | 1.32 | 9.21 |
| **11-13** | 12.46 | 1.47 | 0.60 | 14.06 | 1.27 | 1.41 | 0.80 | 6.97 | 3.77 | 37.82 | 4.82 | - | n.d. | 0.63 | 8.25 |

**Tabelle 3: Gaschromatographische Analyse der Fettsäuren aus Blattmaterial von Brassica juncea Pflanzen, die mit dem Plasmid pGPTV-D6D5E6(Tp)ω3Pi transformiert wurden. Die Messung zeigt den Prozentanteil der einzelnen Fettsäuren in der nicht transgenen Kontrolle (Wt) und den verschiedenen transgenen Linien.**

| **Fettsäure** | **16:0** | **16:1** | **16:2** | **16:3** | **18:0** | **18.1 (n-9)** | **18:1 (n-11)** | **18:2 LA** | **18:3 (GLA)** | **18:3 (ALA)** | **18:4 (SDA)** | **HGLA 20:3 c8,11,14** | **ARA** | **20:4 c8,1,14,17** | **EPA** |
|---|---|---|---|---|---|---|---|---|---|---|---|---|---|---|---|
| **Wt** | 12,73 | 2,14 | 0,49 | 14,34 | 1,07 | 0,93 | 0,70 | 11,79 | - | - | - | - | - | - | - |
| **10-6** | 15,04 | 1,32 | 0,68 | 11,15 | 1,70 | 2,20 | 0,63 | 4,00 | 4,95 | 25,47 | 4,38 | 0,59 | 0,28 | 4,35 | 14,74 |
| **10-10** | 11,53 | 1,49 | 0,66 | 16,71 | 1,07 | 1,59 | 0,60 | 4,10 | 5,62 | 31,15 | 5,63 | nd | nd | 2,62 | 12,25 |
| **10-11** | 15,42 | 1,63 | 0,56 | 14,45 | 1,63 | 1,88 | 0,71 | 4,08 | 6,08 | 27,34 | 5,37 | nd | nd | 2,08 | 13,75 |
| **10-13** | 13,26 | 1,53 | 0,56 | 12,91 | 2,42 | 1,84 | 0,88 | 4,85 | 5,43 | 30,49 | 4,23 | nd | nd | 3,37 | 13,68 |
| **10-14** | 14,92 | 1,55 | 1,13 | 12,72 | 1,88 | 1,46 | 0,68 | 4,76 | 5,19 | 30,23 | 4,24 | 0,46 | 0,27 | 2,68 | 12,78 |

**Tabelle 4: Analyse der verschiedenen Pflanzenorgane von nicht transgenen und transgenen Linien (pGPTV-D6D5E6(Tp)ω3Pi. Die Fettsäuren sind in Prozentanteilen angegeben.**

| **Fettsäuren (w%)** | **Samen** | | **Blätter** | | **Stengel** | | **Büten** | |
|---|---|---|---|---|---|---|---|---|
| | **WT** | **35S** | **WT** | **35S** | **WT** | **35S** | **WT** | **35S** |
| **16:3** | - | | 14.34 | 13.59 | 2.31 | 4.18 | 2.35 | 1.76 |
| **18:1 (n9)** | 33.22 | 45.43 | 0.93 | 1.97 | 1.54 | 3.84 | 1.26 | 1.51 |
| **LA** | 45.17 | 30.34 | 11.79 | 4.36 | 21.60 | 15.88 | 14.31 | 13.18 |
| **GLA (18:3, d6,9,12)** | | 0.55 | | 5.45 | | 1.14 | | 0.86 |
| **ALA (18:3, d9,12,15)** | 9.66 | 10.61 | 53.28 | 28.94 | 46.04 | 43.36 | 36.72 | 36.10 |
| **SDA (18:4, d6,9,12,15)** | | 0.13 | | 4.77 | | 1.52 | | 0.52 |
| **ARA (20:4 d5,8,11,14)** | | 0.10 | | 0.28 | | | | |
| **EPA (20:5, d5,8,11,14,17)** | | 0.10 | | 13.44 | | 0.79 | | 0.72 |

### Äquivalente:

Der Fachmann erkennt oder kann viele Äquivalente der hier beschriebenen erfindungsgemäßen spezifischen Ausführungsformen feststellen, indem er lediglich Routineexperimente verwendet. Diese Äquivalente sollen von den Patentansprüchen umfasst sein.

## Patentansprüche

1. Verfahren zur Herstellung von
(i) Eicosapentaensäure oder
(ii) Arachidonsäure und Eicosapentaensäure
im vegetativen Gewebe von transgenen Nutzpflanzen mit einem Gehalt von mindestens 4 Gew.-% bezogen auf den Gesamtlipidgehalt der transgenen Nutzpflanze, **dadurch gekennzeichnet, dass** mindestens eine Nukleinsäuresequenz, die für eine Δ-6-Desaturase codiert, mindestens eine Nukleinsäuresequenz, die für eine Δ-6-Elongase codiert, und mindestens eine Nukleinsäuresequenz, die für eine Δ-5-Desaturase codiert, in die Pflanze eingebracht werden, wobei die Nukleinsäuren ausgewählt werden aus der Gruppe bestehend aus
a) Nukleinsäuresequenzen mit den in SEQ ID NO: 1, SEQ ID NO: 3, SEQ ID NO: 5 oder SEQ ID NO: 7 dargestellten Sequenzen;
b) Nukleinsäuresequenzen, die sich als Ergebnis des degenerierten genetischen Codes von den in SEQ ID NO: 2, SEQ ID NO: 4, SEQ ID NO: 6 oder SEQ ID NO: 8 dargestellten Aminosäuresequenzen ableiten lassen; und
c) Derivaten der in SEQ ID NO: 1, SEQ ID NO: 3, SEQ ID NO: 5 oder SEQ ID NO: 7 dargestellten Nukleinsäuresequenzen, die für Polypeptide codieren, die auf Aminosäureebene mindestens 70 % Identität mit SEQ ID NO: 2, SEQ ID NO: 4, SEQ ID NO: 6 oder SEQ ID NO: 8 aufweisen und eine Δ-6-Desaturase-, Δ-6-Elongase-, oder Δ-5-Desaturaseaktivität haben;
wobei die genannten Sequenzen mit Hilfe mindestens eines konstitutiven CaMV/35S Promotors, der die Expression der besagten Nukleinsäuresequenzen im vegetativen Gewebe von Pflanzen ermöglicht, und eines Terminators in den Pflanzen exprimiert werden.

2. Verfahren nach Anspruch 1, **dadurch gekennzeichnet, dass** zusätzlich in die Nutzpflanzen eine Nukleinsäuresequenz eingebracht wird, die für eine w-3-Desäturase codiert.

3. Verfahren nach Anspruch 2, **dadurch gekennzeichnet, dass** die Nukleinsäuresequenz, die für Polypeptide mit ω-3-Desaturaseaktivität codiert, ausgewählt ist aus der Gruppe bestehend aus:
a) einer Nukleinsäuresequenz mit der in SEQ ID NO: 9 dargestellten Sequenz, oder
b) Nukleinsäuresequenzen, die sich als Ergebnis des degenerierten genetischen Codes von den in SEQ ID NO: 10 dargestellten Aminosäuresequenzen ableiten lassen, oder
c) Derivate der in SEQ ID NO: 9 dargestellten Nukleinsäuresequenz, die für Polypeptide codieren, die auf Aminosäureebene mindestens 60 % Identität mit SEQ ID NO: 10 aufweisen und eine ω-3-Desaturaseaktivität haben.

4. Verfahren nach den Ansprüchen 1 bis 3, **dadurch gekennzeichnet, dass** die Eicosapentaensäure oder Arachidonsäure und Eicosapentaensäure in den Nutzpflanzen vorwiegend als Ester in Phospholipiden oder Triacylglyceriden gebunden vorliegen.

5. Verfahren nach Anspruch 4, **dadurch gekennzeichnet, dass** die Eicosapentaensäure oder Arachidonsäure und Eicosapentaensäure vorwiegend als Ester in den Phospholipiden gebunden vorliegen und wobei die Arachidonsäure oder Eicosapentaensäure mit einem Gehalt von mindestens 10 Gew.% bezogen auf die Gesamtlipide in den Phosholipidestern vorliegt.

6. Verfahren nach Anspruch 4, **dadurch gekennzeichnet, dass** die Eicosapentaensäure oder Arachidonsäure und Eicosapentaensäure vorwiegend als Ester in den Triacylglyceriden gebunden vorliegt und wobei die Arachidonsäure oder Eicosapentaensäure mit einem Gehalt von mindestens 10 Gew.-% bezogen auf die Gesamtlipide in den Triacylglyceridestern vorliegt.

7. Verfahren nach Anspruch 1, **dadurch gekennzeichnet, dass** die Nutzpflanze eine Öl-produzierende Pflanze, eine Gemüse-, Salat- oder Zierpflanze ist.

8. Verfahren nach Anspruch 7, **dadurch gekennzeichnet, dass** die Nutzpflanze ausgewählt ist aus der Gruppe der Pflanzenfamilien bestehend aus den Familien der *Aceraceae, Actinidiaceae, Anacardiaceae, Apiaceae, Arecaceae, Asteraceae, Arecaceae, Betulaceae, Boraginaceae, Brassicaceae, Bromeliaceae, Cannabaceae, Cannaceae, Caprifoliaceae, Chenopodiaceae, Convolvulaceae, Cucurbitaceae, Dioscoreaceae, Elaeagnaceae, Ericaceae, Euphorbiaceae, Fabaceae, Fagaceae, Grossulariaceae, Juglandaceae, Lauraceae, Liliaceae, Linaceae, Malvaceae, Moraceae, Musaceae, Oleaceae, Oxalidaceae, Papaveraceae, Poaceae,* Polygonaceae, *Punicaceae, Rosaceae*, *Rubiaceae, Rutaceae, Scrophulariaceae, Solanaceae, Sterculiaceae* und *Valerianaceae.*

9. Verfahren nach den Ansprüchen 5 oder 6, **dadurch gekennzeichnet, dass** die Eicosapentaensäure oder Arachidonsäure und Eicosapentaensäure aus den Nutzpflanzen in Form ihrer Öle, Lipide oder freien Fettsäuren isoliert werden.

10. Verfahren nach den Ansprüchen 1 bis 3, **dadurch gekennzeichnet, dass** zusätzliche weitere Biosynthesegene des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-AcylTransferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) in die Nutzpflanzen eingebracht werden.

11. Verfahren nach Anspruch 10, **dadurch gekennzeichnet, dass** das zusätzliche Biosynthesegen des Fettsäure- oder Lipidstoffwechsels ausgewählt ist aus der Gruppe der Δ-4-Desaturase-, A-5-Desaturase-, Δ-6-Desaturase-, Δ-8-Desaturase-, A-9-Desaturase-, Δ-12-Desaturase-, Δ-6-Elongase- oder Δ-9-Elongase.

## Claims

1. A process for producing
(i) eicosapentaenoic acid or
(ii) arachidonic acid and eicosapentaenoic acid
in vegetative tissue of transgenic useful plants with a content of at least 4% by weight based on the total lipid content of the transgenic useful plant, wherein at least one nucleic acid sequence coding for a Δ6-desaturase, selected from the group consisting of:
a) at least one nucleic acid sequence coding for a Δ6-elongase, and at least one nucleic acid sequence coding for a Δ5-desaturase are introduced into the plant, said nucleic acids being nucleic acid sequences having the sequences depicted in SEQ ID NO: 1, SEQ ID NO: 3, SEQ ID NO: 5 or SEQ ID NO: 7;
b) nucleic acid sequences which can be derived from the amino acid sequences depicted in SEQ ID NO: 2, SEQ ID NO: 4, SEQ ID NO: 6 or SEQ ID NO: 8 due to the degeneracy of the genetic code; and
c) derivatives of the nucleic acid sequences depicted in SEQ ID NO: 1, SEQ ID NO: 3, SEQ ID NO: 5 or SEQ ID NO: 7 which code for polypeptides that are at least 70% identical at the amino acid level to SEQ ID NO: 2, SEQ ID NO: 4, SEQ ID. NO: 6 or SEQ ID NO: 8 and have a Δ6-desaturase, Δ6-elongase, or A5-desaturase activity;
wherein the sequences mentioned are expressed with the aid of at least one constitutive CaMV/35S promoter which enables said nucleic acid sequences to be expressed in vegetative tissue of plants, and one terminator in the plants.

2. The process according to claim 1 wherein a nucleic-acid sequence coding for an ω3-desaturase is additional introduced into the useful plants.

3. The process according to claim 2, wherein the nucleic acid sequence coding for polypeptides having ω3-desaturase activity is selected from the group consisting of:
a) a nucleic acid sequence having the sequence depicted in SEQ ID NO: 9, or
b) nucleic acid sequences which can be derived from the amino acid sequences depicted in SEQ ID NO: 10 due to the degeneracy of the genetic code, or
c) derivatives of the nucleic acid sequence depicted in SEQ ID NO: 9 which code for polypeptides that are at least 60% identical at the amino acid level to SEQ ID NO: 10 and have an ω3-desaturase activity.

4. The process according to claims 1 to 3, wherein the eicosapentaenoic acid or arachidonic acid and eicosapentaenoic acid are mainly bound in the form of their esters in phospholipids or triacylglycerides in the useful plants.

5. The process according to claim 4, wherein the eicosapentaenoic acid or arachidonic acid and eicosapentaenoic acid are mainly bound in the form of their esters in the phospholipids and wherein the phospholipid esters have an arachidonic acid or eicosapentaenoic acid content of at least 10% by weight based on total lipids.

6. The process according to claim 4, wherein the eicosapentaenoic acid or arachidonic acid and eicosapentaenoic acid are mainly bound in the form of their esters in the triacylglycerides and wherein the triacylglyceride esters have an arachidonic acid or eicosapentaenoic acid content of at least 10% by weight based on total lipids.

7. The process according to claim 1, wherein the useful plant is an oil-producing plant, a vegetable plant, salad plant or ornamental.

8. The process according to claim 7, wherein the useful plant is selected from the group of plant families consisting of the following families: *Aceraceae, Actinidiaceae, Anacardiaceae, Apiaceae, Arecaceae, Asteraceae, Arecaceae, Betulaceae, Boraginaceae, Brassicaceae, Bromeliaceae, Cannabaceae, Cannaceae, Caprifoliaceae, Chenopodiaceae, Convolvulaceace, Cucurbitaceae, Dioscoreaceae, Elaeagnaceae, Ericaceae, Euphorbiaceae, Fabaceae, Fagaceae, Grossulariaceae, Juglandaceae, Lauraceae, Liliaceae, Linaceae, Malvaceae, Moraceae, Musaceae, Oleaceae, Oxalidaceae, Papaveraceae, Poaceae, Polygonaceae, Punicaceae, Rosaceae, Rubiaceae, Rutaceae, Scrophulariaceae, Solanaceae, Sterculiaceae* and *Valerianaceae.*

9. The process according to either of claims 5 and 6, wherein the eicosapentaenoic acid or arachidonic acid and eicosapentaenoic acid are isolated in the form of their oils, lipids or free fatty acids from the useful plants.

10. The process according to claims 1 to 3, wherein additional further biosynthesis genes of the fatty acid or lipid metabolism selected from the group consisting of acyl-CoA dehydrogenase(s), acyl-ACP[= acyl carrier protein] desaturase(s), acyl-ACP thioesterase(s), fatty acid acyltransferase(s), acyl-CoA:lysophospholipid acyltransferase(s), fatty acid synthase(s), fatty acid hydroxylase(s), acetyl-coenzyme A carboxylase(s), acyl-coenzyme A oxidase(s), fatty acid desaturase(s), fatty acid acetylenases, lipoxygenases, triacylglycerol lipases, allene oxide synthases, hydroperoxide lyases or fatty acid elongase(s) are introduced into the useful plants.

11. The process according to claim 10, wherein the additional biosynthesis gene of the fatty acid or lipid metabolism is selected from the group consisting of the Δ4-desaturase, Δ5-desaturase, Δ6-desaturase, Δ8-desaturase, Δ9-desaturase, Δ12-desaturase, Δ6-elongase or Δ9-elongase.

## Revendications

1. Procédé de préparation
(i) d'acide éicosapentaénoïque ou
(ii) d'acide arachidonique et d'acide éicosapentaénoïque, dans le tissu végétatif de plantes de culture transgéniques, selon une teneur d'au moins 4 % en poids par rapport à la teneur totale en lipides de la plante de culture transgénique, **caractérisé en ce qu'**on introduit dans la plante au moins une séquence d'acide nucléique qui code pour une Δ-6-désaturase, au moins une séquence d'acide nucléique qui code pour une Δ-6-élongase et au moins une séquence d'acide nucléique qui code pour une Δ-5-désaturase, les acides nucléiques étant choisis dans le groupe consistant en :
a) les séquences d'acides nucléiques ayant les séquences présentées dans SEQ ID N° 1, SEQ ID N° 3, SEQ ID N° 5 ou SEQ ID N° 7 ;
b) les séquences d'acides nucléiques qui, en conséquence du code génétique dégénéré, peuvent dériver des séquences d'acides aminés présentées dans SEQ ID N° 2, SEQ ID N° 4, SEQ ID N° 6 ou SEQ ID N° 8 ; et
c) les dérives des séquences d'acides nucléiques présentées dans SEQ ID N° 1, SEQ ID N° 3, SEQ ID N° 5 ou SEQ ID N° 7, qui codent pour des polypeptides qui au niveau des acides aminés présentent une identité d'au moins 70 % avec SEQ ID N° 2, SEQ ID N° 4, SEQ ID N° 6 ou SEQ ID N° 8, et ont une activité de Δ-6-désaturase, de Δ-6-élongase ou de Δ-5-désaturase ;
les séquences mentionnées étant exprimées dans les plantes à l'aide d'au moins un promoteur CaMV/35S constitutif, qui permet l'expression des séquences d'acides nucléiques mentionnées dans le tissu végétatif de plantes, et d'un terminateur.

2. Procédé selon la revendication 1, **caractérisé en ce qu'**on introduit en outre dans les plantes de culture une séquence d'acide nucléique qui code pour une ω-3-désaturase.

3. Procédé selon la revendication 2, **caractérisé en ce que** la séquence d'acide nucléique qui code pour des polypeptides ayant une activité d'ω-3-désaturase est choisie dans le groupe consistant en :
a) une séquence d'acide nucléique ayant la séquence présentée dans SEQ ID N° 9 ; ou
b) les séquences d'acides nucléiques qui en conséquence du code génétique dégénéré peuvent dériver des séquences d'acides aminés présentées dans SEQ ID N° 10 ; ou
c) les dérivés de la séquence d'acide nucléique présentée dans SEQ ID N° 9, qui codent pour des polypeptides qui au niveau des acides aminés présentent une identité d'au moins 60 % avec SEQ ID N° 10 et ont une activité d'ω-3-désaturase.

4. Procédé selon les revendication 1 à 3, **caractérisé en ce que** l'acide éicosapentaénoïque ou l'acide arachidonique et l'acide éicosapentaénoïque se présentent dans les plantes de culture essentiellement sous forme d'esters, liés dans des phospholipides ou des triacylglycérides.

5. Procédé selon la revendication 4, **caractérisé en ce que** l'acide éicosapentaénoïque ou l'acide arachidonique et l'acide éicosapentaénoïque se présentent essentiellement sous forme d'esters liés dans les phospholipides, l'acide arachidonique ou l'acide éicosapentaénoïque étant présent selon une teneur d'au moins 10 % en poids par rapport au total des lipides dans les esters de phospholipides.

6. Procédé selon la revendication 4, **caractérisé en ce que** l'acide éicosapentaénoïque ou l'acide arachidonique et l'acide éicosapentaénoïque se présentent essentiellement sous forme d'esters liés dans les triacylglycérides, l'acide arachidonique ou l'acide éicosapentaénoïque étant présent selon une teneur d'au moins 10 % en poids par rapport au total des lipides dans les esters de triacylglycérides.

7. Procédé selon la revendication 1, **caractérisé en ce que** la plante de culture est une plante oléagineuse, un légume, une laitue ou une plante ornementale.

8. Procédé selon la revendication 7, **caractérisé en ce que** la plante de culture est choisie dans le groupe des familles de plantes consistant en les familles *Aceraceae, Actinidiaceae, Anacardiaceae, Apiaceae, Arecaceae, Asteraceae, Arecaceae, Betulaceae, Boraginaceae, Brassicaceae, Bromeliaceae, Cannabaceae, Cannaceae, Caprifoliaceae, Chenopodiaceae, Convolvulaceae, Cucurbitaceae, Dioscoreaceae, Elaeagnaceae, Ericaceae, Euphorbiaceae, Fabaceae, Fagaceae, Grossulariaceae, Juglandaceae, Lauraceae, Liliaceae, Linaceae, Malvaceae, Moraceae, Musaceae, Oleaceae, Oxalidaceae, Papaveraceae, Poaceae, Polygonaceae, Punicaceae, Rosaceae, Rubiaceae, Rutaceae, Scrophulariaceae, Solanaceae, Sterculiaceae* et *Valerianaceae.*

9. Procédé selon les revendications 5 ou 6, **caractérisé en ce que** l'acide éicosapentaénoïque ou l'acide arachidonique et l'acide éicosapentaénoïque sont isolés des plantes de culture sous forme de leurs huiles, de leurs lipides ou de leurs acides gras libres.

10. Procédé selon les revendications 1 à 3, **caractérisé en ce qu'**on introduit dans les plantes de culture d'autres gènes supplémentaires de biosynthèse du métabolisme des acides gras ou des lipides, choisis dans le groupe de la ou des acyl-CoA-déshydrogénases, de la ou des acyl-ACP[=protéine porteuse d'acyle]-désaturases, de la ou des acyl-ACP-thioestérases, de la ou des acide gras-acyltransfërases, de la ou des acyl-CoA:lysophospholipide-acyltransférases, de la ou des acide gras-synthases, de la ou des acide grashydroxylases, de la ou des acétyl-coenzyme A-carboxylases, de la ou des acyl-coenzyme A-oxydases, de la ou des acide gras-désaturases, des acide grasacétylénases, des lipoxygénases, des triacylglycérollipases, des oxydes d'allène-synthases, des hydroperoxyde-lyases ou de la ou des acide grasélongases.

11. Procède selon la revendication, 10, **caractérisé en ce que** le gène supplémentaire de biosynthèse du métabolisme des acides gras ou des lipides est choisi dans le groupe de la Δ-4-désaturase, de la Δ-5-désaturase, de la Δ-6-désaturase, de la Δ-8-désaturase, de la Δ-9-désaturase, de la Δ-12-désaturase, de la Δ-6-élongase ou de la Δ-9-elongase.
